# EUROPEAN PATENT SPECIFICATION

(11) **EP 4 489 592 B1**
(45) Date of publication and mention of the grant of the patent: **24.12.2025**
(21) Application number: 23710703.2
(22) Date of filing: 08.03.2023
(51) Int. Cl.: A23P 10/30, A61K 9/48

(54) **BIODEGRADABLE MICROCAPSULES AND A METHOD FOR THEIR PREPARATION**
BIOLOGISCH ABBAUBARE MIKROKAPSELN UND EIN VERFAHREN ZU IHRER HERSTELLUNG
MICROCAPSULES BIODEGRADABLES ET PROCEDE POUR LEUR PRODUCTION

(30) Priority: 08.03.2022 LU 501620
(43) Date of publication of application: 15.01.2025
(73) Proprietor: Xampla Limited, Cambridge, Cambridgeshire CB4 0FW (GB)
(72) Inventor: HOLLAND, Lynette Anne Makins, Cambridge Cambridgeshire CB4 0FW (GB); RODRIGUEZ GARCIA, Marc, Cambridge Cambridgeshire CB4 0FW (GB); SOMMERVILLE ROBERTS, Nigel Patrick, Cambridge Cambridgeshire CB4 0FW (GB); KEEN, Polly Helena Ruth, Cambridge Cambridgeshire CB4 0FW (GB); TAYLOR, James Ward, Cambridge Cambridgeshire CB4 0FW (GB); DELARUE, Juliette Marie Caroline, Cambridge Cambridgeshire CB4 0FW (GB); STEVENS, Scott Edward, Cambridge Cambridgeshire CB4 0FW (GB); WANG, Huafu, Cambridge Cambridgeshire CB4 0FW (GB)
(74) Representative: Pott, Thomas
(86) International application number: PCT/EP2023/055903
(87) International publication number: WO 2023/170156

(56) References cited:
- WO-A1-2020/177881
- WO-A1-2021/150456
- WO-A1-2021/191290
- WO-A1-2022/053553
- WO-A2-2007/102915
- WO-A2-2020/131879
- NESTERENKO A. ET AL: "A new way of valorizing biomaterials: The use of sunflower protein for ?-tocopherol microencapsulation", FOOD RESEARCH INTERNATIONAL, vol. 53, no. 1, 1 August 2013 (2013-08-01), AMSTERDAM, NL, pages 115 - 124, XP055843441, ISSN: 0963-9969, DOI: 10.1016/j.foodres.2013.04.020
- ALLA NESTERENKO ET AL: "Vegetable proteins in microencapsulation: A review of recent interventions and their effectiveness", INDUSTRIAL CROPS AND PRODUCTS, vol. 42, 1 March 2013 (2013-03-01), NL, pages 469 - 479, XP055459373, ISSN: 0926-6690, DOI: 10.1016/j.indcrop.2012.06.035

## Description

### FIELD OF THE INVENTION

The present invention relates to a method for preparing a biodegradable microcapsule and to# a method for preparing a biodegradable microcapsule composition. The present invention also relates to the biodegradable microcapsule and the biodegradable microcapsule composition per se. The present invention also relates to uses of the biodegradable microcapsules and to methods involving the biodegradable microcapsule, including to prepare a formulated product. The present invention also relates to the formulated product per se.

### BACKGROUND

Encapsulation techniques, which involve embedding an active ingredient(s) in an external matrix, can be used to protect the active ingredient(s) from its external surroundings (e.g. exposure to chemicals, air, light etc.) and also to protect the external surroundings from the active ingredient itself such as when it is hazardous to handle. For example, this allows the active ingredient(s) to be protected when it is added to a product formulation (e.g. a beverage formulation, a fabric conditioner formulation etc.), meaning that the active ingredient(s) in the product formulation has an acceptable shelf life and/or does not activate prematurely. This also means that the external phase of the product formulation is not negatively impacted by the active ingredient(s). The active ingredient(s) is then released when needed (e.g. upon breaking the encapsulate or upon enzymatic degradation of the encapsulate).

Many encapsulation techniques available commonly rely on the use of synthetic polymers to form a protective shell around the active ingredient. However, synthetic polymers may not be suitable as carriers in pharmaceutical and food applications or may have limited permitted exposure levels in cosmetic applications. Furthermore, synthetic polymer shell materials can lead to the formation of microplastics, which are detrimental to the environment.

Spray drying techniques allow for the large-scale preparation of microcapsules, and spray drying of plant proteins has been attempted to try to achieve production of biodegradable microcapsules. However, in order to achieve robust microcapsules of sufficiently small size via spray drying techniques, it is necessary that the material to be spray-dried has a low viscosity at a high shear rate (e.g. the material needs to be shear thinning to a low enough viscosity for successful spray drying to occur) and also has a sufficiently high protein solids content. Conventional methods to prepare a plant protein-based material suitable for spray drying involve hydrolysing the protein to break it down into lower molecular weight fragments (e.g. via treatment with acid or alkali or enzymes). However, the resultant spray-dried microcapsules are not structurally stable in water or under acidic or alkaline conditions, at elevated temperatures and/or under high shear forces, meaning that they are not suitable for incorporation into product formulations, particularly liquid product formulations, through common manufacturing processes. Furthermore, the processes involved may have a negative impact on the material being encapsulated, as many oils are unstable under alkaline conditions.

Accordingly, there exists a need for a method to prepare active ingredient-containing microcapsules of a sufficiently small size for incorporation into a liquid product formulation, which are both biodegradable and stable in the liquid product formulation (i.e. the microcapsules retain their structural integrity under either acidic or alkaline conditions such that the active ingredient(s) is protected during manufacture and for the duration of the shelf-life of the product).

WO 2007/102915 discloses compositions and methods to reduce the oxidation of an oxidizable material. A composition comprising a phospholipid-stabilized oxidizable material is disclosed. The composition comprises an oxidizable material, a phospholipid, and an optional protein. The phospholipid reduces the oxidation of the oxidizable material in the absence of water.

WO 2021/150456 discloses an encapsulation particle containing an active material and an unhydrolyzed rice, sunflower, or faba bean protein as well as a method of producing the encapsulation particle.

WO 2020/131879 discloses microcapsule compositions comprising a microcapsule dispersed in an aqueous phase. The microcapsule has a microcapsule core and a microcapsule wall encapsulating the microcapsule core. The microcapsule core contains an active material. The microcapsule wall is formed of a polymeric network comprising a first moiety derived from a protein, and a second moiety derived from a multi-functional electrophile. Also disclosed are preparation methods and consumer products containing the microcapsule compositions.

WO 2020/177881 relates to plant based materials, methods for their manufacture and biomaterials incorporating plant based materials.

### SUMMARY OF THE INVENTION

Viewed from a first aspect, the present invention provides a method for the preparation of a biodegradable microcapsule, the method comprising:
(a) forming a solution comprising one or more plant-based protein(s) in a solvent system, wherein the solvent system comprises miscible co-solvents; wherein a first co-solvent increases solubility of the plant-based protein(s), and a second co-solvent decreases solubility of the plant-based protein(s);
(b) inducing the plant-based protein in the solution to undergo a sol-gel transition to form a plant-based protein hydrogel;
(c) subjecting the plant-based protein hydrogel to a shear treatment to form a plant-based protein hydrogel slurry;
(d) dispersing an active ingredient(s) in said plant-based protein hydrogel slurry to form a composition; and
(e) drying said composition to form a microcapsule.

Viewed from a further aspect, the present invention provides a biodegradable microcapsule obtained by or obtainable by the method as hereinbefore described.

Viewed from a further aspect, the present invention provides a method for the preparation of a biodegradable microcapsule composition, the method comprising:
(a) preparing a biodegradable microcapsule according to the method as hereinbefore described; and
(b) suspending the biodegradable microcapsule in an external phase.

Viewed from a further aspect, the present invention provides a biodegradable microcapsule composition obtained by or obtainable by the method as hereinbefore described.

Viewed from a further aspect, the present invention provides a biodegradable microcapsule comprising an active ingredient(s) and a plant-based protein carrier comprising a plant-based protein(s), wherein the plant-based protein carrier encapsulates the active ingredient(s), and wherein the plant-based protein has a solubility of less than 50% when measured at a protein concentration of 5% w/w in an aqueous solution at pH 7 and 25 °C, and wherein the plant-based protein(s) have a protein secondary structure with at least 40% intermolecular β-sheet, wherein the % intermolecular β-sheet content is measured by FTIR.

Viewed from a further aspect, the present invention provides a composition comprising a biodegradable microcapsule as hereinbefore described and an external phase.

Viewed from a further aspect, the present invention provides a formulated product comprising a biodegradable microcapsule as hereinbefore described.

Viewed from a further aspect, the present invention provides method of making a formulated product, comprising:
(a) preparing a biodegradable microcapsule according to the method as hereinbefore described; and
(b) mixing said biodegradable microcapsule with a product formulation.

Viewed from a further aspect, the present invention provides the use of a biodegradable microcapsule as hereinbefore described in a formulated product.

### DEFINITIONS

As used herein, the term "microcapsule" refers to any form of microparticle. For example, the term encompasses core-shell microcapsules (i.e. a microcapsule having a central core containing the active ingredient(s), wherein the core is surrounded by a plant-based protein hydrogel in the form of a shell). As will be understood by a skilled person, a core-shell microcapsule may have a multicore morphology (i.e. wherein the core phase is in the form of a plurality of droplets) or a single core morphology (wherein the core phase is in the form of a single droplet). The term also encompasses matrix microcapsules (i.e. a microcapsule comprised of a plant-based protein hydrogel matrix in which the active ingredient(s) has been dispersed throughout). A microcapsule may have a d₅₀ as determined by laser diffraction of between 500 nm and 2mm.

As used herein, the term "lower shear step" may refer to a process step in which low levels of mechanical energy are applied to a material, preferably by a cutting action, to cause it to break or fragment primarily into large discrete fragments. "Lower shear" does not typically include any milling step that shatters or fragments a material by highspeed impact, for example impacts at a differential velocity of greater than 2 ms⁻¹. Nor does it typically include milling processes based on cavitation. In a particular embodiment, during the lower shear step, a hydrogel is fragmented to give fragments such that at least 80% by weight of the hydrogel fragments have a maximum dimension as determined by optical microscopy, of between 1 mm and 100 mm.

As used herein, the term "higher shear step" may refer to a process step which applies energy to reduce the hydrogel to small fragments, such as to form e.g. a colloidal dispersion. In a particular embodiment, during the higher shear step, a hydrogel is fragmented to give fragments having a d₅₀ particle size as determined by laser diffraction of 0.5 to 50 microns. Laser diffraction can be performed according to the methods defined herein.

For the avoidance of doubt, a higher shear step subjects the hydrogel to higher levels of shear than the lower shear step. In the instance the method involves both a lower shear step and a higher shear step, the higher shear step must happen after the lower shear step (i.e. they are discrete steps occurring in this particular order).

As used herein, the term "sol-gel transition temperature" refers to the temperature at which a plant-based protein transforms from a liquid state into a hydrogel state. Thus, at temperatures above the sol-gel transition temperature, the plant-based protein will be in a liquid state, and at temperatures below the sol-gel transition temperature the plant-based protein will be in a hydrogel state.

As used herein, the term "fragrance" (used interchangeably with the term "perfume") refers to the component of a formulation that is capable of imparting or modifying the odour of a product, such as a fabric conditioner or a hair conditioner or a substrate such as fabric or hair. A fragrance is typically used to impart an overall pleasant odour or odour profile to a product either to provide a pleasurable experience, such as a Fine Fragrance, or to provide sensory cues as to the product's benefit and function, such as a calming effect for a lavender sleep aid, the idea of cleanliness for a laundry product, or to mask an unpleasant odour, such as in an insect repellent product. A "fragrance" may be composed of one or more components that can be a single chemical entity, referred to herein as a "fragrance material" (used interchangeably with the term "perfume material"), or a mixture of different "fragrance materials". Fragrance materials can be created by either synthetic processes or extracted from nature, particularly plants, to obtain naturally occurring plant essential oils and plant extracts such as orange oil. Fragrance materials created by synthetic processes can be either new-to-the-world chemicals or nature-identical fragrance materials. Synthetic and naturally derived fragrance materials can then be blended into fragrances by skilled perfumers, also called noses, for use in consumer products. Fragrance materials can be obtained from specialist fragrance suppliers, known as fragrance houses, as individual chemicals, natural blends or as proprietary specialty blends where the full composition is not disclosed. The individual fragrance materials which comprise a known natural blend can be found by reference to Journals commonly used by those skilled in the art such as "Perfume and Flavourist" or "Journal of Essential Oil Research", or listed in reference texts such as the book by S. Arctander, Perfume and Flavor Chemicals, 1969, Montclair, New Jersey, USA and more recently re-published by Allured Publishing Corporation Illinois (1994) and "Perfume and Flavour Materials of Natural Origin", S. Arctander, Ed., Elizabeth, N.J., 1960. It will be understood that for the purposes of this invention, a "fragrance material" includes a pro-fragrance such as an acetal pro-fragrance, ketal pro-fragrance, ester pro-fragrance, hydrolysable inorganic-organic pro-fragrance, and combinations thereof. The fragrance materials may be released from the pro-fragrances in a number of ways, for example, by hydrolysis, or by a shift in an equilibrium reaction, or by a pH-change, or by enzymatic release, or by UV-radiation.

A fragrance material can be described in terms of its odour strength, detection threshold, odour saturation and its character. In fragrance encapsulates it is preferable to use fragrance materaials with a low odour detection threshold and a high strength so as to maximise the noticeability of even small levels of fragrance encapsulated and released.

In order to impart an odour, a fragrance material must be volatile, even if only to a small degree, as it is necessary for the molecule to be airborne to enter the nose, where it gets attached to specific neuroreceptors and elicits a signal within the olfactory system. Fragrance materials can be classified according to their volatility. Preferably fragrance materials are liquid at 20°C and atmospheric pressure but occasionally they may be solid and can be blended with other liquid fragrance materials or solvents. Typically, the fragrance industry refers to the volatility and substantivity by loosely categorising materials into one of 3 categories: base notes for the least volatile and most substantive, heart notes for those of moderate volatility and substantivity and top notes for the most volatile and least substantive. This is based on the odour perception of the materials and is quite subjective. One way of objectively classifying the volatility of fragrance materials is according to their vapour pressure.

As used herein, the term "vapour pressure" means the partial pressure in air at a defined temperature (e.g., 25°C) and standard atmospheric pressure (760 mmHg) for a given chemical species. It defines a chemical species' affinity for the gas phase rather than the liquid or solid state. The higher the vapour pressure the greater the proportion of the material that will, at equilibrium, be in a closed headspace. It is also related to the rate of evaporation of a fragrance material which is defined in an open environment where the material is leaving the system. The vapour pressure can be readily determined according to the reference program ACD/Percepta Desktop Software, Version 14.0 (Build: Aug/26/2021), Advanced Chemistry Development, Inc (ACD/Labs), Toronto, Canada, www.acdlabs.com.

A physical parameter that is relevant to the encapsulation of a fragrance material is its hydrophobicity, which may be defined in terms of its partition coefficient P. As used herein, the term "partition coefficient" refers to the ratio between the equilibrium concentration of that substance in n-octanol and in water, and is a measure of the differential solubility of said substance between these two solvents. As used herein, the term "logP" refers to the logarithm to the base 10 of the partition coefficient P. The logP can be readily determined according to the reference program ACD/Percepta Desktop Software, Version 14.0 (Build: Aug/26/2021), Advanced Chemistry Development, Inc (ACD/Labs), Toronto, Canada, www.acdlabs.com LogP values are predicted from the SMILE string of fragrance materials molecules. Three different types of logP values can be selected from the software. The logP Classic is based on an algorithm which takes into account a database of experimental logP values while using the principal of isolating carbons. The logP GALAS is based on an algorithm taking into account a database of a training set of compounds as well as adjusting the values with data from structurally close compounds. The consensus logP is a model based on the previous two algorithms, which can be expressed as: consensus logP = a x logP Classic + b x logP GALAS, where a and b are coefficients of the model. The latter value, consensus logP, is the logP value indicated herein.

Another aspect that is relevant to the encapsulation of a fragrance material is its Hansen Solubility Parameters (HSPs). The term HSP refers to a solubility parameter approach proposed by Charles Hansen first used to predict polymer solubility in a given solvent as described in, The Three Dimensional Solubility Parameter and Solvent Diffusion Coefficient, by Charles Hansen, Danish Technical Press (Copenhagen, 1967). This approach has since been reapplied to many other molecules. The fragrance material (or flavour material or solvent) and its interactions with its environment are defined by 3 forces: atomic dispersion forces, molecular permanent dipole forces, and molecular hydrogen bonding forces. Materials with similar HSP parameters are more likely to be miscible. These forces can be quantified by 3 values: δD, the Hansen dispersion value which relates to the Van der Waals interactions (intermolecular forces); δP, the Hansen polarity value which relates to the dipole moment (electrical charges); and δH, the Hansen Hydrogen-bonding ("h-bonding") value. The solubility parameter δ (MPa^{1/2}) is defined as δ² = δ_{D}² + δ_{P}² + δ_{H}² = E/V, where E is the cohesive energy of a solvent and V is the molar volume. The HSP values for a given material can be obtained from the HSPiP (Hansen Solubility Parameters in Practice) software available from www.hansen-solubility.com through two main different ways. Those values can either be retrieved from the Master Dataset, which comprises over 20,000 materials, by searching by name or CAS number; or be predicted by entering the SMILE string of a given molecule in the DIY section of the software, using the Y-MB (Yamamoto-Molecular Breaking) method. Furthermore, the determination of the HSP sphere relative to a given fragrance material is a good way to predict the solubility preferences within a blend of fragrance materials. The radius Ro of the HSP sphere is defined as Ro = Ra/RED, where Ra is the HSP distance between two molecules (1 and 2) expressed by: Ra² = 4 (δ_{D1} - δ_{D2})² + (δ_{P1} - δ_{P2})² + (δ_{H1} - δ_{H2})², and RED is the Relative Energy Difference. This RED value can also be extracted from or predicted through the HSPiP software, and a good solvent for a given material should exhibit a RED value lower or equal to 1, whereas a solvent displaying a RED value greater than 1 should be considered as a bad solvent for the given material.

A fragrance material may be selected from an alcohol, an aldehyde, a ketone, an ester, an ether, an acetate, an alkene, a nitrile, a nitrogenous heterocyclic compound, a sulfurous heterocyclic compound, and a Schiff base.

Preferred aldehyde fragrance materials include, without limitation, alpha-amylcinnamaldehyde, anisic aldehyde, decyl aldehyde, lauric aldehyde, methyl n-nonyl acetaldehyde, methyl octyl acetaldehyde, nonylaldehyde, benzenecarboxaldehyde, neral, geranial, 1,1-diethoxy-3,7-dimethylocta-2,6-diene, 4-isopropylbenzaldehyde, 2,4-dimethyl-3-cyclohexene-1-carboxaldehyde, alpha-methyl-p-isopropyldihydrocinnamaldehyde, 3-(3-isopropylphenyl) butanal, alpha-hexylcinnamaldehyde, 7-hydroxy-3,7-dimethyloctan-1-al, 2,4-dimethyl-3-cyclohexene-1-carboxaldehyde, octyl aldehyde, phenylacetaldehyde, 2,4-dimethyl-3-cyclohexene-1-carboxaldehyde, hexanal, 3,7-dimethyloctanal, 6,6-dimethylbicyclo[3.1.1]hept-2-ene-2-butanal, nonanal, octanal, 2-nonenal undecenal, 2-methyl-4-(2,6,6-trimethyl-1-cyclohexenyl-1)-2-butenal, 2,6-dimethyloctanal, 3-(p-isopropylphenyl)propionaldehyde, 3-phenyl-4-pentenal citronellal, o/p-ethyl-alpha, alpha, 9-decenal, dimethyldihydrocinnamaldehyde, p-isobutyl-alphamethylydrocinnamaldehyde, cis-4-decen-1-al, 2,5-dimethyl-2-ethenyl-4-hexenal, trans-2-methyl-2-butenal, 3-methylnonanal, alpha-sinensal, 3-phenylbutanal, 2,2-dimethyl-3-phenylpropionaldehyde, m-tertbutyl-alpha-methyldihydrocinnamic aldehyde, geranyl oxyacetaldehyde, trans-4-decen-1-al, methoxycitronellal, and mixtures thereof.

Preferred ester fragrance materials include, without limitation, allyl cyclohexane-propionate, allyl heptanoate, allyl amyl glycolate, allyl caproate, amyl acetate (n-pentyl acetate), amyl propionate, benzyl acetate, benzyl propionate, benzyl salicylate, cis-3-hexenylacetate, citronellyl acetate, citronellyl propionate, cyclohexyl salicylate, dihydro isojasmonate, dimethyl benzyl carbinyl acetate, ethyl acetate, ethyl acetoacetate, ethyl butyrate, ethyl-2-methyl butryrate, ethyl-2-methyl pentanoate, fenchyl acetate (1,3,3-trimethyl-2-norbornanyl acetate), tricyclodecenyl acetate, tricyclodecenyl propionate, geranyl acetate, cis-3-hexenyl isobutyrate, hexyl acetate, cis-3-hexenyl salicylate, n-hexyl salicylate, isobornyl acetate, linalyl acetate, para-tertiary-butyl cyclohexyl acetate, (-)-L-menthyl acetate, ortho-tertiary-butylcyclohexyl acetate, methyl benzoate, methyl dihydro isojasmonate, alpha-methylbenzyl acetate, methyl salicylate, 2-phenylethyl acetate, prenyl acetate, cedryl acetate, cyclabute, phenethyl phenylacetate, terpinyl formate, citronellyl anthranilate, ethyl tricyclo[5.2.1.0-2,6]decane-2-carboxylate, n-hexyl ethyl acetoacetate, 2-tertbutyl-4-methyl cyclohexyl acetate, formic acid, 3,5,5-trimethylhexyl ester, phenethyl crotonate, cyclogeranyl acetate, geranyl crotonate, ethyl geranate, geranyl isobutyrate, 3,7-dimethyl-ethyl 2-nonynoate-2,6-octadienoic acid methyl ester, citronellyl valerate, 2-hexenylcyclopentanone, cyclohexyl anthranilate, L-citronellyl tiglate, butyl tiglate, pentyl tiglate, geranyl caprylate, 9-decenyl acetate, 2-isopropyl-5-methylhexyl-1 butyrate, n-pentyl benzoate, 2-methylbutyl benzoate (and mixtures thereof with pentyl benzoate), dimethyl benzyl carbinyl propionate, dimethyl benzyl carbinyl acetate, trans-2-hexenyl salicylate, dimethyl benzyl carbinyl isobutyrate, 3,7-dimethyloctyl formate, rhodinyl formate, rhodinyl isovalerate, rhodinyl acetate, rhodinyl butyrate, rhodinyl propionate, cyclohexylethyl acetate, neryl butyrate, tetrahydrogeranyl butyrate, myrcenyl acetate, 2,5-dimethyl-2-ethenylhex-4-enoic acid, methyl ester, 2,4-dimethylcyclohexane-1-methyl acetate, ocimenyl acetate, linalyl isobutyrate, 6-methyl-5-heptenyl-1 acetate, 4-methyl-2-pentyl acetate, n-pentyl 2-methylbutyrate, propyl acetate, isopropenyl acetate, isopropyl acetate, 1-methylcyclohex-3-ene-carboxylic acid, methyl ester, propyl tiglate, propyl/isobutyl cyclopent-3-enyl-1-acetate (alphavinyl), butyl 2-furoate, ethyl 2-pentenoate, (E)-methyl 3-pentenoate, 3-methoxy-3-methylbutyl acetate, n-pentyl crotonate, n-pentyl isobutyrate, propyl formate, furfuryl butyrate, methyl angelate, methyl pivalate, prenyl caproate, furfuryl propionate, diethyl malate, isopropyl 2-methylbutyrate, dimethyl malonate, bornyl formate, styralyl acetate, 1-(2-furyl)-1-propanone, I-citronellyl acetate, 3,7-dimethyl-1,6-nonadien-3-yl acetate, neryl crotonate, dihydromyrcenyl acetate, tetrahydromyrcenyl acetate, lavandulyl acetate, 4-cyclooctenyl isobutyrate, cyclopentyl isobutyrate, 3-methyl-3-butenyl acetate, allyl acetate, geranyl formate, cis-3-hexenyl caproate, and mixtures thereof.

Preferred alcohol fragrance materials include, without limitation, benzyl alcohol, beta-gamma-hexenol (2-hexen-1-ol), cedrol, citronellol, cinnamic alcohol, p-cresol, cumic alcohol, dihydromyrcenol, 3,7-dimethyl-1-octanol, dimethyl benzyl carbinol, eucalyptol, eugenol, fenchyl alcohol, geraniol, hydratopic alcohol, isononyl alcohol (3,5,5-trimethyl-1-hexanol), linalool, methyl chavicol (estragole), methyl eugenol (eugenyl methyl ether), nerol, 2-octanol, patchouli alcohol, phenyl hexanol (3-methyl-5-phenyl-1-pentanol), phenethyl alcohol, alpha-terpineol, tetrahydrolinalool, tetrahydromyrcenol, 4-methyl-3-decen-5-ol, I-3,7-dimethyloctane-1-ol, 2-(furfuryl-2)-heptanol, 6,8-dimethyl-2-nonanol, ethyl norbornyl cyclohexanol, beta-methyl cyclohexane ethanol, 3,7-dimethyl-(2),6-octen(adien)-1-ol, trans-2-undecen-1-ol, 2-ethyl-2-prenyl-3-hexenol, isobutyl benzyl carbinol, dimethyl benzyl carbinol, ocimenol, 3,7-dimethyl-1,6-nonadien-3-ol (cis & trans), tetrahydromyrcenol, alpha-terpineol, 9-decenol-1, 2-(2-hexenyl)-cyclopentanol, 2,6-dimethyl-2-heptanol, 3-methyl-1-octen-3-ol, 2,6-dimethyl-5-hepten-2-ol, 3,7,9-trimethyl-1,6-decadien-3-ol, 3,7-dimethyl-6-nonen-1-ol, 3,7-dimethyl-1-octyn-3-ol, 2,6-dimethyl-1,5,7-octatrienol-3, dihydromyrcenol, 2,6,-trimethyl-5,9-undecadienol, 2,5-dimethyl-2-propylhex-4-enol-1, (Z)-3-hexenol, o,m,p-methyl-phenylethanol, 2-methyl-5-phenyl-1-pentanol, 3-methylphenethyl alcohol, para-methyl dimethyl benzyl carbinol, methyl benzyl carbinol, p-methylphenylethanol, 3,7-dimethyl-2-octen-1-ol, 2-methyl-6-methylene-7-octen-4-ol, and mixtures thereof.

Preferred ketone fragrance materials include, without limitation, oxacycloheptadec-10-en-2-one, benzylacetone, benzophenone, L-carvone, cis-jasmone, 4-(2,6,6-trimethyl-3-cyclohexen-1-yl)-but-3-en-4-one, ethyl amyl ketone, alpha-ionone, ionone beta, ethanone, octahydro-2,3,8,8-tetramethyl-2-acetonaphthalene, alpha-irone, 1-(5,5-dimethyl-1-cyclo-hexen-1-yl)-4-penten-1-one, 3-nonanone, ethyl hexyl ketone, menthone, 4-methyl-acetophenone, gamma-methyl ionone, methyl pentyl ketone, methyl heptenone (6-methyl-5-hepten-2-one), methyl heptyl ketone, methyl hexyl ketone, delta muscenone, 2-octanone, 2-pentyl-3-methyl-2-cyclopenten-1-one, 2-heptylcyclopentanone, alpha-methylionone, 3-methyl-2-(trans-2-pentenyl)-cyclopentenone, octenyl cyclopentanone, n-amylcyclopentenone, 6-hydroxy-3,7-dimethyloctanoic acid lactone, 2-hydroxy-2-cyclohexen-1-one, 3-methyl-4-phenyl-3-buten-2-one, 2-pentyl-2,5,5-trimethylcyclopentanone, 2-cyclopentylcyclopentanol-1, 5-methylhexan-2-one, gamma-dodecalactone, delta-dodecalactone, gamma-nonalactone, delta-nonalactone, gamma-octalactone, delta-undecalactone, gamma-undecalactone, alpha damascone, beta damascone, gamma damascone, delta damascone and mixtures thereof.

Preferred ether fragrance materials include, without limitation, diphenyl oxide, p-cresyl methyl ether, 4,6,6,7,8,8-hexamethyl-1,3,4,6,7,8-hexahydro-cyclopenta(G)-2-benzopyran, beta-naphthyl methyl ether, methyl isobutenyl tetrahydropyran, 5-acetyl-1,1,2,3,3,6-hexamethylindan (phantolide), 7-acetyl-1,1,3,4,4,6-hexamethyltetralin (tonalid), 2-phenylethyl-3-methylbut-2-enyl ether, ethyl geranyl ether, phenylethyl isopropyl ether, and mixtures thereof.

Preferred alkene fragrance materials include, without limitation, allo-ocimene, camphene, beta-caryophyllene, cadinene, diphenylmethane, d-limonene, lymolene, beta-myrcene, para-cymene, 2-alpha-pinene, beta-pinene, alpha-terpinene, gamma-terpinene, terpineolene, 7-methyl-3-methylene-1,6-octadiene, and mixtures thereof.

Preferred nitrile fragrance materials include, without limitation, 3,7-dimethyl-6-octenenitrile, 3,7-dimethyl-2(3), 6-nonadienenitrile, (2E,6Z)-2,6-nonadienenitrile, n-dodecane nitrile, and mixtures thereof.

Preferred Schiff base fragrance materials include, without limitation, citronellyl nitrile, nonanal/methyl anthranilate, N-octylidene-anthranilic acid methyl ester, hydroxycitronellal/methyl anthranilate, cyclamen aldehyde/methyl anthranilate, methoxyphenylpropanal/methyl anthranilate, ethyl p-aminobenzoate/hydroxycitronellal, citral/methyl anthranilate, 2,4-dimethylcyclohex-3-enecarbaldehyde methyl anthranilate, hydroxycitronellal-indole, and mixtures thereof.

As used herein, the term "flavour" refers to the component of a formulation that is capable of imparting or modifying the taste and smell of a product, such as a toothpaste or foodstuff. A flavour is typically used to impart an overall pleasant taste and smell, or a taste and smell profile, to a product either to simply provide a pleasurable experience, such as in a foodstuff, or to mask an unpleasant taste or smell, such as in a medicine. A flavour or flavour material can be described in terms of its aroma strength, detection threshold and quality. A "flavour" may be composed of one or more components that can be a single chemical entity, referred to herein as a "flavour material", or a mixture of different "flavour materials". Flavour materials can be created by either synthetic processes or extracted from nature, particularly from plants, to create naturally occurring plant and animal oils and exudate, such as vanilla. Synthetic and naturally derived flavour materials can then be blended into flavours by skilled flavourists for use in consumer products. Flavour materials can be obtained from specialist flavour suppliers, known as flavour houses, as individual chemicals, natural blends or as proprietary specialty blends where the full composition is not disclosed. The individual flavour materials which comprise a known natural blend can be found by reference to Journals commonly used by those skilled in the art such as "Perfume and Flavourist" or "Journal of Essential Oil Research", or listed in reference texts such as the book by S. Arctander, Perfume and Flavor Chemicals, 1969, Montclair, New Jersey, USA and more recently re-published by Allured Publishing Corporation Illinois (1994); "Perfume and Flavour Materials of Natural Origin", S. Arctander, Ed., Elizabeth, N.J., 1960; and "Flavourings", E. Ziegler and H. Ziegler (ed.), Wiley-VCH Weinheim, 1998. It will be understood that flavours can be volatile or have volatile components which are detected by the nose in the same way as fragrances. As such, flavour materials can also be classified according to their physical characteristics, such as volatility and hydrophobicity, using the methods described above for fragrance materials. Flavour materials can also be described according to their Hansen Solubility Parameters using the methods described above for fragrance materials.

Sources of flavour materials include essential oils, concretes, absolutes, resins, resinoids, balsams, and tinctures. Preferred flavour materials include anise oil, ethyl-2-methyl butyrate, vanillin, cis-3-heptenol, cis-3-hexenol, trans-2-heptenal, butyl valerate, 2,3-diethyl pyrazine, methylcyclo-pentenolone, benzaldehyde, valerian oil, 3,4-dimeth-oxyphenol, amyl acetate, amyl cinnamate, y-butyryl lactone, trimethyl pyrazine, phenyl acetic acid, isovaleraldehyde, ethyl maltol, ethyl vanillin, ethyl valerate, ethyl butyrate, cocoa extract, coffee extract, peppermint oil, spearmint oil, clove oil, anethol, cardamom oil, wintergreen oil, cinnamic aldehyde, ethyl-2-methyl valerate, g-hexenyl lactone, 2,4-decadienal, 2,4-heptadienal, methyl thiazole alcohol (4-methyl-5-b-hydroxyethyl thiazole), 2-methyl butanethiol, 4-mercapto-2-butanone, 3-mercapto-2-pentanone, 1-mercapto-2-propane, benzaldehyde, furfural, furfuryl alcohol, 2-mercapto propionic acid, alkyl pyrazine, methyl pyrazine, 2-ethyl-3-methyl pyrazine, tetramethyl pyrazine, polysulfides, dipropyl disulphide, methyl benzyl disulphide, alkyl thiophene, 2,3-dimethyl thiophene, 5-methyl furfural, acetyl furan, 2,4-decadienal, guiacol, phenyl acetaldehyde, b-decalactone, d-limonene, acetoin, amyl acetate, maltol, ethyl butyrate, levulinic acid, piperonal, ethyl acetate, n-octanal, n-pentanal, n-hexanal, diacetyl, monosodium glutamate, monopotassium glutamate, sulfur-containing amino acids, e.g., cysteine, 2-methylfuran-3-thiol, 2-methyldihydrofuran-3-thiol, 2,5-dimethylfuran-3-thiol, tetramethyl pyrazine, propylpropenyl disulphide, propylpropenyl trisulfide, diallyl disulphide, diallyl trisulfide, dipropenyl disulphide, dipropenyl trisulfide, 4-methyl-2-[(methylthio)-ethyl]-1,3-dithiolane, 4,5-dimethyl-2-(methylthiomethyl)-1,3-dithiolane, and 4-methyl-2-(methylthiomethyl)-1,3- dithiolane, hop oils, and citrus oils such as lemon, orange, lime, and grapefruit.

### DETAILED DESCRIPTION OF THE INVENTION

The inventors of the present invention have uncovered a method to prepare a high solids content protein colloidal dispersion of controlled particle size and low viscosity comprising an active ingredient(s), which allows for the formation of stable microcapsules upon drying. The resultant microcapsules are stable in liquid product formulations, and also under the conditions necessary to prepare such a liquid product formulation (e.g. pasteurisation conditions in the case of a beverage formulation).

The present invention is directed to a method for the preparation of a biodegradable microcapsule, the method comprising:
(a) forming a solution comprising one or more plant-based protein(s) in a solvent system, wherein the solvent system comprises miscible co-solvents; wherein a first co-solvent increases solubility of the plant-based protein(s), and a second co-solvent decreases solubility of the plant-based protein(s);
(b) inducing the plant-based protein in the solution to undergo a sol-gel transition to form a plant-based protein hydrogel;
(c) subjecting the plant-based protein hydrogel to a shear treatment to form a plant-based protein hydrogel slurry;
(d) dispersing an active ingredient(s) in said plant-based protein hydrogel slurry to form a composition; and
(e) drying said composition to form a microcapsule.

Any suitable plant-based proteins may be used in the present invention. In preferred methods of the present invention, the plant-based protein(s) is obtained from fava bean, mung bean, pea, rice, potato, rapeseed, lentil, chickpea, sunflower seed, pumpkin seed, flax, chia, canola, lupine, alfalfa, moringa, wheat, corn zein or sorghum; preferably the plant protein(s) is selected from pea protein, potato protein, rapeseed protein, lentil protein, chickpea protein, fava bean protein, mung bean protein, sunflower seed protein, pumpkin seed protein, flax protein, chia protein, canola protein, lupine protein, alfalfa protein, moringa protein and/or rice protein. More preferably, the plant-based protein is pea protein and/or potato protein. Such proteins are considered to be low allergenicity proteins.

Suitable plant-based proteins further include:
- Brassicas: including Brassica balearica: Mallorca cabbage, Brassica carinata: Abyssinian mustard or Abyssinian cabbage, Brassica elongata: elongated mustard, Brassica fruticulosa: Mediterranean cabbage, Brassica hilarionis: St Hilarion cabbage, Brassica juncea: Indian mustard, brown and leaf mustards, Sarepta mustard, Brassica napus: rapeseed, canola, rutabaga, Brassica narinosa: broadbeaked mustard, Brassica nigra: black mustard, Brassica oleracea: kale, cabbage, collard greens, broccoli, cauliflower, kai-lan, Brussels sprouts, kohlrabi, Brassica perviridis: tender green, mustard spinach, Brassica rapa (syn. B. campestris): Chinese cabbage, turnip, rapini, komatsuna, Brassica rupestris: brown mustard, Brassica tournefortii: Asian mustard
- Solanaceae: including tomatoes, potatoes, eggplant, bell and chili peppers;
- cereals: including maize, rice, wheat, barley, sorghum, millet, oats, rye, triticale, fonio
- pseudocereals: including amaranth (love-lies-bleeding, red amaranth, prince-of-Wales-feather), breadnut, buckwheat, chia, cockscomb (also called quail grass or soko), pitseed Goosefoot, qañiwa, quinoa and, wattleseed (also called acacia seed);
- Legume: including Acacia alata (Winged Wattle), Acacia decipiens, Acacia saligna (commonly known by various names including coojong, golden wreath wattle, orange wattle, blue-leafed wattle), Arachis hypogaea (peanut), Astragalus galegiformis, Cytisus laburnum (the common laburnum, golden chain or golden rain), Cytisus supinus, Dolichios lablab (common names include hyacinth bean, lablab-bean bonavist bean/pea, dolichos bean, seim bean, lablab bean, Egyptian kidney bean, Indian bean, bataw and Australian pea.), Ervum lens (Lentil), Genista tinctorial (common names include dyer's whin, waxen woad and waxen wood), Glycine max (Soybean), Lathyrus clymenum (peavines or vetchlings), Lathyrus odoratus (peavines or vetchlings), Lathyrus staivus (peavines or vetchlings), Lathyrus Silvetris (peavines or vetchlings), Lotus tetragonolobus (asparagus-pea or winged pea), Lupinus albus (Lupin), Lupinus angustifolius (lupin), Lupinus luteus (Lupin), Lupinus polyphyllus (Lupin), Medicago sativa (Alfalfa), Phaseolus aureus (Mung bean), Phaseolus coccineus (Runner bean), Phaseolus nanus (Green bean / French bean), Phaseolus vulgaris (Green bean / French bean), Pisum sativum (pea), Trifolium hybridum (Clover), Trifolium pretense (Red clover), Vicia faba (Broad bean), Vicia sativa (Vetch), Vigna unguiculate (cowpea)
- Non-Legumes: including: Acanshosicyos horrida (Acanshosicyos horrida), Aesculus hyppocastanum (Conker tree / Horsechestnut), Anacardium occidentale (Cashew tree), Balanites aegyptica, Bertholletia excels (Brazil nut), Beta vulgaris (Sugar beet), Brassica napus (Rapeseed), Brassica juncea (Brown mustard), Brassica nigra (Black mustard), Brassica hirta (Eurasian mustard), Cannabis sativa (marijuana), Citrullus vulgaris (Sort of watermelon), Citrus aurantiaca (Citrus), Cucurbita maxima (squash), Fagopyrum esculentum (knotweed), Gossypium barbadense (Extra-long staple cotton), Heianthus annuus (sunflower), Nicotiana sp. (Tobacco plant), Prunus avium (cherry), Prunus cerasus (Sour cherry), Prunus domestica (plum), Prunus amygdalus (almond), Rricinus communis (Caster bean/ caster oil plant), Sasamum indicum (Sesame), Sinapis alba (White mustard), Terlfalrea pedata (Oyster nut).

For the avoidance of doubt, the plant-based microcapsules of the present invention do not encompass plants in their natural state, e.g. naturally formed plant cells, organelles or vesicles are not plant-based microcapsules of the present invention.

In step (a), the first co-solvent increases solubility of the plant-based protein(s). The first co-solvent may be considered a solubilising co-solvent. There may be one or more solubilising co-solvent(s) and the solubilising co-solvent(s) may fully or partially solubilise the plant-based protein(s). The co-solvents may be added in step (a) in a highly concentrated form or in a diluted form.

Examples of solubilising co-solvents are organic acids. An organic acid is an organic compound with acidic properties. Preferably, the organic acids are sourced from natural plant-based or bio-based feed-stocks.

In preferred methods of the present invention, the first co-solvent is an organic acid. Preferably, the organic acid is acetic acid, lactic acid, formic acid, gluconic acid, propionic acid, an α-hydroxy acid and/or a β-hydroxy acid. Preferred α-hydroxy acids include glycolic acid, lactic acid, acetic acid, malic acid, citric acid and/or tartaric acid, preferably lactic acid or acetic acid. Preferrred β-hydroxy acid may include β-hydroxypropionic acid, β-hydroxybutyric acid, β-hydroxy β-methylbutyric acid, 2-hydroxybenzoic acid and carnitine. In particularly preferred methods of the present invention, the organic acid is acetic acid and/or lactic acid.

Using an organic acid enables solubilisation of the plant protein and also allows for mild hydrolysis of the protein. For example, without wishing to be bound by theory, the solubility of plant-based proteins in organic acid is possible due to: i) the protonation of proteins and ii) the presence of an anion solvation layer which contributes to a reduction of hydrophobic interactions. Once initially dissolved in organic acid, the protonation of plant-based proteins can help to stabilise them in its non-solvent, for example water.

In step (a), the second co-solvent has decreased solubility of the plant based protein(s), as compared to the first co-solvent. The second co-solvent may be considered a de-solubilising co-solvent. There may be one or more de-solubilising co-solvent(s).

In preferred methods of the present invention, the second co-solvent is selected from water, ethanol, and/or ethyl acetate, more preferably water and/or ethanol, even more preferably water.

In preferred methods of the present invention, the solvent system comprises a co-solvent ratio of first co-solvent to second co-solvent of about 10-90% v/v, preferably about 20-90% v/v, preferably about 20-80% v/v, preferably about 20-60% v/v, about 25-55% v/v, about 30-50% v/v, about 20%, about 30%, about 40% about 50% or about 60% v/v, most preferably about 30-50% v/v.

In preferred methods of the present invention, the first co-solvent is present in the solution of step (a) at a concentration based on weight-% of equal or greater than the concentration of the protein.

In preferred methods of the present invention the pH of the plant-based protein solution in step (a) is at least 0.5 pH units, more preferably at least 1.0 pH units, below the isoelectric point of the plant-based protein.

In preferred methods of the present invention, the concentration of the plant-based protein(s) in the solvent system is 25-200mg/ml, more preferably 50-150mg/ml.

The ratio of organic acid may vary depending on protein concentration, e.g. using a higher organic acid ratio with increasing protein concentration.

In preferred methods of the present invention, the degree of protein hydrolysis (i.e. the percentage of cleaved peptide bonds in a protein hydrolysate) is controlled to modify the properties of the resultant hydrogel. For example, increasing the acid concentration present during formation will increase the degree of protein hydrolysis. Higher degree of protein hydrolysis leads to the formation of less rigid hydrogels.

In preferred methods of the present invention, the degree of protein hydrolysis is 0.1 to 10%, preferably 0.1 to 5%, even more preferably 0.1 to 2.5%.

In order to form the solution comprising one or more plant-based protein(s), it may be necessary to apply physical stimulus to the protein / solvent system mixture to enable dissolution of the protein. Suitable physical stimulus includes heating, ultrasonication, agitation, high-shear mixing, high-shear homogenisation or other physical techniques. A preferred technique is heating, optionally with subsequent ultrasonication.

Preferably, the protein / solvent system mixture is subjected to a physical stimulus which is heating, wherein the solution is heated to about or above 70°C. More preferably, the protein / solvent system mixture is heated to about or above 75°C, about or above 80°C, about or above 85°C or about 90°C. Even more preferably, the protein / solvent system mixture is heated to 85°C.

Preferably, the protein / solvent system mixture is subjected to a physical stimulus which is heating for a period of about 5 minutes, 10 minutes, 15 minutes, 20 minutes, 25 minutes, 30 minutes, or greater than 30 minutes. The heated protein / solvent system mixture is optionally subjected to subsequent ultrasonication.

In preferred methods of the present invention, in step (b) the protein solution is heated to a first temperature above the sol-gel transition temperature of the one or more plant-based protein(s) solution, then reduced to a second temperature below the sol-gel transition temperature of the one or more plant-based protein(s) solution to form a hydrogel.

The protein solution is heated such that the liquid solution is held above the sol-gel transition for the protein(s). By modifying the solvent system (for example through selection of the choice of organic acid, the ratio of organic acid to further solvent or through further means) it is possible to modify the sol-gel transition temperature for the protein(s). Through appropriate selection of conditions, it is possible to carefully control the sol-gel transition of the protein thereby controlling the formation of the hydrogel.

Preferably, the protein solution is heated to about or above 70°C. More preferably, the protein is heated to about or above 75°C, about or above 80°C, about or above 85°C or about 90°C. Even more preferably, the protein is heated to 85°C.

The protein solution may be held at elevated temperature for a time period of about 5 minutes, 10 minutes, 15 minutes, 20 minutes, 25 minutes, 30 minutes, 45 minutes or 1 hour. A preferred time period is at least 30 minutes to enable the proteins to fully solubilise. It is possible to hold the protein solution at an elevated temperature for a longer period of time.

Having heated the protein solution to above the sol-gel transition temperature, the temperature of the protein solution can be reduced to a second temperature below the sol-gel transition temperature to facilitate formation of the hydrogel. The second temperature may be room temperature. The second temperature may be in the range 5 to 25°C, preferably 10 to 20°C. The protein solution may be held at the reduced temperature for long periods of time, e.g. days, weeks prior to performing the shear treatment in step (c). The protein solution may be held at the reduced temperature for a time period of about 5 minutes, 10 minutes, 15 minutes, 20 minutes, 25 minutes or about 30 minutes. A particular reduced time period is about 5 minutes.

The particular temperatures will depend on the properties of the protein source, the solvent conditions used and therefore the sol-gel transition temperature. Alternatively, the elevated and reduced temperatures may be relatively fixed (for example about 85°C then about room temperature) and the co-solvent mixture conditions are adjusted to ensure a suitable sol-gel transition temperature for the selected plant-based protein.

Without wishing to be bound by theory, it is believed that when the plant protein is added to the solvent system the plant protein forms a dispersion of insoluble colloidal protein aggregates. Aggregate size may be measured by Dynamic Light Scattering (DLS). Suitable apparatus to measure aggregate size is a Zetasizer Nano S (Malvern).

It is believed that upon heating the protein solution in the presence of a co-solvent system to above the sol-gel transition temperature, the plant proteins partially unfold, resulting in the exposure of hydrophobic amino acids initially buried within the protein native structure. Once partially unfolded, the co-solvents are able to interact with the unfolded protein molecules. For example, an organic acid has greater access to protonate amino acid residues, as well as enabling the formation of anion salt bridges that stabilise hydrophobic interactions. Also, upon heating at elevated temperatures, protein-protein non-covalent intermolecular contacts are disrupted.

Further, it is believed that the application of mechanical agitation, for example ultrasonication, disrupts large colloidal protein aggregates into smaller ones, as well as disrupting protein intermolecular interactions. Using this approach, the size of the protein aggregates can be significantly reduced, before gelation, to particle sizes below 100nm. Preferably, the method described above comprises protein aggregates with an average size less than 200nm, preferably less than 150nm, less than 125nm, less than 100nm, less than 90nm, less than 80nm, less than 70nm, less than 60nm, less than 50nm, less than 40nm, or less than 30nm. It is therefore thought that at this stage in the method the plant-based protein(s) have protein secondary structures with high levels of α-helix and random coil.

Further, it is believed that upon cooling the protein solution to below the sol-gel transition temperature, protein-protein non-covalent intermolecular contacts are enabled, thus promoting the self-assembly of plant protein molecules into a hydrogel of interconnected protein aggregates.

After gelation, the aggregates may be fine stranded. The aggregates may have a median average length of between 50 to 500nm. The aggregates may have a mean average length of between 50 to 500nm. 80% of the aggregates may have an average length of between 50 to 500nm. The aggregates may have a median height of between 5 to 50nm. The aggregates may have a mean average height of between 5 to 50nm. 80% of the aggregates may have an average height of between 5 to 50nm. In a preferred embodiment, the aggregates have a median average length of between 50 to 500nm and/or a median average height of between 5 to 50nm.

It is believed that the method of the present invention allows the plant proteins to aggregate into supramolecular structures held by intermolecular hydrogen bonding interactions, and in particular between the β-strands.

The methods of the present invention enable materials to be formed in which there are high levels of β-sheet intermolecular interactions. Thus, in methods of the present invention, the plant-based protein(s) have a protein secondary structure with at least 40% intermolecular β-sheet, at least 50% intermolecular β-sheet, at least 60% intermolecular β-sheet, at least 70% intermolecular β-sheet, at least 80% intermolecular β-sheet, or at least 90% intermolecular β-sheet, wherein the % intermolecular β-sheet content is measured by **FTIR** (Fourier transform infrared spectroscopy).

In preferred methods of the present invention, the plant-based protein hydrogel is subjected to a solvent reduction step, preferably a solubilising solvent reduction step, between step (b) and step (c).

By solubilising solvent, we mean a solvent or mixture of solvents in which the plant-based protein hydrogel dissolves. Examples include organic acids such as acetic acid, lactic acid, formic acid, propionic acid, an α-hydroxy acid and/or a β-hydroxy acid. The α-hydroxy acid may preferably be selected from glycolic acid, acetic acid, lactic acid, malic acid, citric acid and/or tartaric acid. The β-hydroxy acid may preferably be selected from β-hydroxypropionic acid, β-hydroxybutyric acid, β-hydroxy β-methylbutyric acid, 2-hydroxybenzoic acid and carnitine.

In preferred methods of the present invention, said solvent reduction step involves:
(i) contacting the plant-based protein hydrogel with a non-solubilising solvent;
(ii) separating the plant-based hydrogel from the non-solubilising solvent to give a washed plant-based protein hydrogel; and
(iii) optionally repeating steps (i) and (ii).

Step (i) involves contacting the plant-based protein hydrogel with a non-solubilising solvent. By non-solubilising solvent, we mean a solvent or mixture of solvents in which the plant-based protein hydrogel does not dissolve. Examples include water or a mixture of water and ethanol.

In preferred methods of the present invention, said shear treatment comprises one step (i.e. a single shear step). The single shear step may be a higher shear step. Preferably, said single shear step involves fragmenting the plant-based protein hydrogel into fragments.

In preferred methods of the present invention, said fragments produced in the single shear step have a d₅₀ as determined by laser diffraction of 0.5 to 50 microns, preferably 1 to 40 microns, preferably 2 to 30 microns.

In preferred methods of the present invention, said single shear step involves ultrasonication (e.g. using equipment such as a Bandelin HD4200 or a Hielscher UIP1000hdT), high-shear mechanical stirring (e.g. using equipment such as a Silverson rotor-stator high-shear mixer), high-pressure homogenisation, or cavitation, preferably ultrasonication.

In preferred methods of the present invention, the single shear step is conducted at a temperature that is below the sol-gel transition temperature of the plant-based protein solution. In preferred methods of the present invention, said first shear step is conducted for a duration of at least 5 minutes, more preferably at least 1 minute.

In preferred methods of the present invention, said shear treatment comprises two steps. Preferably, said shear treatment comprises a first shear step followed by a second shear step. The first shear step may be a lower shear step and the second shear step may be a higher shear step.

In preferred methods of the present invention, said first shear step involves fragmenting the plant-based protein hydrogel into fragments. Preferably, at least 50 wt% of said fragments produced in said first shear step have a particle dimension in the range 1 mm to 100 mm, preferably 1 mm to 50 mm, preferably 5 mm to 30 mm, more preferably 10 mm to 30 mm. More preferably, at least 80 wt% of said fragments produced in said first shear step have a particle dimension in the range 1 mm to 100 mm, preferably 1 mm to 50 mm, preferably 5 mm to 30 mm, more preferably 10 mm to 30 mm. This can be measured by optical microscopy, or visually.

In preferred methods of the present invention, the first shear step is conducted at a temperature that is below the sol-gel transition temperature of the plant-based protein solution.

In preferred methods of the present invention, said first shear step involves mechanical cutting. By mechanical cutting, we mean cutting using a knife edge (e.g. a knife, an extruder blade etc.)

In alternative preferred methods of the present invention, said first shear step involves extrusion. For example, the plant-based protein solution formed in step (a) can be extruded into a non-solubilising solvent (e.g. water) to form the plant-based protein hydrogel in large discrete fragments, e.g. the large discrete fragments may take the form of extrudates having a thread or string form. In this way, the fragments can be directly subjected to a solvent reduction step, as described in more detail below. A first shear step of this nature is more amenable to large scale processing. In this case, the first shear step may reduce the at least one dimension of the large fragment to between 1mm and 100mm, for example a diameter of the extrudate. Preferably, at least 50 wt% of said fragments produced in said first shear step have at least one internal dimension in the range 1 mm to 100 mm, preferably 1 mm to 50 mm, preferably 5 mm to 30 mm, more preferably 10 mm to 30 mm. More preferably, at least 80 wt% of said fragments produced in said first shear step have at least one internal dimension in the range 1 mm to 100 mm, preferably 1 mm to 50 mm, preferably 5 mm to 30 mm, more preferably 10 mm to 30 mm. This can be measured by optical microscopy, or visually.

In preferred methods of the present invention, said second shear step involves further fragmenting the plant-based protein hydrogel. Preferably, said fragments produced in the second shear step have a d₅₀ as determined by laser diffraction of 0.5 to 50 microns, preferably 1 to 40 microns, preferably 2 to 30 microns.

In preferred methods of the present invention, the particle size distribution of the hydrogel fragments in the plant-based protein hydrogel slurry can be adjusted by varying the nature and the intensity of the second shear step. In another preferred method, the particle size distribution of the hydrogel fragments in the plant-based protein hydrogel slurry can be adjusted by blending or combining two or more different hydrogel slurries that have been subjected to different second shear steps and having different particle size distributions.

In preferred methods of the present invention, said second shear step is conducted at a temperature that is below the sol-gel transition temperature of the plant-based protein(s).

In preferred methods of the present invention, said second shear step is conducted at a temperature that is below the protein denaturation temperature of the plant-based protein(s).

In preferred methods of the present invention, said second shear step is conducted for a duration of at least 5 minutes, more preferably at least 1 minute.

In preferred methods of the present invention, said second shear step involves ultrasonication (e.g. using equipment such as a Bandelin HD4200 or a Hielscher UIP1000hdT), high-shear mechanical stirring (e.g. using equipment such as a Silverson rotor-stator high-shear mixer), high pressure homogenisation, or cavitation, preferably ultrasonication.

In preferred methods of the present invention, step (c) further comprises subjecting the plant-based protein hydrogel slurry to a solvent reduction step, preferably a solubilising solvent reduction step, between said first shear step and said second shear step.

In preferred methods of the present invention, said solvent reduction step comprises the steps of:
(i) contacting the fragments of the plant-based protein hydrogel slurry with a non-solubilising solvent;
(ii) separating the fragments of the plant-based protein hydrogel slurry from the non-solubilising solvent to give a washed plant-based protein hydrogel; and
(iii) optionally repeating steps (i) and (ii).

Solubilising solvents are as defined above.

Step (i) involves contacting the fragments of the plant-based protein hydrogel slurry with a non-solubilising solvent. Non-solubilising solvents are as defined above.

In preferred methods of the present invention, step (ii) involves mesh filtration or centrifugation. More preferably, step (ii) involves mesh filtration using multiple meshes of decreasing size.

As would be understood by a skilled person, if the fragments produced in the first shear step are too small the solvent reduction step can prove difficult as the fragments can end up blocking the meshes or the collected yield is low. However, if the fragments produced in the first shear step are too large, the solvent reduction step can take excessive amounts of time due to the slow mass transport of solvent from the core of the fragments.

Without wishing to be bound by theory, it is thought that due to the porous nature of the hydrogel, the solvent reduction step can remove some or all of the solvent (e.g. organic acid) from the hydrogel via a solvent exchange.

The strength of a protein hydrogel can be altered by varying the concentration of protein and organic acid, amongst other variables.

It is useful for the strength of the hydrogel used to form the hydrogel slurries to be within certain limits. This can be measured by oscillatory rheometry. A suitable measure of hydrogel strength is the storage modulus, G', of the hydrogel. Suitable test conditions are 1% strain at an oscillatory frequency of 1 Hz at 20 °C. Suitable equipment is an Anton Paar MCR 92 Rheometer with a 50mm diameter, 1 degree angle cone and plate measurement geometry.

Thus, in preferred methods of the present invention, prior to washing said plant-based protein hydrogel has a storage modulus (G') at 10 rad/s of greater than 1000Pa, preferably greater than 2000Pa, more preferably greater than 5000Pa, even more preferably greater than 6000Pa, most preferably greater than 8000Pa. As would be understood by a skilled person, 2π rad/s is equivalent to 1 Hz.

In preferred methods of the present invention, prior to washing said plant-based protein hydrogel has a storage modulus (G') at 10 rad/s of less than 20,000Pa, preferably less than 15,000Pa, more preferably less than 10,000Pa.

In preferred methods of the present invention, prior to washing said plant-based protein hydrogel has a storage modulus (G') at 10 rad/s of between about 1000 to 20,000Pa, preferably between about 2000 to 15,000Pa, more preferably between about 2000 to 10,000Pa.

Further, in preferred methods of the present invention, the washed plant-based protein hydrogel has a storage modulus (G') at 10 rad/s of greater than 200Pa, preferably greater than 250Pa, more preferably greater than 300Pa, even more preferably greater than 350Pa, more preferably greater than 400Pa, more preferably greater than 450Pa.

In preferred methods of the present invention, the washed plant-based protein hydrogel has a storage modulus (G') at 10 rad/s of less than 5000Pa, preferably less than 2500Pa, preferably less than 1000Pa, preferably less than 950Pa, more preferably less than 900Pa, more preferably less than 850Pa, more preferably less than 800Pa, more preferably less than 750Pa.

In preferred methods of the present invention, the washed plant-based protein hydrogel has a storage modulus (G') at 10 rad/s of between about 200 to 5000Pa, between about 200 to 2500Pa, between about 200 to 1000Pa, between about 250 to 950Pa, between about 300 to 900Pa, between about 350 to 850Pa, between about 400 to 800Pa, or between about 450 to 750Pa.

Preferred methods of the present invention further comprise adding an additional ingredient to the plant-based protein hydrogel slurry in step (c). Preferably, said additional ingredient is selected from plasticisers, surfactants, rheology modifiers, opacifiers, preservatives, pigments, carbohydrates, gums, polymers, and nanoparticles, or mixtures thereof.

In preferred methods of said present invention, said additional ingredient is a plasticiser. Preferably, said plasticiser is selected from glycerol, ethylene glycol, , triethylene glycol, tetraethylene glycol, polyethylene glycol, propylene glycol, sorbitol, mannitol, xylitol, fatty acids, glucose, mannose, fructose, sucrose, lactic acid, citric acid, glycolic acid, malic acid, gluconic acid, tartaric acid, ethanolamine, urea, triethanolamine, vegetable oils, lecithin, waxes and amino acids.

Preferred methods of the present invention further comprise a step of altering the pH of the plant-based protein hydrogel slurry such that it is different to the isoelectric point of the plant-based protein by more than 1 pH unit.

Preferably, said step of altering the pH of the plant-based protein hydrogel slurry is carried out after step (c). Alternatively, said step of altering the pH of the plant-based protein hydrogel slurry is carried out sequentially with step (c).

During adjustment of the pH of the plant-based protein hydrogel slurry, it is possible for the slurry to pass through the isoelectric point of the protein. Due to the lack of charge repulsion at the isoelectric point, the dispersed protein fragments in the plant-based protein hydrogel slurry can quickly coagulate. To avoid this, pH modification materials can be used to rapidly change the pH and therefore minimise the time that the slurry is at the isoelectric point. The isoelectric point of a particular plant-based protein can be determined using the method described in Helmick et al., Food Biophysics (2021) 16:474-483.

Thus, in preferred methods of the present invention, said step of altering the pH of the plant-based protein hydrogel slurry involves adding a pH-modification material to the plant-based protein hydrogel slurry. Preferably, said pH-modification material is a solution comprising monovalent metal ions, divalent metal ions or ammonium ions, preferably an aqueous alkaline solution comprising monovalent metal ions, divalent metal ions or ammonium ions. More preferably, said pH-modification material is an aqueous hydroxide solution, preferably sodium hydroxide, potassium hydroxide, or ammonium hydroxide.

In preferred methods of the present invention, the pH of the plant-based protein hydrogel slurry after said step of altering the pH of the plant-based protein hydrogel slurry is below the isoelectric point of the plant-based protein by at least 1 pH unit.

In preferred methods of the present invention, the pH of the plant-based protein hydrogel slurry after said step of altering the pH of the plant-based protein hydrogel slurry is above the isoelectric point of the plant-based protein by at least 1 pH unit.

As would be understood by a skilled person, the addition of an additional ingredient(s) to the plant-based protein hydrogel slurry in step (c) may impact the pH of the slurry. As such, the step of altering the pH of the plant-based protein hydrogel slurry preferably takes place after the addition of any additional ingredient(s).

In preferred methods of the present invention, the composition formed in step (d) is a shear thinning composition.

In preferred methods of the present invention, the composition formed in step (d) has a viscosity in the range 1 to 10000 cP at 20°C and 50 s⁻¹, preferably in the range 10 to 7500 cP at 20°C and 50 s⁻¹, preferably in the range 15 to 5000 cP at 20°C and 50 s⁻¹.

In preferred methods of the present invention, the composition formed in step (d) has a protein solids content in the range 1 wt% to 25 wt% based upon the total weight of the composition , preferably 2 wt% to 20 wt%, more preferably 4 wt% to 15 wt%, even more preferably 5 wt% to 12 wt%. The term "protein solids" refers to the dried dispersed hydrogel solids that originate from the plant protein added in step (a). The plant protein is typically a plant protein isolate that is mainly composed of material chemically identifiable as protein but also includes a low level of other insoluble plant materials such as fibres. The % of protein solids is measured according to the method described herein.

In preferred methods of the present invention, the active ingredient(s) is selected from a vitamin, a mineral, a flavour material, a fragrance material, a pro-flavour, a pro-fragrance, a flavour enhancer, a malodour counteractant, a nutraceutical, a live organism (e.g. a probiotic), a pharmaceutical, an anti-microbial agent, an anti-viral agent, an anti-inflammatory agent, a pesticide, a herbicide, a fertiliser, a fungicide, an insecticide, an animal repellent, an anti-acne agent, a skin lightening agent, an emollient, a skin moisturizing agent, a wrinkle control agent, a fabric softener active, a surface cleaning active, a skin conditioning agent, a hair conditioning agent, a dye, a pigment, and an adhesive, or combinations thereof.

In particularly preferred methods of the present invention, the active ingredient(s) is at least one fragrance material or flavour material. Preferably, the at least one fragrance material or flavour material is selected from an alcohol, an aldehyde, a ketone, an ester, an ether, an acetate, an alkene, a nitrile, a nitrogenous heterocyclic compound, a sulfurous heterocyclic compound, and a Schiff base.

The fragrance materials and flavour materials employed in the present invention may be of natural origin (i.e. they are extracted from a natural source and are not synthetically modified in any way). Preferred fragrance materials or flavour materials of natural origin include nutmeg extract, cardamon extract, ginger extract, cinnamon extract, patchouli oil, geranium oil, orange oil, mandarin oil, orange flower extract, cedarwood, vetyver, lavandin, ylang extract, tuberose extract, sandalwood oil, bergamot oil, rosemary oil, spearmint oil, peppermint oil, lemon oil, lavender oil, citronella oil, chamomile oil, clove oil, sage oil, neroli oil, labdanum oil, eucalyptus oil, verbena oil, mimosa extract, narcissus extract, jasmine extract, olibanum extract, rose extract, vanillin, coffee extract, hop oil or combinations thereof. Preferably, the fragrance materials or flavour materials of natural origin are plant-derived. The fragrance materials or flavour materials of natural origin may be used alone, or in combination, or in combination with synthetic fragrance materials.

In preferred methods of the present invention, the at least one fragrance material or flavour material has a vapour pressure greater than or equal to 0.00001 Torr at 25 °C.

In preferred methods of the present invention, the at least one fragrance material or flavour material has a logP greater than or equal to 3.0, preferably greater than or equal to 3.5, more preferably greater than or equal to 4.0.

In preferred methods of the present invention, the at least one fragrance material or flavour material has at least two Hansen solubility parameters selected from: an atomic dispersion force (δD) from 14 to 20, a dipole moment (δP) of less than 8, and a hydrogen bonding (δH) from 2.5 to 11.

In preferred methods of the present invention, the at least one fragrance material or flavour material is part of a fragrance or flavour.

Preferably, the fragrance or flavour contains at least 20 wt% of fragrance material(s) or flavour material(s) with a logP greater than or equal to 3.0 based upon the total weight of the fragrance or flavour, more preferably greater than or equal to 3.5, more preferably greater than or equal to 4.0.

Preferably, the fragrance or flavour contains at least 40 wt% of fragrance material(s) or flavour material(s) with a logP greater than 3.0 based upon the total weight of the fragrance or flavour, more preferably greater than 3.5, more preferably greater than 4.0.

Preferably, the fragrance or flavour contains at least 50 wt% of fragrance material(s) or flavour material(s) with a logP greater than 3.0 based upon the total weight of the fragrance or flavour, more preferably greater than 3.5, more preferably greater than 4.0.

Preferably, the fragrance or flavour contains at least 60 wt% of fragrance material(s) or flavour material(s) with a logP greater than 3.0 based upon the total weight of the fragrance or flavour, more preferably greater than 3.5, more preferably greater than 4.0.

Preferably, the fragrance or flavour contains at least 10 wt% of fragrance material(s) or flavour material(s) of natural origin based upon the total weight of the fragrance or flavour, preferably at least 30 wt%, more preferably at least 50 wt%, more preferably at least 70 wt%.

Preferably, the fragrance or flavour contains at least 10 wt% of fragrance material(s) or flavour material(s) which have a biodegradation percentage based upon O₂ consumption as measured according to ISO-14851 version 2019 after 28 days of 60 to 100% based upon the ratio of the Biological Oxygen Demand (BOD) to the Theoretical Oxygen Demand, more preferably 65%, even more preferably 70 to 100%, even more preferably 75 to 100%, even more preferably 80 to 100%, even more preferably 85 to 100%, most preferably 90 to 100%. Due to their low aqueous solubility, fragrance or flavours can be added to the biodegradation test on an inert support according to the method in ISO 10634 version 2018: Water quality - Preparation and treatment of poorly water-soluble organic compounds for the subsequent evaluation of their biodegradability in an aqueous medium. In addition the fragrance or flavour can be checked for any inhibitory effect on the microorganisms as specified in the method.

Preferably, the fragrance or flavour contains at least 80 wt%, preferably at least 90 wt%, more preferably at least 95 wt% of fragrance material(s) or flavour material(s) having at least two Hansen solubility parameters selected from: an atomic dispersion force (δD) from 14 to 20, a dipole moment (δP) of less than 8, and a hydrogen bonding (δH) from 2.5 to 11, based upon the total weight of the fragrance or flavour.

Preferably, the fragrance or flavour contains only low levels of materials with an alcohol functionality (e.g. a primary alcohol functionality). In preferred methods of the present invention, the fragrance or flavour comprises less than 40 %wt alcohol-containing material based upon the total weight of the fragrance or flavour, more preferably less than 20 %wt. In particularly preferred methods of the present invention, the fragrance or flavour does not comprise an alcohol-containing material. Without wishing to be bound by theory, it is thought that alcohols, and particularly primary alcohols having a straight chain alkyl group, can easily diffuse through the shell of a microcapsule due to their structure, meaning that they can be difficult materials to encapsulate.

Preferably, the fragrance or flavour material is of high odour impact. This is advantageous as it ensures that even low levels of fragrance are perceived when released from the microcapsules.

In alternative particularly preferred methods of the present invention, the active ingredient(s) is a vitamin or a mineral. Preferably, said active ingredient(s) is a vitamin or a mineral selected from Vitamin A, Vitamin B1, Vitamin B2, Vitamin B3, Vitamin B5, Vitamin B6, Vitamin B7, Vitamin B9, Vitamin B12, Vitamin C, Vitamin D, Vitamin **E,** Vitamin K, magnesium, sodium, potassium, zinc, iron, calcium, iodine, flaxseed oil, omega-3 fatty acid, folic acid, thiamin, riboflavin, niacin and phosphorous, or mixtures thereof. More preferably, said active ingredient is Vitamin D.

The vitamins and minerals employed in the present invention may be sourced from a vitamin-containing and/or mineral-containing material. An example of such a material is seaweed.

In alternatively preferred methods of the present invention the active ingredient is an agrochemical, such as a pesticide, a herbicide, a fertiliser, a fungicide, an insecticide, an animal repellent, or combinations thereof.

Preferably, the agrochemicals are a natural alternative to synthetic materials and are based on plant extracts and/or plant essential oils (EOs) or components of essential oils, such as thymol. Preferably, the agrochemical is a biopesticide. Preferably, the agrochemicals are suitable for use in formulations for plant care and production that can be certified as organic by organisations, such as the USDA (US Department of Agriculture) or Ecocert in Europe.

In preferred methods of the present invention, the active ingredient(s) is part of a composition comprising the active ingredient(s) and an active carrier phase. Preferably, the active carrier phase is a solvent, a fat or a wax.

In preferred methods of the present invention, the active carrier phase is a solvent. Preferably, the solvent is a solvent with low volatility (e.g. having a vapour pressure of less than 0.1 Torr at 25°C, preferably less than 0.01 Torr at 25°C, preferably less than 0.001 Torr at 25°C).

Preferably, the solvent has low or no odour.

Preferably, the solvent has at least two Hansen solubility parameters selected from: an atomic dispersion force (δD) of less than 20, a dipole moment (δP) of less than 8, and a hydrogen bonding (δH) of less than 11. More preferably, the solvent has at least two Hansen solubility parameters selected from: an atomic dispersion force (δD) of less than 20, a dipole moment (δP) of less than 4, and a hydrogen bonding (δH) of less than 5.

Preferably, the solvent has a density of greater than 1.07g/cm³. Solvents having this property are advantageously able to prevent creaming of the encapsulates (e.g. in a final product formulation).

Preferably, the solvent contains only low levels of materials with an alcohol functionality (e.g. a primary alcohol functionality). In preferred methods of the present invention, the solvent comprises less than 40 %wt alcohol-containing material based upon the total weight of the solvent, more preferably less than 20 %wt. In particularly preferred methods of the present invention, the solvent does not comprise an alcohol-containing material.

In preferred methods of the present invention, the active carrier phase is a solvent selected from a carboxylic acid ester, a fatty acid ester, a phthalate ester, a triol, a diol, a rosin resin, an isoparaffin, a terpene, and a vegetable oil, or combinations thereof.

Preferably, the solvent is selected from Miglyol^{®} 840, Miglyol^{®} 812 N, Miglyol^{®} 829, Miglyol^{®} 829 ECO, Miglyol^{®} Coco 810, Miglyol^{®} 810 N, Miglyol^{®} 128, Miglyol^{®} 808, Miglyol^{®} T-C7, Miglyol^{®} 8810, Miglyol^{®} PPG 810, Miglyol^{®} OE, Miglyol^{®} DO, and Miglyol^{®} 818, Abalyn^{®}, limonene, benzyl benzoate, diethyl phthalate, isopropyl myristate, triethyl citrate, dipropylene glycol, and propylene glycol, triacetin, glycerin, 1,3 propanediol or combinations thereof, preferably Miglyol^{®} 812 N.

Preferably, the solvent is a vegetable oil selected from coconut oil, corn oil, canola oil, cottonseed oil, olive oil, palm oil, peanut oil, rapeseed oil, safflower oil, sesame oil, soybean oil, and sunflower oil. Other examples of vegetable oils are given in the CTFA Cosmetic Ingredient Handbook, J.M. Nikitakis (ed.), 1st ed., The Cosmetic, Toiletry and Fragrance Association, Inc., Washington, 1988. A vegetable oil is an oil that comes from plant sources. Alternatively, the solvent is derived from a vegetable oil.

In preferred methods of the present invention, the active carrier phase is a fat or wax having a melting point of less than 60°C, preferably less than 45°C, preferably less than 25°C.

Preferably, the wax is selected from Softisan^{®} 100, Softisan^{®} 142, and Softisan^{®} 154, or combinations thereof, preferably Softisan^{®} 100.

In the methods of the present invention, the method used to form the composition in step (d) is not particularly limited. For example, step (d) may involve membrane emulsification, mechanical stirring (e.g. low or high shear mechanical stirring), ultrasonication, high-shear mechanical stirring, high-pressure homogenisation and/or cavitation.

In preferred methods of the present invention, step (d) is conducted at a temperature in the range 30°C to 50°C, preferably 35°C to 45°C.

In preferred methods of the present invention, the composition formed in step (d) comprises droplets of said active ingredient(s) or said composition comprising the active ingredient(s) and an active carrier phase having a d₅₀ as determined by laser diffraction of 0.5 to 50 microns, preferably 1 to 40 microns, preferably 2 to 30 microns.

In preferred methods of the present invention, in step (d) an additive is added to the composition, preferably a water-soluble additive.

Preferably, said additive is selected from plasticisers, surfactants, rheology modifiers, opacifiers, preservatives, pigments, carbohydrates, gums, polymers, and nanoparticles, or mixtures thereof.

In preferred methods of the present invention, the additive is a plasticiser. Preferably, the additive is selected from glycerol, ethylene glycol, , triethylene glycol, tetraethylene glycol, polyethylene glycol, propylene glycol, sorbitol, mannitol, xylitol, fatty acids, glucose, mannose, fructose, sucrose, lactic acid, citric acid, glycolic acid, malic acid, gluconic acid, tartaric acid, ethanolamine, urea, triethanolamine, vegetable oils, lecithin, waxes and amino acids.

In preferred methods of the present invention, the additive is a polysaccharide, preferably maltodextrin or OSA starch. The use of polysaccharide additives allows for control over the composition of the matrix of the resultant microcapsule. For example, the use of polysaccharides means that the amount of plant-based protein(s) can be controlled.

In preferred methods of the present invention, the water-soluble additive is a water-soluble vitamin, preferably Vitamin C. The presence of additives, such as water-soluble vitamins, means that the final dried microcapsule is able to deliver the additive in addition to the encapsulated active ingredient(s) during end use, e.g. upon enzymatic degradation of the plant-based protein hydrogel matrix in the oral cavity or digestive tract of a human or animal.

In preferred methods of the present invention the weight ratio in the composition formed in step (d) of plant-based protein(s) to the composition comprising the active ingredient(s) and an active carrier phase is in the range 20:1 to 1:20.

In preferred methods of the present invention, the composition formed in step (d) is a homogenous composition.

In preferred methods of the present invention, the composition formed in step (d) is a two-phase system (e.g. an aqueous two-phase system).

In the methods of the present invention, step (e) involves drying the composition formed in step (d) to form a microcapsule or an agglomerate of microcapsules or larger bodies. A skilled person will be familiar with drying techniques and would know how to choose the necessary conditions to achieve dried microcapsules or larger dried bodies. Suitable drying techniques include spray drying as used herein, electrostatic spray drying, , spray freeze drying and spray vacuum drying wherein the slurry is sprayed into a chamber under reduced pressure such that solvent can be removed at lower temperature, thus avoiding issues of thermal degradation. Advantageously, in the methods of the present invention, step (e) can be conducted at relatively low temperatures (e.g. at temperatures less than 100°C) under spraying conditions to form fine slurry droplets. This aids the formation of small-sized microcapsules, and also prevents the loss or degradation of any volatile or sensitive materials being encapsulated.

Alternative or additional drying techniques that can be used either independently or in combination with spray-drying to evaporate the solvents from the composition of step (d) include fluidised bed drying, film drying, drum drying, belt drying, conduction drying, infrared drying or a combination thereof. The composition of step (d) can be dispersed and at least partially dried by spray-drying to form microcapsules which are then further dried by secondary processes, such as fluid-bed drying. The extended drying times typical of fluid-bed drying typically allow for lower drying temperatures to be used. Some designs of spray-dryer incorporate this two-stage drying process as an integral part of their design. Some fluid bed drying processes, which typically comprises spraying or dispersing the composition of step (d) onto or into a fluidised bed of particles, for example encapsulates of already dried composition (d), are preferred when larger encapsulates are required. The composition can be sprayed onto the fluidised bed or can be sprayed within the fluidised bed. Depending on process conditions, the composition spray will agglomerate and/or coat (and hence build up) passing microcapsules. One example of a fluidised bed coating and drying process is the Wurster-type of powder coater. The skilled person will be readily able to define conditions for control of encapsulate size from a fluid-bed drying process.

Other drying processes can be selected depending on the required form of the dried composition from step (e). Film drying, wherein the composition is cast as a thin film on a heated belt or drum, can be used to form aesthetic shapes comprising dried composition (d). Belt drying, which can use conduction heating or infra-red heating, can be used in combination with other drying techniques to finish off the drying under controlled conditions.

During drying in step (e), the plant-based protein hydrogel slurry forms a plant-based protein carrier that encapsulates the active ingredient(s) in the form of a microcapsule. During drying in step (e), at least part of both the first and second co-solvent are evaporated to enable the microcapsule to form. This evaporation does not occur at the same rate for the two co-solvents so that during the process the slurry will typically become more concentrated in the least volatile co-solvent. The microcapsules formed during drying can still contain both first and second co-solvents trapped within the dry microcapsules and their ratio can be different to their starting ratio in the solution in step (a).

In preferred methods of the present invention, the biodegradation percentage based upon O₂ consumption of the plant-based protein carrier as measured according to ISO-14851 version 2019 after 28 days is 60 to 100% based upon the ratio of the Biological Oxygen Demand (BOD) to the Theoretical Oxygen Demand, more preferably 65 to 10%, even more preferably 70 to 100%, even more preferably 75 to 100%, even more preferably 80 to 100%, even more preferably 85 to 100%, most preferably 90 to 100%. ISO-14851 version 2019 describes a method whereby the biological oxygen demand in a closed respirometer is used to determine the degree of biodegradation of materials in a natural aqueous environment. This is achieved by exposure of the material under lab conditions in an aqueous standard test medium to an inoculum from unadapted activated sludge that has not been pre-exposed. The measured value is calculated as a percentage of the theoretical oxygen demand calculated from the molecular formula. An internal reference of microcrystalline cellulose is also tested and the test is valid if its biodegradation % is greater than 60% at the end of the test.

In preferred methods of the present invention, the biodegradation percentage based upon O₂ consumption of the microcapsules as measured according to ISO-14851 version 2019 after 28 days is 60 to 100% based upon the ratio of the Biological Oxygen Demand (BOD) to the Theoretical Oxygen Demand, more preferably 65 to 10%, even more preferably 70 to 100%, even more preferably 75 to 100%, even more preferably 80 to 100%, even more preferably 85 to 100%, most preferably 90 to 100%. ISO-14851 version 2019 describes a method whereby the biological oxygen demand in a closed respirometer is used to determine the degree of biodegradation of materials in a natural aqueous environment. This is achieved by exposure of the material under lab conditions in an aqueous standard test medium to an inoculum from unadapted activated sludge that has not been pre-exposed. The measured value is calculated as a percentage of the theoretical oxygen demand calculated from the molecular formula. An internal reference of microcrystalline cellulose is also tested and the test is valid if its biodegradation % is greater than 60% at the end of the test.

In preferred methods of the present invention, step (e) involves electrostatic spray drying.

In preferred methods of the present invention, plant-based protein residues formed during step (e) but not incorporated into the microcapsule are recycled, preferably being added back into step (a).

In preferred methods of the present invention, the microcapsule has a d₅₀ as determined by laser diffraction of less than or equal to 250 µm, less than or equal to 200 µm, less than or equal to 150 µm, less than or equal to 100 µm, less than or equal to 50 µm, less than or equal to 30 µm, less than or equal to 10 µm.

In preferred methods of the present invention, the microcapsule has a diameter as determined by optical microscopy of less than or equal to 250 µm, less than or equal to 200 µm, less than or equal to 150 µm, less than or equal to 100 µm, less than or equal to 50 µm, less than or equal to 30 µm, less than or equal to 10 µm.

Preferred methods of the present invention further comprise subjecting the microcapsule to a post-treatment step. Preferably, the post-treatment step comprises a non-covalent cross-linking step, a covalent cross-linking step or a coating formation step.

In preferred methods of the present invention, the post-treatment step comprises a non-covalent cross-linking step. Preferably, the non-covalent cross-linking step comprises treating the microcapsule with a non-covalent cross linker selected from sodium tripolyphosphate (NaTPP), sodium hexametaphosphate, and phenolic compounds (e.g. tannic acid, caffeic acid etc.).

In preferred methods of the present invention, the post-treatment step comprises a covalent cross-linking step. Preferably, the covalent cross-linking step comprises treating the microcapsule with a covalent cross linker selected from genipin, epoxy compounds, glyceraldehyde, glutaraldehyde, formaldehyde, glyoxal, dialdehyde starch, microbial transglutaminase, and PolyCup^{®} crosslinking resin, or combinations thereof.

In preferred methods of the present invention, the post-treatment step comprises a coating formation step.

In preferred methods of the present invention, the coating formation step comprises treating the microcapsule with a metal compound. Preferably, the metal compound is selected from a silver compound or a gold compound, preferably a silver compound.

In preferred methods of the present invention, the coating formation step comprises subjecting the microcapsule to a complex coacervation step using a polysaccharide. Preferably, the polysaccharide is selected from xanthan gum, gellan gum, and chitosan, or combinations thereof.

In preferred methods of the present invention, the coating formation step comprises treating the microcapsule with an aqueous mineral solution to form a mineral coating. Preferably, the aqueous mineral solution comprises iron salts, calcium salts, phosphate salts, carbonate salts, titanium salts or zinc salts, or combinations thereof.

As will be understood by a skilled person, it is possible for multiple post-treatment steps to be performed. For example, in a preferred method of the present invention, the microcapsule is subjected to a non-covalent cross-linking step (e.g. using NaTPP) followed by a coating formation step (e.g. using chitosan), and optionally a further non-covalent cross-linking step (e.g. using NaTPP).

Preferred methods of the present invention further comprise adding a flow aid to the microcapsule after step (e). Preferably, the flow aid is fumed silica. The addition of a flow aid can avoid clumping of the newly formed microcapsules.

Preferred methods of the present invention comprise further drying the microcapsule after step (e). Preferably, the further drying is selected from fluid bed drying and/or tray drying.

Preferred methods of the present invention further comprise re-suspending the microcapsule in an external phase, preferably an external aqueous phase, more preferably hard water or an acidic buffer solution.

Preferred methods of the present invention further comprise sieving the microcapsule, e.g. for size classification.

The present invention also provides a biodegradable microcapsule obtained by or obtainable by the method as hereinbefore described.

The present invention also provides a method for the preparation of a biodegradable microcapsule composition, the method comprising:
(a) preparing a biodegradable microcapsule according to the method as hereinbefore described; and
(b) suspending the biodegradable microcapsule in an external phase.

Preferably, said external phase is an external aqueous phase, preferably hard water or an acidic buffer solution.

Preferred methods of the present invention further comprise adding a suspending agent(s) to said external phase.

Preferably, the suspending agent(s) is selected from acacia gum, alginic acid, pectin, xanthan gum, gellan gum, carbomer, dextrin, gelatin, guar gum, hydrogenated vegetable oil category 1, aluminum magnesium silicate, maltodextrin, carboxymethyl cellulose, polymethacrylate, poly vinyl pyrrolidone, sodium alginate, starch, zein, water-insoluble cross-linked polymers such as cross-linked cellulose, cross-linked starch, cross-linked CMC, cross-linked carboxymethyl starch, cross-linked polyacrylate, and cross-linked polyvinylpyrrolidone, and expanded clays such as bentonite and laponite.

The present invention also provides a biodegradable microcapsule composition obtained by or obtainable by the method as hereinbefore described.

The present invention also provides a biodegradable microcapsule comprising an active ingredient(s) and a plant-based protein carrier comprising a plant-based protein(s), wherein the plant-based protein carrier encapsulates the active ingredient(s), and wherein the plant-based protein has a solubility of less than 50% when measured at a protein concentration of 5% w/w in an aqueous solution at pH 7 and 25°C.

Protein solubility is determined using the following protocol: a known amount of dried microcapsules are added to an aqueous solution, which is then centrifuged to separate the soluble and insoluble fractions. After centrifugation, the liquid supernatant (i.e. the soluble fraction) is removed without obtaining any of the solids that precipitate at the bottom (i.e. the insoluble fraction). The resultant supernatant is analysed for nitrogen content. Protein content in the supernatant is then calculated based on nitrogen content using the factor of 6.25. Protein solubility is defined as the amount of protein in the supernatant divided by the amount of protein in the whole aqueous solution.

In preferred biodegradable microcapsules of the present invention, the plant-based protein has a solubility of less than 30% when measured at a protein concentration of 5% w/w in an aqueous solution at pH 7 and 25°C, more preferably a solubility of less than 10%.

In preferred biodegradable microcapsules of the present invention, at least 25%, more preferably at least 40%, even more preferably at least 50%, even more preferably at least 60% of the active ingredient(s) initially encapsulated remains present inside the microcapsule after incubation in water at 20°C for 10 days, as determined by high-performance liquid chromatography (HPLC). In alternative preferred biodegradable microcapsules of the present invention, at least 25%, more preferably at least 40%, even more preferably at least 50%, even more preferably at least 60% of the active ingredient(s) initially encapsulated remains present inside the microcapsule after incubation in water at 20°C for 10 days, as determined by gas chromatography (GC).

More preferably, said water has been acidified such that it has a pH of less than 4.0, more preferably less than 3.0, more preferably less than 2.0, more preferably less than 1.0. Thus, the biodegradable microcapsules of the present invention are insoluble in water and are also stable under strongly acidic conditions. This has the advantage that the biodegradable microcapsules of the present invention can be used in a variety of applications in which such harsh conditions are prevalent. For example, the biodegradable microcapsules can be added to beverage formulations, which are generally acidic, in order to deliver an active ingredient (e.g. a vitamin or a mineral) to the consumer. The acid stability of the biodegradable microcapsules of the present invention also means that, once consumed, the microcapsules can survive the harsh acidic conditions of the stomach, such that the active ingredient(s) will not be released until it is in the small intestine and can be better absorbed. Thus, the biodegradable microcapsules of the present invention can provide controlled release of the encapsulated active ingredient(s). This is because the plant-based protein hydrogel matrix acts as an enteric coating.

In preferred biodegradable microcapsules of the present invention, the plant-based protein(s) is obtained from fava bean, mung bean, pea, rice, potato, rapeseed, lentil, chickpea, sunflower seed, pumpkin seed, flax, chia, canola, lupine, alfalfa, moringa, wheat, corn zein or sorghum; preferably the plant protein(s) is selected from pea protein, potato protein, rapeseed protein, lentil protein, chickpea protein, fava bean protein, mung bean protein, sunflower seed protein, pumpkin seed protein, flax protein, chia protein, canola protein, lupine protein, alfalfa protein, moringa protein and/or rice protein. More preferably, the plant-based protein is pea protein and/or potato protein. Such proteins are considered to be low allergenicity proteins.

In preferred biodegradable microcapsules of the present invention, the plant-based protein(s) has been pre-treated with an organic acid. Preferably, the organic acid is acetic acid, lactic acid, formic acid, propionic acid, an α-hydroxy acid and/or a β-hydroxy acid. Particularly preferably, the organic acid is acetic acid and/or lactic acid.

Preferred α-hydroxy acids include glycolic acid, lactic acid, acetic acid, malic acid, citric acid and/or tartaric acid, more preferably lactic acid or acetic acid. Preferred β-hydroxy acids include β-hydroxypropionic acid, β-hydroxybutyric acid, β-hydroxy β-methylbutyric acid, 2-hydroxybenzoic acid and carnitine.

In preferred biodegradable microcapsules of the present invention, the active ingredient(s) is selected from a vitamin, a mineral, a flavour material, a fragrance material, a pro-favour, a pro-fragrance, a flavour enhancer, a malodour counteractant, a nutraceutical, a live organism (e.g. a probiotic), a pharmaceutical, an anti-microbial agent, an anti-viral agent, an anti-inflammatory agent, a pesticide, a herbicide, a fertiliser, a fungicide, an insecticide, an animal repellent, an anti-acne agent, a skin lightening agent, an emollient, a skin moisturizing agent, a wrinkle control agent, a fabric softener active, a surface cleaning active, a skin conditioning agent, a hair conditioning agent, a dye, a pigment, and an adhesive, or combinations thereof.

In particularly biodegradable microcapsules of the present invention, the active ingredient(s) is at least one fragrance material or flavour material. Preferably, the at least one fragrance material or flavour material is selected from an alcohol, an aldehyde, a ketone, an ester, an ether, an acetate, an alkene, a nitrile, a nitrogenous heterocyclic compound, a sulfurous heterocyclic compound, and a Schiff base.

The fragrance materials and flavour materials employed in the present invention may be of natural origin (i.e. they are extracted from a natural source and are not synthetically modified in any way). Preferred fragrance materials or flavour materials of natural origin include nutmeg extract, cardamon extract, ginger extract, cinnamon extract, patchouli oil, geranium oil, orange oil, mandarin oil, orange flower extract, cedarwood, vetyver, lavandin, ylang extract, tuberose extract, sandalwood oil, bergamot oil, rosemary oil, spearmint oil, peppermint oil, lemon oil, lavender oil, citronella oil, chamomile oil, clove oil, sage oil, neroli oil, labdanum oil, eucalyptus oil, verbena oil, mimosa extract, narcissus extract, jasmine extract, olibanum extract, rose extract, vanillin, coffee extract, hop oil or combinations thereof. Preferably, the fragrance materials or flavour materials of natural origin are plant-derived. The fragrance materials or flavour materials of natural origin may be used alone, or in combination, or in combination with synthetic fragrance materials.

In preferred biodegradable microcapsules of the present invention, the at least one fragrance material or flavour material has a vapour pressure greater than or equal to 0.00001 Torr at 25°C.

In preferred biodegradable microcapsules of the present invention, the at least one fragrance material or flavour material has a logP greater than or equal to 3.0, preferably greater than or equal to 3.5, more preferably greater than or equal to 4.0.

In preferred biodegradable microcapsules of the present invention, the at least one fragrance material or flavour material has at least two Hansen solubility parameters selected from: an atomic dispersion force (δD) from 14 to 20, a dipole moment (δP) of less than 8, and a hydrogen bonding (δH) from 2.5 to 11.

In preferred biodegradable microcapsules of the present invention, the at least one fragrance material or flavour material is part of a fragrance or flavour.

Preferably, the fragrance or flavour contains at least 20 wt% of fragrance material(s) or flavour material(s) with a logP greater than or equal to 3.0 based upon the total weight of the fragrance or flavour, more preferably greater than or equal to 3.5, more preferably greater than or equal to 4.0.

Preferably, the fragrance or flavour contains at least 40 wt% of fragrance material(s) or flavour material(s) with a logP greater than 3.0 based upon the total weight of the fragrance or flavour, more preferably greater than 3.5, more preferably greater than 4.0.

Preferably, the fragrance or flavour contains at least 50 wt% of fragrance material(s) or flavour material(s) with a logP greater than 3.0 based upon the total weight of the fragrance or flavour, more preferably greater than 3.5, more preferably greater than 4.0.

Preferably, the fragrance or flavour contains at least 60 wt% of fragrance material(s) or flavour material(s) with a logP greater than 3.0 based upon the total weight of the fragrance or flavour, more preferably greater than 3.5, more preferably greater than 4.0.

Preferably, the fragrance or flavour contains at least 10 wt% of fragrance material(s) or flavour material(s) of natural origin based upon the total weight of the fragrance or flavour, preferably at least 30 wt%, more preferably at least 50 wt%, more preferably at least 70 wt%.

Preferably, the fragrance or flavour contains at least 10 wt% of fragrance material(s) or flavour material(s) which have a biodegradation percentage based upon O₂ consumption as measured according to ISO-14851 version 2019 after 28 days of 60 to 100% based upon the ratio of the Biological Oxygen Demand (BOD) to the Theoretical Oxygen Demand, more preferably 65%, even more preferably 70 to 100%, even more preferably 75 to 100%, even more preferably 80 to 100%, even more preferably 85 to 100%, most preferably 90 to 100%. Due to their low aqueous solubility, fragrance or flavours can be added to the biodegradation test on an inert support according to the method in ISO 10634 version 2018: Water quality - Preparation and treatment of poorly water-soluble organic compounds for the subsequent evaluation of their biodegradability in an aqueous medium. In addition the fragrance or flavour can be checked for any inhibitory effect on the microorganisms as specified in the method.

Preferably, the fragrance or flavour contains at least 80 wt%, preferably at least 90 wt%, more preferably at least 95 wt% of fragrance material(s) or flavour material(s) having at least two Hansen solubility parameters selected from: an atomic dispersion force (δD) from 14 to 20, a dipole moment (δP) of less than 8, and a hydrogen bonding (δH) from 2.5 to 11, based upon the total weight of the fragrance or flavour.

Preferably, the fragrance or flavour contains only low levels of materials with an alcohol functionality (e.g. a primary alcohol functionality. In preferred biodegradable microcapsules of the present invention, the fragrance or flavour comprises less than 40 %wt alcohol-containing material based upon the total weight of the fragrance or flavour, more preferably less than 20 %wt. In particularly preferred biodegradable microcapsules of the present invention, the fragrance or flavour does not comprise an alcohol-containing material.

Preferably, the fragrance or flavour material is of high odour impact. This is advantageous as it ensures that even low levels of fragrance are perceived when released from the microcapsules.

In alternative particularly preferred biodegradable microcapsules of the present invention, the active ingredient(s) is a vitamin or a mineral. Preferably, said active ingredient(s) is a vitamin or a mineral selected from Vitamin A, Vitamin B1, Vitamin B2, Vitamin B3, Vitamin B5, Vitamin B6, Vitamin B7, Vitamin B9, Vitamin B12, Vitamin C, Vitamin D, Vitamin **E,** Vitamin K, magnesium, sodium, potassium, zinc, iron, calcium, iodine, flaxseed oil, omega-3 fatty acid, folic acid, thiamin, riboflavin, niacin and phosphorous, or mixtures thereof. More preferably, said active ingredient is Vitamin D.

The vitamins and minerals employed in the present invention may be sourced from a vitamin-containing and/or mineral-containing material. An example of such a material is seaweed.

In preferred biodegradable microcapsules of the present invention, the active ingredient(s) is part of a composition comprising the active ingredient(s) and an active carrier phase. Preferably, the active carrier phase is a solvent, a fat or a wax.

In preferred biodegradable microcapsules of the present invention, the active carrier phase is a solvent. Preferably, the solvent is a solvent with low volatility (e.g. having a vapour pressure of less than 0.1 Torr at 25°C, preferably less than 0.01 Torr at 25°C, preferably less than 0.001 Torr at 25°C).

Preferably, the solvent has low or no odour.

Preferably, the solvent has at least two Hansen solubility parameters selected from: an atomic dispersion force (δD) of less than 20, a dipole moment (δP) of less than 8, and a hydrogen bonding (δH) of less than 11. More preferably, the solvent has at least two Hansen solubility parameters selected from: an atomic dispersion force (δD) of less than 20, a dipole moment (δP) of less than 4, and a hydrogen bonding (δH) of less than 5.

Preferably, the solvent has a density of greater than 1.07g/cm³. Solvents having this property are advantageously able to prevent creaming of the encapsulates (e.g. in a final product formulation).

Preferably, the solvent contains only low levels of materials with an alcohol functionality (e.g. a primary alcohol functionality). In preferred biodegradable microcapsules of the present invention, the solvent comprises less than 40 %wt alcohol-containing material based upon the total weight of the solvent, more preferably less than 20 %wt. In particularly preferred biodegradable microcapsules of the present invention, the solvent does not comprise an alcohol-containing material.

In preferred biodegradable microcapsules of the present invention, the active carrier phase is a solvent selected from a carboxylic acid ester, a fatty acid ester, a phthalate ester, a triol, a diol, a rosin resin, an isoparaffin, a terpene, and a vegetable oil, or combinations thereof.

Preferably, the solvent is selected from Miglyol^{®} 840, Miglyol^{®} 812 N, Miglyol^{®} 829, Miglyol^{®} 829 ECO, Miglyol^{®} Coco 810, Miglyol^{®} 810 N, Miglyol^{®} 128, Miglyol^{®} 808, Miglyol^{®} T-C7, Miglyol^{®} 8810, Miglyol^{®} PPG 810, Miglyol^{®} OE, Miglyol^{®} DO, and Miglyol^{®} 818, Abalyn^{®}, limonene, benzyl benzoate, diethyl phthalate, isopropyl myristate, triethyl citrate, dipropylene glycol, and propylene glycol, triacetin, glycerin, 1,3 propanediol or combinations thereof, preferably Miglyol^{®} 812 N.

Preferably, the solvent is a vegetable oil selected from coconut oil, corn oil, canola oil, cottonseed oil, olive oil, palm oil, peanut oil, rapeseed oil, safflower oil, sesame oil, soybean oil, and sunflower oil. Other examples of vegetable oils are given in the CTFA Cosmetic Ingredient Handbook, J.M. Nikitakis (ed.), 1st ed., The Cosmetic, Toiletry and Fragrance Association, Inc., Washington, 1988. Alternatively, the solvent is derived from a vegetable oil.

In preferred biodegradable microcapsules of the present invention, the active carrier phase is a fat or a wax having a melting point of less than 60 °C, preferably less than 45°C, preferably less than 25°C.

Preferably, the wax is selected from Softisan^{®} 100, Softisan^{®} 142, and Softisan^{®} 154, or combinations thereof, preferably Softisan^{®} 100.

In preferred biodegradable microcapsules of the present invention, the plant-based protein carrier further comprises an additive, preferably a water-soluble additive.

Preferably, said additive is selected from plasticisers, surfactants, rheology modifiers, opacifiers, preservatives, pigments, carbohydrates, gums, polymers, and nanoparticles, or mixtures thereof.

In preferred biodegradable microcapsules of the present invention, the additive is a plasticiser. Preferably, the additive is selected from glycerol, ethylene glycol, , triethylene glycol, tetraethylene glycol, polyethylene glycol, propylene glycol, sorbitol, mannitol, xylitol, fatty acids, glucose, mannose, fructose, sucrose, lactic acid, citric acid, glycolic acid, malic acid, gluconic acid, tartaric acid, ethanolamine, urea, triethanolamine, vegetable oils, lecithin, waxes and amino acids.

In preferred biodegradable microcapsules of the present invention, the additive is a polysaccharide, preferably maltodextrin or OSA starch. The use of polysaccharide additives for allows control over the composition of the matrix of the resultant microcapsule. For example, the use of polysaccharides means that the amount of plant-based protein(s) can be controlled.

In preferred biodegradable microcapsules of the present invention, the water-soluble additive is a water-soluble vitamin, preferably Vitamin C. The presence of additives, such as water-soluble vitamins, means that the final microcapsule is able to deliver the additive in addition to the encapsulated active ingredient(s) during end use, e.g. upon enzymatic degradation of the plant-based protein hydrogel matrix in the oral cavity or digestive tract of a human or animal.

In preferred biodegradable microcapsules of the present invention, the microcapsule has a d₅₀ as determined by laser diffraction of less than or equal to 250 µm, less than or equal to 200 µm, less than or equal to 150 µm, less than or equal to 100 µm, less than or equal to 50 µm, less than or equal to 30 µm, less than or equal to 10 µm.

In preferred biodegradable microcapsules of the present invention, the microcapsule has a diameter as determined by optical microscopy of less than or equal to 250 µm, less than or equal to 200 µm, less than or equal to 150 µm, less than or equal to 100 µm, less than or equal to 50 µm, less than or equal to 30 µm, less than or equal to 10 µm.

In preferred biodegradable microcapsules of the present invention, the plant-based protein(s) have a protein secondary structure with at least 40% intermolecular β-sheet, at least 50% intermolecular β-sheet, at least 60% intermolecular β-sheet, at least 70% intermolecular β-sheet, at least 80% intermolecular β-sheet, or at least 90% intermolecular β-sheet, wherein the % intermolecular β-sheet content is measured by **FTIR.**

Preferred biodegradable microcapsules of the present invention do not contain or do not substantially contain a cross-linking agent.

In alternative preferred biodegradable microcapsules of the present invention, the plant-based protein carrier has been non-covalently modified by a non-covalent cross-linker. Preferably, the non-covalent cross linker is selected from sodium tripolyphosphate, sodium hexametaphosphate, and phenolic compounds (e.g. tannic acid, caffeic acid etc.).

In alternative preferred biodegradable microcapsules of the present invention, the plant-based protein carrier has been covalently modified by a covalent cross-linker. Preferably, the non-covalent cross-linker is selected from genipin, epoxy compounds, glyceraldehyde, glutaraldehyde, formaldehyde, glyoxal, dialdehyde starch, microbial transglutaminase, and PolyCup^{®} crosslinking resin, or combinations thereof.

In preferred biodegradable microcapsules of the present invention, the plant-based protein carrier has a coating deposited thereon. Preferably, the coating is a metal coating, a coacervate coating or a mineral coating.

In preferred biodegradable microcapsules of the present invention, the metal coating is a silver coating or a gold coating, preferably a silver coating.

In preferred biodegradable microcapsules of the present invention, the coacervate coating is formed from a polysaccharide. Preferably, the polysaccharide is selected from xanthan gum, gellan gum, and chitosan, or combinations thereof.

In preferred biodegradable microcapsules of the present invention, the mineral coating is formed from an aqueous mineral solution. Preferably, the aqueous mineral solution comprises iron salts, calcium salts, phosphate salts, carbonate salts, titanium salts or zinc salts, or combinations thereof.

Preferred biodegradable microcapsules of the present invention have a coating deposited on the covalently-modified or non-covalently modified plant-based protein matrix. Preferred features of the coatings are as described above.

In particularly preferred biodegradable microcapsules of the present invention, the plant-based protein is pea protein and the active ingredient is Vitamin D.

In preferred biodegradable microcapsules of the present invention, the biodegradation percentage based upon O₂ consumption of the plant-based protein carrier as measured according to ISO-14851 version2019 after 28 days is 60 to 100% based upon the ratio of the Biological Oxygen Demand (BOD) to the Theoretical Oxygen Demand, more preferably 65 to 100%, even more preferably 70 to 100%, even preferably 75 to 100%, even more preferably 80 to 100%, even more preferably 85 to 100%, most preferably 90 to 100%. ISO-14851 version 2019 describes a method whereby the biological oxygen demand in a closed respirometer is used to determine the degree of biodegradation of materials in a natural aqueous environment. This is achieved by exposure of the material under lab conditions in an aqueous standard test medium to an inoculum from unadapted activated sludge that has not been pre-exposed. The measured value is calculated as a percentage of the theoretical oxygen demand calculated from the molecular formula. An internal reference of microcrystalline cellulose is also tested and the test is valid if its biodegradation % is greater than 60% at the end of the test.

In preferred biodegradable microcapsules of the present invention, the biodegradation percentage based upon O₂ consumption of the microcapsule as measured according to ISO-14851 version2019 after 28 days is 60 to 100% based upon the ratio of the Biological Oxygen Demand (BOD) to the Theoretical Oxygen Demand, more preferably 65 to 100%, even more preferably 70 to 100%, even preferably 75 to 100%, even more preferably 80 to 100%, even more preferably 85 to 100%, most preferably 90 to 100%. ISO-14851 version 2019 describes a method whereby the biological oxygen demand in a closed respirometer is used to determine the degree of biodegradation of materials in a natural aqueous environment. This is achieved by exposure of the material under lab conditions in an aqueous standard test medium to an inoculum from unadapted activated sludge that has not been pre-exposed. The measured value is calculated as a percentage of the theoretical oxygen demand calculated from the molecular formula. An internal reference of microcrystalline cellulose is also tested and the test is valid if its biodegradation % is greater than 60% at the end of the test.

The present invention also provides a composition comprising a biodegradable microcapsule as hereinbefore described and an external phase.

Preferably, said external phase is an external aqueous phase, preferably hard water or an acidic buffer solution.

Preferred compositions of the present invention further comprise a suspending agent(s) in the external phase.

Preferably, the suspending agent(s) is selected from acacia gum, alginic acid, pectin, xanthan gum, gellan gum, carbomer, dextrin, gelatin, guar gum, hydrogenated vegetable oil category 1, aluminium magnesium silicate, maltodextrin, carboxymethyl cellulose, polymethacrylate, poly vinyl pyrrolidone, sodium alginate, starch, zein, water-insoluble cross-linked polymers such as cross-linked cellulose, cross-linked starch, cross-linked CMC, cross-linked carboxymethyl starch, cross-linked polyacrylate, and cross-linked polyvinylpyrrolidone, and expanded clays such as bentonite and laponite.

The present invention also provides a formulated product comprising a biodegradable microcapsule as hereinbefore described.

The present invention also provides a method of making a formulated product, comprising:
(a) preparing a biodegradable microcapsule according to the method as hereinbefore described; and
(b) mixing said biodegradable microcapsule with a product formulation.

Preferably, the formulated product is a food, beverage, cosmetic, home care product, personal care product, pharmaceutical, industrial product (e.g. paint, adhesive, sandpaper, tape etc.), medical device, biomaterial, or agrochemical.

The present invention also provides the use of a biodegradable microcapsule as hereinbefore described in a formulated product.

Preferably, the formulated product is a food, beverage, cosmetic, home care product, personal care product, pharmaceutical, industrial product (e.g. paint, adhesive, sandpaper, tape etc.), medical device, biomaterial, or agrochemical.

The formulated product described in various aspects of the present invention may take any form suitable for use. Preferably, the formulated product is in the form of a vapour spray, aerosol, emulsion, lotion, liquid, cream, gel, stick, ointment, paste, mousse, powder, granular product, substrate, or semi-solid.

### BRIEF DESCRIPTION OF THE FIGURES

Figures 1a and 1b are 10X optical microscope photographs of the microcapsules of Examples 2B and 2E, respectively.
Figures 2a and 2b are 10X optical microscope photographs of the microcapsules of Examples 4A and 4B, respectively.
Figure 3 is a 10X optical microscope image of Example 5 control in water
Figures 4a and 4b are SEM images of the microcapsules of Example 6A at a magnification of x80 and x300, respectively.
Figures 5a, 5b and 5c are SEM images of the microcapsules of Example 6B at a magnification of x60, x500, and x1250, respectively.
Figure 6 is an SEM image of the microcapsules of Example 7 at a magnification of x1200.
Figures 7a and 7b are 10X optical microscope images of the microcapsules of Example 7 before (Figure 7a) and after (Figure 7b) pressing them to release the oil.
Figures 8a, 8b and 8c are 10X microscope images of the concentrated slurry of Example 10A, 10B and Comparative Example 10C.
Figure 9 is a 10X optical microscope image of the microcapsules of Example 15 in single strength citrate buffer.
Figure 10 is a photograph of the 4 inverted tubes of Example 16.

### EXAMPLES

### Materials

Pea Protein Isolate (PPI) (80% protein, 4 wt% carbohydrate - ProEarth P16109) was purchased from Cambridge Commodities Ltd.
Lactic acid (85%, Food Grade) was purchased from Fisher Scientific.
Acetic acid (glacial 99%) was purchased from Fisher Scientific.
Sodium benzoate was purchased from Fisher Scientific.
Vitamin D2 Oil (1MlU/g) was purchased from Prinova Europe, UK.
Miglyol^{®} 812N was purchased from IOI Oleochemical.
Softisan^{®} 100 was purchased from IOI Oleochemical.
Vitamin D2 powder, VITAMIN D2 (ERGOCALCIFEROL) 100,000 IU/G SD, Gum Acacia based, was obtained from Prinova Europe, UK.
Robinsons Double Strength No Added Sugar Squash Apple & Blackcurrant (manufactured by Britvic Plc and commercially available from supermarkets in the United Kingdom).
Miwadi Single concentrate Blackcurrant (manufactured by Britvic Plc and commercially available from supermarkets in the Republic of Ireland).
Thymol was purchased from Fisher Scientific, UK
Calcium sulphate dihydrate (CaSO₄.2H₂O) was purchased from Fisher Scientific.
Magnesium sulphate dried (MgSO₄) was purchased from Fisher Scientific.
Potassium Chloride, Extra Pure, SLR, Eur. Ph., (KCl) was purchased from Fisher Scientific.
Sodium hydrogen carbonate, certified AR for analysis (NaHCO₃) was purchased from Fisher Scientific.
Potassium hydroxide (KOH) (>85%) was purchased from Sigma Aldrich.
Ethanol Absolute 99.8+%, (EtOH) certified AR for analysis was purchased from Fisher Scientific.
Maltodextrin was purchased from Sigma-Aldrich Gillingham, UK
Polysorbate 80 was purchase from Sigma-Aldrich Gillingham, UK
All components for Phosphate buffered saline (PBS) (pH 7.4) were purchased from Fisher Scientific, UK, and were prepared in the following ratios: 138 mM NaCl, 2.7 mM KCI, 10 mM Na₂HPO₄, 2 mM NaH₂PO₄
Sodium acetate trihydrate was purchased from Alfar Aesar, UK
Calcium chloride dihydrate was purchased from Fisher Scientific, UK
Methylparaben was purchased from Alfar Aesar, UK
Tannic acid was purchased from Fisher Scientific, UK
Gellan gum (high acyl) was purchased from Special Ingredients Ltd, UK
Flaxseed oil was purchased from Naissance, UK

### Fragrance materials

| **Common/trade name** | **CAS number** | **Supplier** |
|---|---|---|
| Delta damascone | 57378-68-4 | Augustus Oils Limited and Carvansons |
| Geraniol BJ | 106-24-1 | Carvansons |
| Undecavertol | 81782-77-6 | Carvansons |
| Clonal (dodecane nitrile) | 2437-25-4 | Carvansons |
| Dipentene | 138-86-3 | Carvansons |
| Ortho tert.-butylcyclohexyl acetate (OTBCHA) | 88-41-5 | Carvansons |
| Diphenyl oxide | 101-84-8 | Carvansons |
| Benzyl salicylate | 118-58-1 | Carvansons |

### Standard hard water and buffer compositions

### 1) Hard water

This was prepared with the following concentrations of salts in deionised (DI) water: 192mg/l of NaHCO₃, 120 mg/l CaSO₄.2H₂O, 120 mg/l MgSO₄ ,8mg/l KCI. pH was adjusted to 3 with 1M HCl.

### 2) Single strength buffer

100 ml of single strength buffer solution was prepared as follows:
i. 1.904g of citric acid monohydrate and 0.276 g of trisodium citrate dihydrate were weighed out;
ii. The mixture was completed to 80 g using DI water;
iii. The pH of the solution was adjusted to 3 using 1M HCl and 1M NaOH solutions, and the volume of the solution was completed to 100 g with DI water;
iv. 0.38 g of CaCl₂ dihydrate was added;
v. 0.2 g of sodium benzoate was added;
vi. The solution was placed in an ultrasonic bath at 80°C for 5-10 min (i.e. until complete dissolution was observed);
vii. The solution was allowed to cool down to 20 °C.

### 3) Double strength buffer

100 ml of double strength buffer solution was prepared as for single strength buffer but with twice the level of all materials but with the mixture still being completed to 80gs.

### Measurement Methods

### Vitamin D2 analysis for dry powder samples of microcapsules: Version A

The vitamin D2 is extracted from the sample into dimethyl sulfoxide and then partitioned into hexane. The vitamin D2 is quantified using normal phase HPLC with UV detection. This method is suitable for the determination of vitamin D2 at high levels, typically from 35iu/g to 500,000iu/g.

### Vitamin D2 analysis for dry powder samples of microcapsules: Version B

The powder sample (approx. 0.1 g) is suspended in 4 g of 50% w/v aqueous potassium hydroxide and 8 g of absolute ethanol. 0.1 g of sodium ascorbate is added. The sample is placed in an ultrasonic bath for 1 to 1.5 hours without heating, cooled on ice, then centrifuged at 14,500 rpm for 10 minutes before HPLC analysis on a reverse phase column using 100% methanol as eluent, and UV detection at 265 nm.

### Vitamin D2 analysis for dilute liquid systems containing microcapsules

The sample is cold saponified overnight with alcoholic potassium hydroxide (containing pyrogallol). The unsaponified fraction is separated by extraction into hexane. The extracted sample is then concentrated and injected onto a semi-preparative HPLC column. The fraction of the eluent containing the vitamin D2 is collected, evaporated to dryness, dissolved in methanol and the vitamin D2 is separated using reverse phase HPLC with UV detection. This method is suitable for determination of vitamin D2 at lower levels, typically from 0.5iu/g to 100iu/g. Lower levels can be quantified with further dilutions.

### Hydrogel slurry particle size

The particle size of the hydrogel slurry was measured using an Anton Paar laser diffraction particle size analyser PSA 1190. Measurements were carried out by diluting the hydrogel within 1 hour of finishing manufacturing in an aqueous solution with acetic acid or lactic acid adjusted to the same pH. The test material was diluted to the required concentration to ensure there are no agglomerates present and have the desired optical density (normally 5-15% obscuration) for the measurement. The d₅₀ quoted is for the volume distribution, as calculated via a general analysis using Mie theory.

### Droplet size of composition for spray drying

The droplet size of the oil or wax containing the active ingredient in the compositions used for spray drying was measured using an Anton Paar laser diffraction particle size analyser PSA 1190. Measurements were carried out by diluting the composition within 1 h of finishing manufacturing in an aqueous solution with acetic acid or lactic acid adjusted to the same pH. The test material was diluted to the required concentration to have the desired optical density (normally 5-15% obscuration) for the measurement. The d₅₀ quoted is for the volume distribution, as calculated via a general analysis using Mie theory.

### Protein solids content

The solids content of the hydrogel slurry was measured by the mass remaining on drying. Approximately 5g of the hydrogel slurry was pipetted into a small polypropylene dish and the mass was accurately recorded. The dish was placed in a 40°C oven overnight to dry. The dry mass was measured immediately after removing the dish from the oven and the solids content of the protein hydrogel slurry was calculated as a percentage of the initial wet mass.

### Viscosity of composition for spray-drying

The viscosity of the compositions for spray drying were measured using an Anton Paar MCR 92 rheometer, within 1 h of finishing manufacturing the composition. The rheometer was set up using a cone (1 degree, 50mm diameter) and plate geometry. The viscosity was measured at a temperature of 20°C and a shear rate of 50/s.

### Spray-dried microcapsule particle size

The particle size of the final microcapsule powder was measured using an Anton Paar laser diffraction particle size analyser PSA 1190. The powder was added to reverse osmosis water diluted to the required concentration in order to have the desired optical density (normally 5-15% obscuration) for the measurement. The dispersion should be checked via an optical microscope for any larger agglomerates of microcapsules. If these are visible 0.5wt% acetic acid can be added to ensure that the primary particles are well dispersed. The d₅₀ quoted is for the volume distribution, as calculated via a general analysis using Mie theory.

Alternatively, particle size can be measured using optical microscopy (e.g. using an openFrame microscope equipped with a CellCam 200CR camera, Aura Pro phase contrast illuminator and universal plan fluorite objectives at 4x, 10x and 20x). The microcapsule powder is added to either reverse osmosis water or single strength buffer as needed. In such a method, particle sizes are taken from the mean size measurements of 50 microcapsules, for each respective sample. The optical microscope is then calibrated using the grid of a Hirschmann counting chamber, (Fuchs Rosenthal). Using the straight line tool in ImageJ 1.53, particle diameters are measured from two center-edges with the overlay-text feature enabled to avoid repeating capsules.

### Scanning Electron Microscopy (SEM)

SEM images were obtained using a Hitachi TM3030.

### Optical Microscopy

Optical microscopy images were obtained using an openFrame microscope equipped with a CellCam 200CR camera, Aura Pro phase contrast illuminator and universal plan fluorite objectives at 4x, 10x and 20x. Samples were prepared for optical microscopy by first rehydrating the dried microcapsules in hard water or single strength buffer for 5 minutes before microscopy imaging. A cover slip was then placed on the top of the rehydrated sample, and images were taken. The slide was then removed from the microscope stage, and firm pressure was applied by hand to the cover slip before replacing the sample on the stage and taking images of the broken capsules.

### Total fragrance and fragrance material loading

Fragrance and fragrance material levels in the spray-dried microcapsule can be determined by extraction of the fragrance materials and injection into a GC column. Quantification is achieved by use of a calibration curve for the given fragrance material diluted in ethanol.

To measure the total level of fragrance materials in an encapsulate sample, the capsules must first be broken by sonication. For example, 10-50 mg of dried encapsulate or 0.3-0.5 g of a wet encapsulate collected on a 40 µm cell strainer in 3.0 g of deionised (DI) water and 10% KOH are vortexed to mix thoroughly. The solution is then sonicated with a Bandelin, small probe TS104 for 1-2 minutes at 30% amplitude (around 1.5-3.0 kJ in total). While sonicating, ice is used to keep the temperature below 20°C to avoid any loss of fragrance through evaporation. An optical microscope as detailed above is used to check visually if the capsules are fully broken. If not, the sonication step is repeated. Ethanol is added to the mixture, which is then mixed and centrifuged. The supernatant is collected and then diluted as appropriate and injected into the GC for analysis.

For samples containing benzyl salicylate, this is quantified by measuring the benzyl alcohol formed from its hydrolysis.

### Total thymol loading

Thymol levels in the spray-dried microcapsule was determined by extraction of the thymol and injection into a GC column as per the total fragrance loading method herein.

### Microcapsule slurry olfactive evaluation

For any given encapsulate, the total fragrance level is quantified via GC as described herein. Based on the encapsulate loading the material is diluted to deliver the appropriate level of fragrance for an olfactive assessment. Optionally, preservatives and suspending agents are added to the dilute encapsulate sample.

50 µl of dilute encapsulate is pipetted using a low shear micropipette tip onto a standard microscope glass slide so that most of the surface area is covered, leaving the edges clear for handling the slide. This is repeated at least four times.

50 µl of dilute encapsulate slurry is also pipetted onto a rectangular fragrance evaluation card blotter (approx. 9 cm x 5 cm). This is repeated at least in duplicate.

Slides and blotters were evaluated by trained evaluators at different points in time:
1) Immediately after application;
2) After being left overnight to dry at 20-25 °C;
3) After being left overnight to dry at 20-25 °C and then for glass slides rubbed with a gloved index finger with 10 strokes (in either direction);
4) After being left overnight to dry at20-25°C and then for glass slides pressed with another glass slide for 10 seconds and removed.
3) After being left overnight to dry at 20-25°C and then for blotters rubbed with another clean blotter with 10 strokes (in either direction).

The evaluators graded the strength according to the following scale and provided comments on the character of the fragrance:

| Odour strength | Description |
|---|---|
| 5 | Very strong |
| 4 | Strong |
| 3 | Moderate |
| 2 | Weak |
| 1 | Very weak |
| 0 | No odour |

### Example 1 - Preparation of protein hydrogel slurry with acetic acid

### Preparation of the protein hydrogel

800 ml of a mixture was prepared consisting of 12.5 % (w/v) Pea Protein Isolate (PPI) in 30% (v/v) acetic acid solution.

The mixture was then heated in a water bath at 85°C, followed by a sonication step to disrupt large colloidal aggregates (Hielscher UIP), after which a transparent solution was obtained. The energy applied was 200kJ.

The solution was then poured into a container to a depth of 1cm and left to cool down at 5 °C for 28 hours to obtain a self-standing protein hydrogel.

### Application of shear to protein hydrogel

Shear was then applied to the hydrogel as follows. The protein hydrogel was cut into ~1cm cubes via a low-shear cutting step. The cubes were placed inside a 75 micron filter bag, which was then submerged inside a bucket containing 10L of reverse osmosis water. This formed a coarse protein hydrogel slurry within the filter bag. The hydrogel cubes were left to soak, with occasional gentle agitation. This step was performed to reduce the concentration of acetic acid in the hydrogel by diffusion to the continuous aqueous phase.

The strained gel cubes were transferred into a bottle with 4 wt% sodium benzoate added and were exposed to high shear followed by ultra-high shear (probe sonication with a Hielscher sonicator to 200 kJ) on ice so as to form a homogeneous low-viscosity protein hydrogel slurry.

### Example 2 - Preparation of spray-dried microcapsules containing vitamin D2

### Preparation of wax/Vitamin D2 mixtures

A 22 wt% (220,000 IU/g) and a 11 wt% (110,000 IU/g) mixture was made of Vitamin D2 oil (1MIU/g) in Softisan^{®} 100 wax. The wax was melted above 40 °C, the vitamin oil added, and the mixture homogenised by hand shaking. The mixture was held above 40 °C until required.

### Example 2A: 5:1 ratio of acid-treated hydrogel slurry solids to wax, with Vitamin D2 added by sonication

1500 g of the protein hydrogel slurry was made as per Example 1, wherein the PPI and acetic acid mixture was heated for 20 minutes, and the gel cubes were soaked for 2 hours, then high sheared at 15,000 rpm for 15 minutes and then ultra-high sheared at 0.25 kj/ml to form the hydrogel slurry.

### Preparation of composition

The protein hydrogel slurry was heated to 35 °C and the 22 wt% wax/vitamin D2 molten mixture was added, such that a 98.3:1.7 by weight ratio of protein hydrogel slurry to wax/Vitamin D2 was achieved. The resultant mixture was mechanically high sheared using an Ultra-Turrax disperser at 15,000rpm for 10minutes, followed by ultra-high shearing on ice using a Hielscher sonicator applying energy of 0.1 kJ/ml. This resulted in a composition having a viscosity of 27.3 cP, and a mean droplet size, d₅₀, of 0.67 microns.

### Spray-drying of composition

The composition was spray dried according to the process settings in Table 1, resulting in microcapsules having a mean particle size, d₅₀, of 23.8 microns measured by laser diffraction. The Vitamin D2 concentration in the microcapsule powder was measured using the method version A herein described and was found to be 100,250 ug/100g.

### Example 2B: 2:1 ratio of acid-treated hydrogel slurry solids to wax, with Vitamin D2 added by mechanical mixing

1500 g of the protein hydrogel was made as per Example 1 wherein the PPI and acetic acid mixture was heated for 20 minutes and the gel cubes were soaked for 2 hours, then high sheared at 15,000 rpm for 15 minutes and then ultra-high sheared at 0.25 kj/ml to form the hydrogel slurry.

### Preparation of composition

The protein hydrogel was heated to 35 °C and the 11 wt% wax/vitamin D2 molten mixture was added, such that a 96:4 by weight ratio of protein hydrogel slurry to wax/Vitamin D2 was achieved. The resultant mixture was mechanically high sheared using an Ultra-Turrax disperser at 15,000rpm for 10minutes. This resulted in a composition with a viscosity of 31.4 cP, and a mean droplet size, d₅₀, of 4.6 microns.

### Spray-drying of composition

The slurry was spray dried according to the process settings in Table 1resulting in microcapsules having a mean particle size, d₅₀, of 35.9 microns measured by laser diffraction.. The Vitamin D2 concentration in the microcapsule powder was measured using the method version A herein described and was found to be 94,750 ug/100g.

### Example 2C: 5:1 ratio of acid-treated hydrogel slurry solids to wax, with Vitamin D2 added by mechanical mixing

1500 g of the protein hydrogel slurry was made as per Example 1, wherein the PPI and acetic acid mixture was heated for 20 minutes and the gel cubes were soaked for 2 hours, then high sheared at 15,000 rpm for 15 minutes and then ultra-high sheared at 0.25 kj/ml to form the hydrogel slurry.

### Preparation of composition

The protein hydrogel slurry was heated to 35 °C and the 22 wt% wax/vitamin D2 molten mixture was added, such that a 96:4 by weight ratio of protein hydrogel slurry to wax/Vitamin D2 was achieved. The mixture was mechanically high sheared using an Ultra-Turrax disperser at 15,000rpm for 10mins. This resulted in a composition with a viscosity of 35.8 cP, and a mean droplet size, d₅₀, of 6.2 microns.

### Spray-drying of composition

The composition was spray dried according to the process settings in Table 1. Two different settings were used, yielding samples Ci and Cii. The Ci and Cii microcapsules were each measured to have a mean particle size, d₅₀, of 31.3 microns measured by laser diffraction. The Vitamin D2 concentration in the Cii microcapsule powder was measured using the method version A herein described and was found to be 95,500 ug/100g.

### Control Example 2D: 5:1 ratio of protein solids in sonicated PPI (in 5 wt% acetic acid) to wax, with Vitamin D2 added by mechanical mixing

### Preparation of PPI mixture

A mixture of 8 wt% pea protein isolate in 5 wt% (v/v) acetic acid solution was prepared so that the solids concentration would be comparable to the protein hydrogel slurry prepared in Example 1. The mixture was ultra-high sheared using a Hielscher probe sonicator to an energy input of 1 kJ/ml. This yielded a protein mixture with a mean particle size, d₅₀, of 2.9 microns. The pH of the protein mixture was measured as 3.4.

### Preparation of composition

The protein mixture was heated to 35 °C and the 22 wt% wax/vitamin D2 molten mixture was added, such that a 98.3:1.7 by weight ratio of protein mixture to wax/Vitamin D2 was achieved. The mixture was mechanically high sheared using an Ultra-Turrax disperser at 15,000rpm for 10minutes. This resulted in a composition having a viscosity of 5.0 cP, and a mean droplet size, d₅₀, of 4.4 microns.

### Spray-drying of composition

The composition was spray dried according to the process settings in Table 1 resulting in microcapsules with a mean particle size, d₅₀, of 20.2 microns measured by laser diffraction. The Vitamin D2 concentration in the microcapsule powder was measured using the method version A herein described and was found to be 86,500 ug/100g.

### Control Example 2E: 5:1 ratio of protein solids in sonicated PPI to wax, Vitamin D added by mechanical mixing

### Preparation of PPI mixture

A slurry of 8wt% pea protein isolate in reverse osmosis water was prepared so that the solids concentration would be comparable to the protein hydrogel slurry prepared in Example 1. The mixture was ultra-high sheared using a Hielscher probe sonicator to an energy input of 1 kJ/ml. This yielded a protein mixture with a mean particle size, d₅₀, of 1.4 microns. The pH of the protein mixture was measured as 6.85.

### Preparation of composition

The protein mixture was heated to 35 °C and the 22 wt% wax/vitamin D2 molten mixture was added, such that a 98.3:1.7 by weight ratio of protein mixture to wax/Vitamin D2 was achieved. The mixture was mechanically high sheared using an Ultra-Turrax disperser at 15,000rpm for 10minutes. This resulted in a composition with a viscosity of 4.9 cP, and a mean droplet size, d₅₀, of 4.6 microns.

### Spray-drying of composition

The composition was spray dried according to the process settings in Table 1 resulting in microcapsules with a mean particle size, d₅₀, of 40.0 microns measured by laser diffraction. The Vitamin D2 concentration in the microcapsule powder was measured using the method version A herein described and was found to be 93,000 ug/100g.

### Spray drying conditions for Examples 2A to 2E

All the compositions prepared in Examples 2A to 2E were spray dried in a cocurrent spray dryer with a 1 m diameter chamber and a Spraying Systems FloMax nozzle. The inlet air flow was maintained at 1.5 kg/min and the slurry feed addition rate was adjusted within the specified range to maintain the outlet temperature. The feed properties and spray drying conditions are summarised in Table 1 below.

**Table 1**

| Sample | Weight ratio of protein to VitD/wax | % Solids in feed | Viscosity of feed at 50/s (cP) | Feed droplet size (microns) | Atomising air pressure (psi) | Feed addition rate litres/min | Inlet air temp (°C) | Air out temp (°C) |
|---|---|---|---|---|---|---|---|---|
| 2A | 5:1 | 10.5% | 27.28 | 0.67 | 40 | 3.3 - 3.5 | 160 | 80 |
| 2B | 2:1 | 12.4% | 31.4 | 4.57 | 40 | 4.5 | 160 | 80 |
| 2Ci | 5:1 | 10.4% | 35.82 | 6.16 | 40 | 3.3 - 3.5 | 180 | 90 |
| 2Cii | 5:1 | 10.4% | 35.82 | 6.16 | 40 | 3.3 - 3.5 | 160 | 80 |
| 2D | 5:1 | 9.6% | 4.95 | 4.37 | 65 | 3.3 - 3.5 | 160 | 80 |
| 2E | 5:1 | 9.6% | 4.93 | 4.62 | 40 | 3.5 | 160 | 80 |

The conditions in Table 1 demonstrate that despite the higher feed viscosities of Examples 2A, 2B and 2C of the present invention, compared to the controls 2D and 2E, the compositions were successfully spray dried with standard equipment and process conditions. This is thought to be due to the shear thinning nature of the compositions of Examples 2A, 2B and 2C of the present invention.

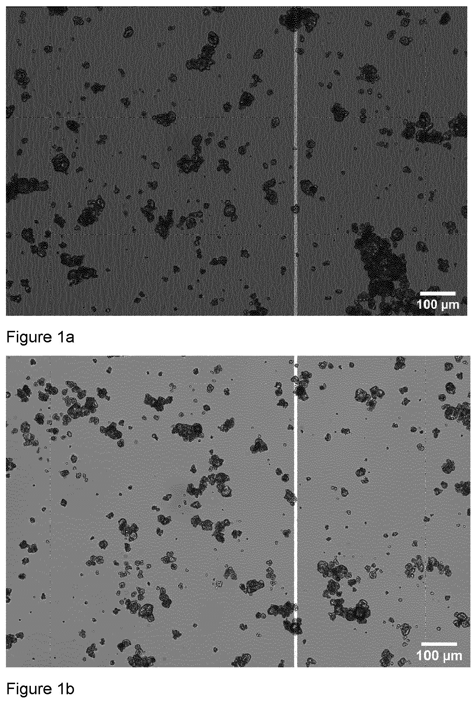

All compositions were successfully spray dried producing free flowing powders with a mean particle size, d₅₀, between 20 and 40 microns measured by laser diffraction. Figures 1a and 1b are 10X optical microscope photographs of the spray-dried microcapsules of Examples 2B and 2E, respectively, showing the visual similarity of the microcapsule powders produced. To achieve this a very much higher level of energy was used in preparing the protein dispersion of Example 2E than that of Example 2B to achieve similar emulsion droplet and microencapsulate sizes.

### Example 3 - Preparation of protein hydrogel slurry with lactic acid

### Preparation of the protein hydrogel

50.0 g pea protein isolate was added to 212.5 g of reverse osmosis water in a bottle. The bottle was shaken to disperse the protein and 187.5 ml of 85% (v/v) lactic acid was mixed in. The mixture was placed in an 85 °C water bath for 30 minutes and then ultrasonicated with a Bandelin sonicator until 100 kJ had been delivered. The hot protein solution was poured into a petri dish and allowed to cool at 5 °C overnight to obtain a self-standing protein hydrogel.

### Application of shear to protein hydrogel

The hydrogel was cut into 1 cm cubes and placed in a 75 micron nylon mesh bag in a bucket. 5 L of reverse osmosis water was added and the cubes were left to soak for approximately 1.5 hours, with occasional gentle agitation. The water was then drained from the bag and the same soaking procedure repeated three more times, until the pH was above 3.0.

The hydrogel was then drained and added to a solution of 5 wt % of sodium benzoate at 20 mls per kilo of gel (with the pH adjusted to 4-5 using lactic acid). The mixture was sonicated using a Bandelin sonicator until 0.1 kJ/ml had been applied. The solids content of the hydrogel slurry was measured to be 5.1wt% by the method described herein.

### Example 4 - Preparation of spray-dried microcapsules containing vitamin D2

### Preparation of composition

Vitamin D2 oil (1MIU/g) was diluted in Miglyol^{®} 812N or Softisan^{®} 100 wax to a concentration of 6000ug/g. This concentration was chosen to target the manufacture of spray dried particles containing a Vitamin D2 concentration of 1,000 ug/g. In the case of Softisan^{®} 100 wax, the wax was melted above 40 °C, the vitamin oil added, and the mixture homogenised by hand shaking. The mixture was held above 40 °C until required.

The required mass of oil or wax and Vitamin D2 mixture was then added to the protein hydrogel slurry made in Example 3 to achieve a mass ratio of 5:1 hydrogel slurry solids to Vitamin D2 : wax/oil mixture. A composition was formed by mixing with a IKA Ultra-Turrax for 15 minutes at 5000 rpm, followed by probe sonication to 0.1 kJ/ml with a Bandelin sonicator.

### Spray drying conditions

The spray drying was performed using a ProCept spray dryer in its three-column set-up. This gave a column height of 1.8 m with a column diameter of 0.15 m. The two-fluid nozzle was used with a tip size of 0.8mm. The atomisation air flow rate was 10ml/ml. The fluid was pumped into the spray dryer with a syringe pump at 6ml/min. Other settings are given in Table 2 below. An additional air flow of 100 I/min was set for the cyclone.

**Table 2**

| Example | Sample type | Viscosity at 50/s (cP) | Inlet air flow rate (m³/min) | Inlet air temp (°C) | Chamber out temp (°C) | Air out temp (°C) |
|---|---|---|---|---|---|---|
| 4A | Miglyol^{®} 812N oil/VitD2 | 57.6 | 0.6 | 140 | 79 | 69 |
| 4B | Softisan^{®} 100 Wax/VitD2 | 58.1 | 0.4 | 138 | 68 | 66 |

The results demonstrate that protein hydrogel slurries formed using lactic acid can also be spray dried using standard equipment and under standard conditions. The results also demonstrate that a range of different carrier materials for the active ingredient, here Vitamin D2, can be employed (e.g. oils and waxes).

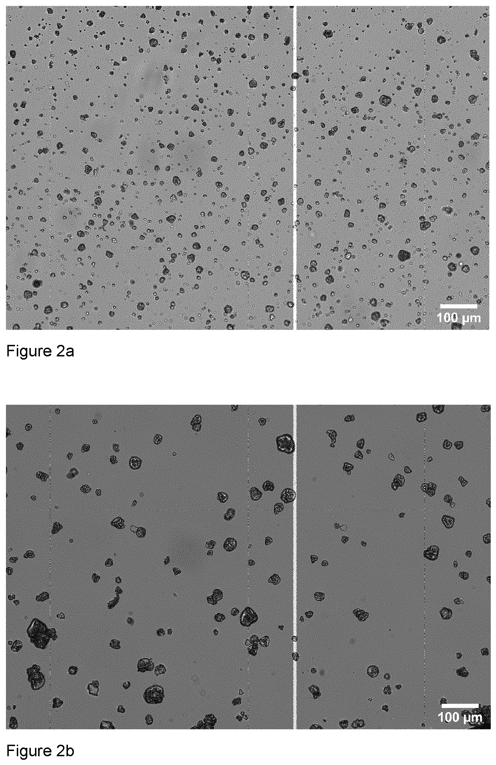

The spray drying experiments produced two free flowing powders with a mean particle size, d₅₀, of 15.8 microns for Example 4A and 32.5 microns for Example 4B measured by laser diffraction. The Vitamin D2 concentration in the microcapsule powder was measured using the method version A described herein and was found to be 89,000 µg/100g and 86,500 µg/100g in samples 4A and 4B, respectively. Figures 2a and 2b are 10X optical microscope photographs of the spray-dried microcapsules of Examples 4A and 4B, respectively, showing the visual similarity of the powders produced where the difference in particle size can be observed.

### Example 5 - Accelerated stability testing of Vitamin D2 microcapsules in a beverage formulation

### Preparation of samples

The microcapsules of Examples 4A and 4B were each added to Robinsons Double Concentrate No Added Sugar Apple & Blackcurrant squash. More specifically, 45 mg of microcapsules were added to 1 litre of squash, to give a vitamin D concentration of 4.5ug/100ml in the product, on the assumption that the vitamin D concentration in the microcapsules was 100,000ug/100g. A control was also prepared by adding 9 mg of Prinova spray dried Vitamin D2 to a sample of the same squash. Prinova spray dried Vitamin D2 is a commercially available powdered Vitamin D2 material. The three mixtures were heated to 95 °C on a hotplate, with constant stirring, and then held at 95 °C for 30 seconds to mimic the temperature profile during flash pasteurization. The mixtures were then allowed to cool to 20°C.

### Accelerated Stability Testing

200ml transparent plastic PET bottles were filled with each of the three squash samples and placed in a Binder KBF P 240-230 V chamber, which was set for accelerated ambient light stability studies with UV-A set at 1.1 W/m2 at 20°C and lab humidity (typically 50% relative humidity (RH)). Initial Vitamin D2 loading in the squash was measured using the method herein described and then again after 4 weeks in the Binder chamber. The % reduction in Vitamin D2 content over 4 weeks was calculated and is recorded in Table 3. Vitamin D2 is expected to degrade significantly on exposure to UV over this time frame.

**Table 3**

| Example | Powder Description | Vitamin D2 reduction from Week 0 to Week 4 | Vitamin D carrier |
|---|---|---|---|
| Control | Prinova spray dried Vit D2 | 95% | Unknown, likely gum acacia |
| 4A | Spray dried Vit D2 encapsulate with oil core | 54% | Miglyol^{®} 812N |
| 4B | Spray dried Vit D2 encapsulate with wax core | 0% (no reduction within method measurement error of +/- 18%) | Softisan^{®} 100 |

As can be seen from Table 3, after 4 weeks in the squash the commercially available powdered Vitamin D2 degrades almost completely with 95% of the initial Vitamin D2 degraded.

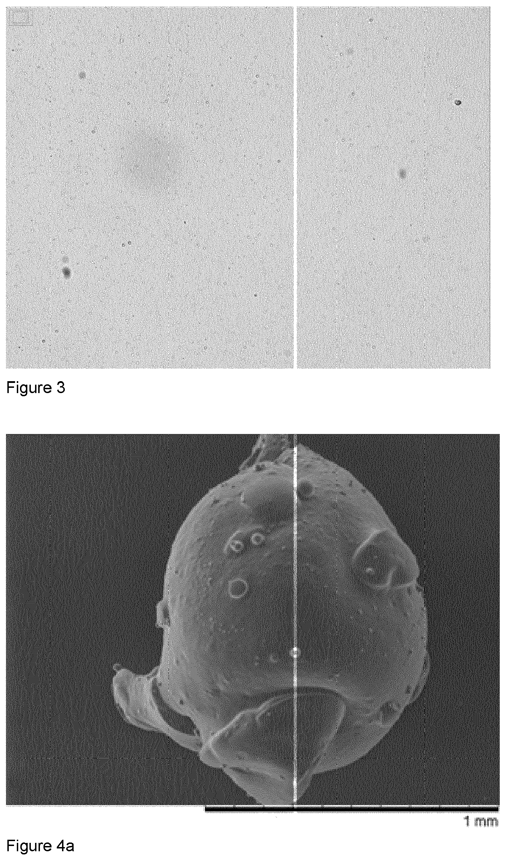

Figure 3 is a 10X microscope image of the powdered control Vitamin D2 added to cold tap water showing the presence of a fine dispersion of material and no microcapsules. Without wishing to be bound by theory, it is believed that the carrier material in this powder is highly soluble in water so that it disintegrates in water and so is not able to protect the Vitamin D2 in accelerated stability testing.

However, for Example 4A, after 4 weeks in the squash the Vitamin D2 level is still approximately half of the original level, demonstrating that this microcapsule having a plant-based protein carrier and an oil-based active carrier phase has protected it from exposure to UV and hence reduced degradation. The presence of an insoluble plant-based protein carrier clearly helped to protect the encapsulated Vitamin D2 compared to the commercially available material tested in the control experiment. Furthermore, for Example 4B, after 4 weeks in the squash the Vitamin D2 level is substantially unchanged from the original level, demonstrating that this microcapsule having a plant-based protein carrier and a wax-based active carrier phase was able to fully protect the Vitamin D2 from degradation. Without wishing to be bound by theory, it is thought that the use of a solid wax carrier for the Vitamin D2 means the material surrounding the vitamin is more opaque and therefore provides even better protection for the vitamin from UV.

### Example 6 - Preparation of spray dried fragrance microcapsules

### Preparation of fragrance/solvent mixture

A mixture was prepared of delta-damascone and Miglyol^{®} 812 in the weight ratio 80:20 by gentle mixing.

### Preparation of spray dried microcapsules

The protein hydrogel slurry was made as per Example 1, wherein the PPI and acetic acid mixture was heated for 30 minutes, the gel cubes were soaked for 1 hour then high sheared at 25,000 rpm for 15 mins using an IKA Ultra-Turrax and then ultra-high sheared using sonication (Hielscher sonicator) to 1.0 kJ/ml to form the hydrogel slurry.

The mean particle size of the hydrogel slurry, d₅₀, was measured according to the method herein described and found to be 2.5 microns. The mean solids content of the hydrogel slurry was measured according to the method herein described and was found to be 8.6wt%.

Two samples were prepared with the compositions shown in Table 4 by pouring the fragrance/solvent mixture into the hydrogel slurry and mixing with an Ultra-Turrax at 15,000 rpm for 5-15 seconds to achieve the desired dispersed droplet size of 5 µm.

**Table 4**

| Material (wt %) | Example 6A | Example 6B |
|---|---|---|
| Pea Protein | 4.9 | 5.4 |
| Delta-damascone | 36.7 | 32.2 |
| Miglyol^{®}812N | 12.4 | 10.9 |
| Water | 46.0 | 51.5 |
| Ratio PPI: Oil phase (fragrance + Miglyol) | 1:12.5 | 1:8 |

Example 6A phase easily separated to give a clear liquid top layer and a bottom layer of hydrogel fragments, but this could be made homogenous again with vigorous mixing prior to spray drying. The well mixed slurry had a viscosity of 150 cP at 50/sec.

A 1.5 mg droplet of Example 6A was suspended on a fine wire connected to a microbalance and positioned in the middle of a pipe of internal diameter 2.5 cm. Air at a temperature of 97 °C and a velocity of 0.85 m/s was passed around the droplet and the weight of the droplet was recorded over 11 minutes. At the end of the experiment, the dried droplet was carefully detached from the wire and positioned on a Hitachi TM3030 SEM mounting pad for examination.

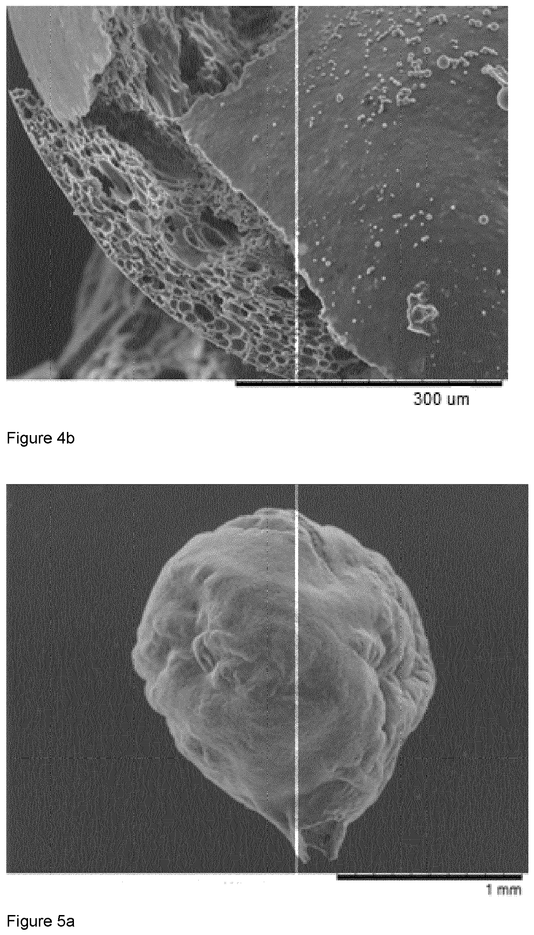

The resulting particle had an internal matrix structure and an external smooth skin as seen in the SEM images of Figures 4a and 4b.

Example 6B was a homogeneous, viscous and semi-solid with a viscosity of 1000 cP at 50/sec.

A 2.5 mg droplet of Example 6B was suspended on a fine wire connected to a microbalance and positioned in the middle of a pipe of internal diameter 2.5 cm. Air at a temperature of 85°C and a velocity of 0.85 m/s was passed around the droplet and the weight of the droplet was recorded over 3.5 minutes. At the end of the experiment, the dried droplet was carefully detached from the wire and positioned on a Hitachi TM3030 SEM mounting pad for examination.

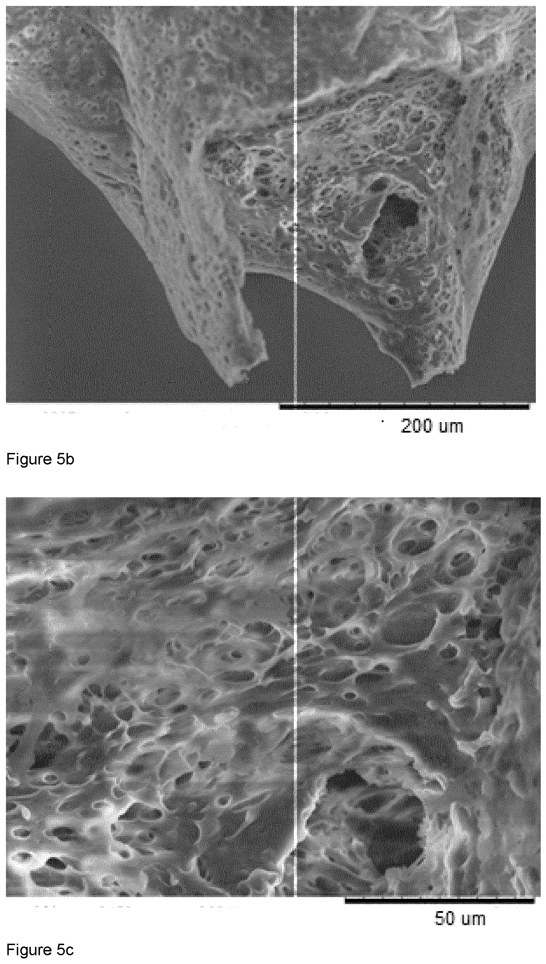

The resulting particle had a matrix structure and porous surface as can be seen in the SEM images of Figures 5a, 5b, and 5c.

### Example 7 - Preparation of spray dried fragrance microcapsules

### Preparation of fragrance/solvent mixture

A mixture was prepared of delta-damascone and Miglyol^{®} 812 in the volume ratio 80:20 by gentle mixing.

### Preparation of spray dried microcapsules

The protein hydrogel slurry was made as per Example 1. The hydrogel slurry was diluted with acetic acid to a mean protein solids content of 8.0 wt%. A 221 g batch was stirred with an UltraTurrax lab mixer at 8000 rpm for 2 minutes. 131g of the fragrance : oil mixture containing 80% delta-damascone and 20% Miglyol^{®} 812 oil was then added. The composition is given in Table 5 below.

**Table 5**

| Material (wt %) | Example 7 |
|---|---|
| Pea Protein | 5.0 |
| Delta-damascone | 29.8 |
| Miglyol^{®} 812N | 7.4 |
| Water | 57.8 |
| Ratio PPI: Oil phase (fragrance + Miglyol) | 1:7.4 |

The batch was subjected to high shear using an UltraTurrax lab mixer set to 8000 rpm for 5 minutes to make a fine emulsion. It was expected that the droplet size was around 5 microns.

The sample was then spray-dried using a ProCepT R&D Spray Dryer in its three-column set-up. This gave a column height of 1.8 m with a column diameter of 0.15 m. The air inlet temperature was 130 °C at a flowrate of 550 I/min. The two-fluid nozzle was used with a tip size of 1.0mm. The atomisation air flow rate was 10 I/min. The fluid was pumped into the spray dryer with a syringe pump at 6ml/min. An additional air flow of 140 I/min was introduced below the drying chamber to convey air to the cyclone.

The dried microcapsule powder formed from Example 7 was collected from the cyclone and also from the internal surfaces of the equipment where it had stuck, for example in the transfer pipe to the cyclone.

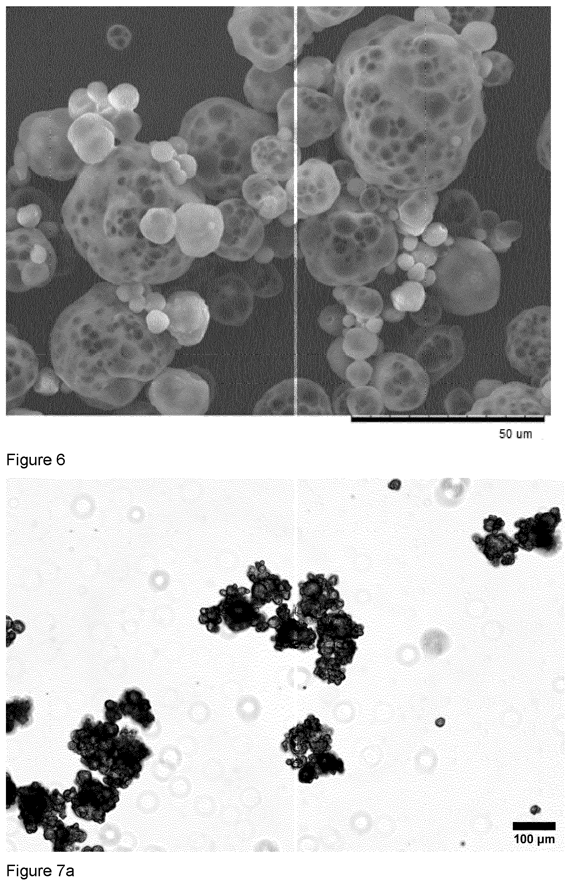

The microcapsules of Example 7 were examined under a Hitachi TM3030 SEM and mainly spherical fragrance encapsulates of diameter around 30 µm or less were observed (Figure 6). The microcapsules appear to have a pock-marked surface, indicating that there was minimal free fragrance present and that the samples were reasonably dry.

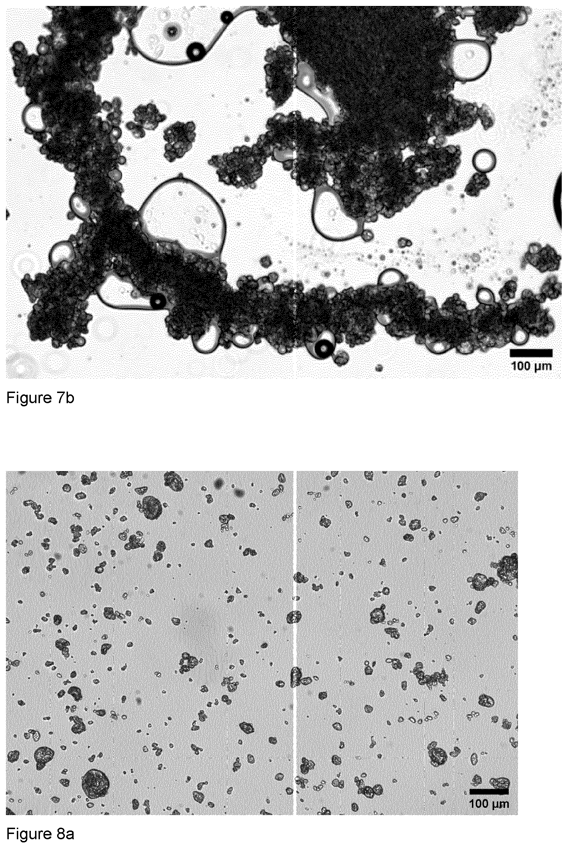

The microcapsules of Example 7 were rehydrated in hard water and optical microscope photographs taken (Figure 7a). The same capsules were then pressed so that they broke to release the oil within (Figure 7b). The oil droplets are clearly visible surrounding the broken microcapsules, with a very well-defined dark interface between the oil and water.

The microcapsules of Example 7 were analysed by the method described herein for their total fragrance loading. This was found to be 28.8%.

### Example 8 - Preparation of protein hydrogel slurry with acetic acid

### Preparation of the protein hydrogel

1120 g of reverse osmosis (RO) water was added to a 2-litre stainless steel container, and 216 g of pea protein isolate was added. The container was placed in a 92 °C water bath and mixed with an overhead stirrer at 1500 rpm. After stirring for 3 minutes, 480g glacial acetic acid was added. The mixture was stirred for 15 minutes at 1500 rpm, then for 30 minutes at 1200 rpm, ensuring that the temperature of the mix surpassed 85 °C for at least 10 minutes. The mixture was poured into trays to a depth of approximately 10 mm, and left at room temperature overnight.

### Application of shear to protein hydrogel

Shear was then applied to the hydrogel as follows. The protein hydrogel was cut into ~1cm cubes via a low-shear cutting step. The cubes were split between two 75 micron filter bags, which were each then submerged inside a bucket containing 16L of RO water. This formed a coarse protein hydrogel slurry within the filter bag. The hydrogel cubes were left to soak, with agitation from an overhead stirrer at 600-800 rpm, for 90-150 min. This step was performed to reduce the concentration of acetic acid in the hydrogel by diffusion to the continuous aqueous phase. The pH of the wash water was then measured, and if it was above 3.2, soaking was continued for a further 30 minutes. If it was below 2.9, half of the water was drained and replaced with fresh RO water, then soaking was continued for a further 30 minutes. The filter bag was then suspended above the bucket to drain for 5 minutes. The washed gel from both filter bags was transferred to a 5 liter beaker, and homogenized with a Silverson mixer at 5000 rpm for 5 minutes, 6000 rpm for 5 minutes, and 7000 rpm for 5 minutes. The smooth slurry was then transferred to 1 L Nalgene bottles (800 g in each), and exposed to high shear ultrasonication (Hielscher UP500Hdt) with cooling on ice, until 250 kJ had been applied, with shaking every 75 kJ. The hydrogel slurry was then passed through a 200 micron sieve before use.

### Comparative Example 9 - Preparation of protein slurry without acid

450g of reverse osmosis water was added to a 1 L stainless steel container, and 50 g of pea protein isolate was added. The container was placed in a 92 °C water batch and mixed with an overhead stirrer at 1500 rpm. The mixture was stirred for 15 minutes at 1500 rpm, then for 30 minutes at 1200 rpm, ensuring that the temperature of the mix surpassed 85 °C for at least 10 minutes. On cooling the mixture to ambient temperature, in contrast to the experiments with acetic acid, a gel was not formed. Shear was then applied with a Silverson mixer at 8000 rpm for 10 minutes, then with an ultrasonicator (Hielscher UP500Hdt) until 156 kJ had been applied. For comparison 0.3125 kJ/g was used for protein slurries prepared with organic acid. The mixture was then passed through a 200 micron sieve before use.

### Example 10 - Preparation of spray-dried microcapsules containing vitamin D2

### Preparation of composition

Vitamin D2 oil (1MIU/g) was diluted in Softisan^{®} 100 wax to a concentration of 6000ug/g. This concentration was chosen to target the manufacture of spray dried particles containing a Vitamin D2 concentration of 1,000 ug/g. The wax was melted above 40 °C, the vitamin oil added, and the mixture homogenised by hand shaking. The mixture was held above 40 °C until required.

The required mass of wax and Vitamin D2 mixture was then added to the protein hydrogel slurry of Example 8 and mixed with a Silverson L5M-A homogenizer at 7000 rpm for 10 minutes to form a composition with 11.9 weight % total solids including vitamin D2 and wax. The composition was then further diluted with 3.0% aqueous acetic acid to produce 2 samples: Example 10A with a total solids content of 10.0% including the vitamin D2 and wax (8.9 weight % protein solids) and Example 10B with a total solids content of 5.0% including the vitamin D2 and wax (4.4 weight % protein solids).

The required mass of wax and Vitamin D2 mixture was also added to the protein slurry of Comparative Example 9 and mixed with a Silverson L5M-A homogenizer at 7000 rpm for 10 minutes to form a composition. The composition was then further diluted with 3.0% aqueous acetic acid to produce Comparative Example 10C with a total solids content of 11.7 weight % including the Vitamin D2 and wax (10 weight % protein solids).

### Spray drying conditions

For Examples 10A and 10B the spray drying was performed using a ProCept spray dryer in its three-column set-up. This gave a column height of 1.8 m with a column diameter of 0.15 m.

For Example 10A the two-fluid nozzle was used with a tip size of 1mm. The atomisation air flow rate was 10l/min. The fluid was pumped into the spray dryer with a syringe pump at 6ml/min. An additional air flow of 150 I/min was set for the cyclone.

For Example 10B the ultrasonic nozzle was used. The atomisation air flow rate was 10l/min. The fluid was pumped into the spray dryer with a syringe pump at 6ml/min. An additional air flow of 150 I/min was set for the cyclone.

For comparative Example 10C the spray drying was performed using Buchi B290 Spray Dryer with a two-fluid nozzle was used with a tip size of 1.4 mm. The aspirator flow rate was set at 100%, with a Q-flow setting of 40. The fluid was pumped into the spray dryer with a peristaltic pump at a speed setting of 35% (approx. 10ml/min). The dried microcapsule powder formed was collected from the collection pot.

Other process settings are given in Table 6 below.

**Table 6**

| Microcapsule Example | Inlet air flow rate (m³/min) | Inlet air temp (°C) | Chamber out temp (°C) | Air out temp (°C) |
|---|---|---|---|---|
| Example 10A | 0.4 | 140 | 79.6 | 63.1 |
| Example 10B | 0.4 | 140 | 82.6 | 71.0 |
| Comparative Example 10C | 0.58 | 180 | 110-114 | n/a |

The spray drying experiments produced three free flowing powders with a mean particle size, d₅₀, measured by laser diffraction according to the method therein, of 28.9 microns, 40.8 microns and 9.1 microns for Examples 10A, 10B and Comparative Example 10C respectively. It is noted that for the acid treated plant protein encapsulate the more diluted composition dried with an ultrasonic nozzle produced larger microcapsules. The non-acid treated plant protein with a similar solids content the powder had a much smaller average particle size.

The Vitamin D2 concentration in the microcapsule powder was measured using the method Version B described herein and was found to be 963 µg/g, 919 µg/g and 1149 µg/g in Examples 10A, 10B and Comparative Example 10C, respectively. This was close to the expected target of 1000 µg/g of microcapsule indicating very little loss of Vitamin D2 during the spray drying process.

### Example 11 - Accelerated stability testing of Vitamin D2 microcapsules in a beverage formulation

### Preparation of beverage samples

The microcapsules of Examples 10A, 10B and Comparative Example 10C were each prepared into a concentrated slurry so that they could then be added to Miwadi Blackcurrant single concentrate squash.

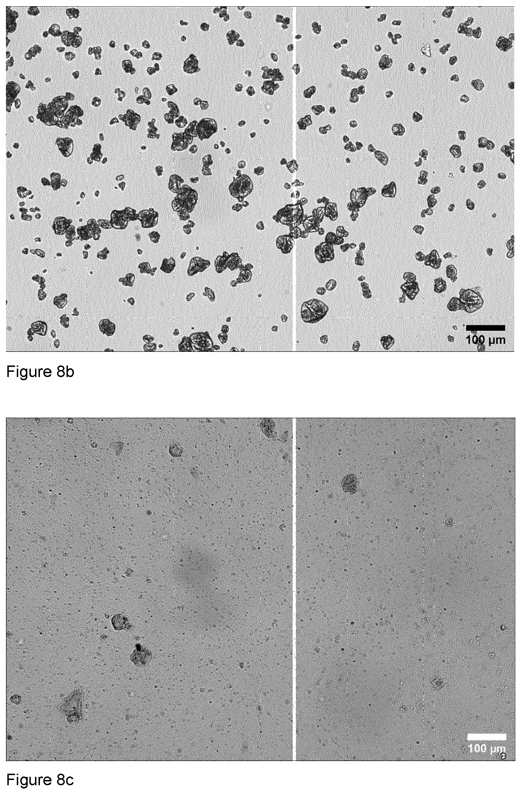

0.2g of each microcapsule was first suspended in 30g of boiling water with vigorous shaking, followed by vortexing at 2000 rpm for 1 minute to mimic the temperature profile during flash pasteurization. It was noted that the concentrated slurry prepared with Comparative Example 10C was more hazy and appeared finer than Examples 10A and 10B. Figures 8a, 8b and 8c are 10X microscope images of the concentrated slurry of Example 10A, 10B and Comparative Example 10C. Example 10A and 10B microcapsules can be seen and have clearly survived the processing. For Comparative Example 10C the image is notable for the lack of intact microcapsules demonstrating that subjecting them to heat and shear caused the microcapsules to quickly disintegrate. In their place smaller aggregates of proteins and very small particles of wax and Vitamin D2 can be seen.

### Accelerated Stability Testing

A calculated mass of each concentrated slurry was added to 2 liters of squash and mixed with an overhead mixer at 800 rpm for 5 minutes to ensure homogeneity before transferring to bottles.

200ml transparent plastic PET bottles were filled with each of the three squash samples and initial Vitamin D2 loading was measured using the method herein described. The initial beverage Vitamin D loading of Examples 10A and 10B are given in Table 7. However no initial measured beverage loading of Vitamin D2 in Comparative Example 10C could not be detected. Without wishing to be bound by theory it is believed that this is because in the concentrated slurry preparation, where the microcapsules were suspended and heated, they were broken so releasing the Vitamin D2. The Vitamin D2 oil floated to the top of the bottle and was not sampled.

Filled PET bottles of Examples 10A and 10B were then placed in a Binder KBF P 240-230 V chamber, which was set for accelerated ambient light stability studies with UV-A set at 1.1 W/m2 at 20°C and lab humidity (typically 50% relative humidity (RH)). The Vitamin D loading was again measure after 4 weeks in the Binder chamber. The % reduction in Vitamin D2 content was calculated and recorded in Table 7.

**Table 7**

| Microcapsule | Initial Beverage Vit D2 Loading | Microcapsule Particle Size, d50 | % Vit D2 loss after 4 weeks |
|---|---|---|---|
| Example 10A | 3.0 | 28.9 | 22.5% |
| Example 10B | 2.0 | 40.8 | 5.5% |

After 4 weeks in the squash the Vitamin D2 in the encapsulate of the invention had been protected from degradation so that for Example 10A only 22.5% of the initial Vitamin D2 had been lost and for Example 10B only 5.5% of the initial Vitamin D2 had been lost. Unencapsulated vitamin D2 is expected to degrade significantly on exposure to UV over this time frame, but this low level of loss demonstrated the protection provided by the inventive microcapsule. It can also be seen that the lowest loss was achieved with the larger average encapsulate size. Without wishing to be bound by theory, it is believed that the larger the average particle size of the encapsulate the lower the surface area available per mass of Vitamin D2 for exposure to UV and therefore the less the degradation.

### Example 12 - Encapsulation of natural agrochemical actives

### Example 12A: Thymol protein microcapsule preparation

### Preparation and application of shear to the protein hydrogel

The protein hydrogel shell was prepared and sheared as described in Example 8.

The protein solid content was measured at 9.3 wt%. Dilute acetic acid (3 wt% in DI water) was added to reduce the protein solids content to 8.0 %.

### Preparation of spray-dried microcapsule

The diluted dispersion was homogenised with a Silverson L5M-A high-shear mixer (2 minutes at 8000 rpm). 16.81 g of thymol, diluted at 40 wt% in Miglyol 812N, was added to 103.25 g of diluted dispersion. The mixture was homogenised further with the Silverson at 8000 RPM for 5 minutes. It was expected that the droplet size was around 5 microns.

The sample was then spray-dried using a Buchi B290 Spray Dryer. The air inlet temperature was 120 °C at Q-flow setting of 40. The two-fluid nozzle was used with a tip size of 1.4mm. The aspirator flow rate was set at 100%. The fluid was pumped into the spray dryer with a peristaltic pump at a speed setting of 13% (4-5 ml/min).

The dried microcapsule powder formed was collected from the collection pot. The microcapsules were analysed for their total thymol loading. This was found to be 4.9 wt%. The particle size was measured via laser diffraction and found to have a d50 of 23 microns.

### Example 12B: Comparative Maltodextrin thymol microcapsule preparation

45g maltodextrin was dissolved in 105g of deionised water and mixed with a magnetic stirrer bar until a homogenous slurry was formed. 15.23 g of thymol, diluted at 40 wt% in Miglyol 812N, was added to the slurry, in addition to 0.14g of polysorbate 80. The mixture was homogenised with a Silverson L5M-A high-shear mixer for 5 minutes at 8000 rpm.

The sample was then spray-dried using a Buchi B290 Spray Dryer. The air inlet temperature was 130 °C at Q-flow setting of 40. The two-fluid nozzle was used with a tip size of 1.4mm. The aspirator flow rate was set at 100%. The fluid was pumped into the spray dryer with a peristaltic pump at a speed setting of 13% (4-5 ml/min).

The dried microcapsule powder formed was collected from the collection pot. The microcapsules were analysed for their total thymol loading. This was found to be 3.4 wt%. It was not possible to measure the particle size of these microcapsules as they were too fragile. When placed on a microscope slide with a cover slip it could be seen that the weight of the cover was sufficient to break them.

### Example 12C: Aqueous release test

The thymol microcapsule powder of Example 12A was suspended in phosphate buffered saline (PBS) (pH 7.4) containing 0.2% w/v methylparaben and a solution of sodium acetate and calcium chloride according to Table 8, such that the sample contained 4 mg of thymol. Replicate samples were prepared so that samples could be analysed at t=0, 24 hours, 72 hours, 1 week, and 2 weeks of incubation.

**Table 8 - Composition of test sample**

| Test sample | Mass of microcapsule powder (mg) | Mass of 10 mM NaOAc + 5 mM CaCl2 (mg) | Mass of PBS pH 7.4, + 0.2%wt methylparaben (mg) |
|---|---|---|---|
| Example 12A | 82 | 459 | 3541 |

The samples were mixed on day 0 and placed in a 37°C incubator in the dark, except for t=0 samples, which were analysed immediately. At each time point, samples were centrifuged for 30 min at 4900 RPM, and the supernatant transferred to 50ml Falcon tubes. Ethanol was added to achieve a tenfold dilution of the supernatant samples. The concentration of thymol in the supernatant was measured by gas chromatography, which was used to calculate the quantity of thymol released from the microcapsules.

To the pelleted material after centrifugation of each sample, 2.9 ml of DI water and 0.1 ml of 10% KOH solution were added. The mixtures were ultrasonicated (Bandelin Sonopuls HD4200, with probe TS104) for 2 minutes at 30% amplitude, vortexed and centrifuged for 5 minutes at 4900 rpm. 100 µl of the supernatant from each sample was diluted tenfold with ethanol, then the concentration of thymol measured by gas chromatography. This was used to calculate the quantity of thymol remaining in the sample at the end of the experiment, which was taken to be the quantity of thymol not originally released in the first supernatants. The results are shown in Table 9.

**Table 9**

| Microcapsule Example 12A | % extractable thymol in supernatant | % extractable thymol in remaining shell material |
|---|---|---|
| t=0 | 3 | 97 |
| t=24hr | 5 | 95 |
| t=72hr | 5 | 95 |
| t=1 week | 8 | 92 |
| t=2 weeks | 6 | 94 |

This release study demonstrated that most of the active ingredient, more than 90%, remained encapsulated over 2 weeks. Such a slurry would be suitable for storage prior to being applied to fields and crops.

### Example 12D: Comparative Maltodextrin-thymol release test

The thymol microcapsule powder of Comparative Example 12B was suspended in phosphate buffered saline (PBS) (pH 7.4) containing 0.2% w/v methylparaben according to Table 10, such that the sample contained 4 mg of thymol, the same level as in Example 12A. There was only a minor difference in the suspension composition with the absence of the low levels of NaOAc and CaCl2 which is of no consequence in this example.

**Table 10**

| Test sample | Mass of microcapsule powder (mg) | Mass of PBS pH 7.4, + 0.2%wt methylparaben (mg) |
|---|---|---|
| Comparative Example 12B Maltodextrin microcapsules | 119 | 4000 |

The sample was prepared, mixed and incubated for one hour. The sample was centrifuged for 10 min at 4900 RPM, and the supernatant transferred to 50ml Falcon tube. Ethanol was added to achieve a tenfold dilution of the supernatant samples. The concentration of thymol in the supernatant was measured by gas chromatography, which was used to calculate the quantity of thymol released from the capsules in buffered water.

To the pelleted material after centrifugation of sample, 2.9 ml of deionised water and 0.1 ml of 10% KOH solution were added. The mixture was ultrasonicated (Bandelin Sonopuls HD4200, with probe TS104) for 2 minutes at 30% amplitude, vortexed and centrifuged for 5 minutes at 4900 rpm. 100 µl of supernatant was diluted tenfold with ethanol, then the concentration of thymol measured by gas chromatography. This was used to calculate the quantity of thymol remaining in the sample at the end of the experiment, which was taken to be the quantity of thymol not originally released in the first supernatant. The results are shown in Table 11.

**Table 11**

| Comparative Example 12B Maltodextrin microcapsules | % extractable thymol in supernatant | % extractable thymol in remaining shell material |
|---|---|---|
| t=1 hour | 42 | 58 |

This release study demonstrated that for maltodextrin microcapsules, in Comparative Example 12B, a substantial portion of active ingredient, more than 40%, was released after 1 hour in an aqueous buffer. Such a slurry would not be suitable for storage prior to being applied to fields and crops. Once prepared into an aqueous composition, or in the presence of water in the environment such as rain, it would prematurely release the active ingredient thereby demonstrating no controlled release properties.

### Example 13 - Preparation of spray-dried microcapsules containing fragrance oils

### Fragrance/solvent mixtures

Two different fragrances (13A and 13B) were prepared according to Table 12. Each fragrance was then blended with one of two solvents: either Miglyol^{®} 812N or isopropyl myristate (IPM) in a weight ratio of 80:20 fragrance: solvent.

Fragrance 13A comprises only moderately volatile fragrance materials of similar volatility with a range of hydrophobicity from very low to very high. Fragrance 13B comprises materials of similar hydrophobicity with the full range of volatilities including low, moderate and high volatility materials. Both fragrances had fragrance materials with HSP parameters in the range δD from 15 to 20, δP from 1 to 7 and δH from 2.5 to 8.

**Table 12**

| | | | LogP | HSP | | | | | |
|---|---|---|---|---|---|---|---|---|---|
| Fragranc e material | CAS number | Vapour pressure (Torr) | | δD | δP | δH | Functionality | Fragran ce 13A (wt%) | Fragran ce 13B (wt%) |
| Dipenten e | 138-86-3 | 1.541 | 4.40 | 17. 2 | 1.8 | 4.3 | Alkene | n/a | 20 |
| Ortho tertiary butyl cyclo hexyl acetate (OTBCH A) | 88-41-5 | 0.103 | 4.46 | 15. 9 | 1.9 | 2.9 | Ester | n/a | 20 |
| Diphenyl oxide | 101-84-8 | 0.022 | 4.03 | 19. 4 | 3.4 | 4.0 | Ether | n/a | 20 |
| Geraniol | 106-24-1 | 0.013 | 3.42 | 16. 9 | 4.2 | 7.6 | Primary alcohol | 25 | n/a |
| Undecav ertol | 81782-77-6 | 0.011 | 3.97 | 16. 4 | 3.4 | 7.5 | Secondar y alcohol | 25 | n/a |
| Delta damasco ne | 57378-68-4 | 0.010 | 3.56 | 16. 9 | 3.5 | 2.6 | Ketone | 25 | 20 |
| dodecan e nitrile | 2437-25-4 | 0.005 | 5.12 | 16. 0 | 5.4 | 3.1 | Nitrile | 25 | n/a |
| Benzyl salicylate | 118-58-1 | 0.0002 | 4.09 | 19. 3 | 6.5 | 7.8 | Ester | n/a | 20 |

### Preparation of spray dried microcapsules

The protein hydrogel slurry was made as per Example 8.

A first batch of fragrance composition was prepared as follows. A batch of protein hydrogel slurry of Example 8 of between 100 and 120g was diluted by 20% with 3% acetic acid solution to give a slurry with a protein solids content of 8.0 wt%. The diluted slurry was then stirred with a Silverson lab mixer at 8000 rpm for 2 minutes in a 400ml Nalgene beaker. 31.5 wt% (based on the weight of the slurry) of Fragrance 13B/Miglyol^{®} 812N blend was then added to the slurry and emulsified using a Silverson mixer at 8000 rpm for a further 5 mins to form a fine emulsion. This fine emulsion was left to age at room temperature for approximately 2 days prior to spray drying. This mix was used in Run 2 TTB.

A second batch of fragrance composition was prepared as above except that the fragrance blend was emulsified just prior to spray-drying by gentle hand shaking rather than by use of the Silverson mixer. This mix was used in Run 3 TTB.

A third batch of fragrance composition was prepared as the first batch except that the Miglyol^{®} 812N was replaced by isopropyl myristate (IPM) to form the fragrance:solvent blend. The fragrance and slurry mixture was emulsified by using a Silverson mixer at 8000 rpm for a further 5 mins to form a fine emulsion. This fine emulsion was left to age at room temperature for approximately 2 days prior to spray drying. This mix was used in Run 6 TTB.

A fourth batch of fragrance composition was prepared with the same composition and in the same manner as the material used in Run 6 TTB and used in Run 7 CPB and Run 8 TTB.

The samples were spray-dried using a ProCepT R&D Spray Dryer in its three-column set-up. This gave a column height of 1.8 m with a column diameter of 0.15 m.

The air inlet temperature was varied to achieve a dry powder. It was kept as low as possible to reduce the loss of volatile fragrance materials. The drying inlet air flow rate was kept constant at 0.4m³/min. The two-fluid nozzle from Spray Systems was used with a tip size of 1.0mm. The atomisation air flow rate was varied between runs as shown in Table 13 below. The fluid was pumped into the spray dryer with a syringe pump at 4ml/min for all the runs. An additional air flow of 150 I/min was introduced below the drying chamber to convey air to the cyclone.

The dried microcapsule powder formed was collected in the collection pot and also from the internal surfaces of the equipment where it had stuck, for example in the transfer pipe to the cyclone. The microcapsules particle size was measured by laser diffraction.

Fragrance 13B was successfully encapsulated as given in Table 13.

**Table 13**

| Fragrance composition 13B | Run2TTB | Run3TTB | Run6TTB | Run8TTB | Run7CPB |
|---|---|---|---|---|---|
| Core | Miglyol^{®} 812N | Miglyol^{®} 812N | IPM | IPM | IPM |
| Emulsion type | Fine aged | Coarse fresh | Fine Aged | Fine Aged | Fine Aged |
| Atomisation (Itr/min) | 15 | 15 | 15 | 12 | 8 |
| Air inlet °C | 68.6-68.2 | 68.5-68.7 | 68.5-68.8 | 80.1-88.4 | 82.3-83.5 |
| Air outlet °C | 43.5-41.5 | 41.6-43.7 | 42.1-54.7 | 48.4-61.5 | 40.3-42.6 |
| Particle size, d50 (microns) | 13.3 | 30.1 | 12.2 | 14.6 | 40.1 |

When drying at a lower temperature (inlet around 69°C) the largest particle size of 30.1 microns, was produced with the coarse fresh emulsion with Miglyol^{®} 812N as the solvent, prepared just before spray drying. Changing the fragrance solvent from Miglyol^{®} 812N to isopropyl myristate within the fine aged emulsion had very little effect on the particle size.

Reducing the atomising air flowrate resulted in larger particles (compare Run 8TT with Run 7CP).

The fragrance material composition of these microcapsules was analysed using the fragrance loading quantification method via GC described herein. Results are given in Table 14.

**Table 14**

| Fragrance composition 13 B | Run2TTB | Run3TTB | Run6TTB | Run8TTB | Run7CPB |
|---|---|---|---|---|---|
| Core | Miglyol^{®} 812N | Miglyol^{®} 812N | IPM | IPM | IPM |
| Emulsion type | Fine aged | Coarse fresh | Fine Aged | Fine Aged | Fine Aged |
| Total fragrance oil loading wt% | 45.4 | 51.3 | 48.3 | 41.5 | 44.4 |
| Wt% Dipentene | 0.6 | 1.5 | 0.7 | 0.6 | 0.7 |
| Dipentene oil % | 1.3 | 2.9 | 1.4 | 1.4 | 1.6 |
| Wt% (OTBCHA) | 5.0 | 6.3 | 5.5 | 4.3 | 5.1 |
| (OTBCHA) oil % | 11.0 | 12.2 | 11.4 | 10.4 | 11.5 |
| Wt% Diphenyl ether | 11.4 | 11.8 | 11.6 | 9.0 | 10.5 |
| Diphenyl ether % | 25.1 | 23.0 | 24.0 | 21.7 | 23.6 |
| Wt% Delta Damascone | 6.4 | 6.8 | 5.8 | 4.5 | 5.7 |
| Delta damascone oil % | 14.1 | 13.3 | 12.0 | 10.8 | 12.8 |
| Wt% Benzyl salicylate | 22.0 | 24.9 | 24.7 | 23.1 | 22.5 |
| Benzyl salicylate oil% | 48.4 | 48.5 | 51.1 | 56.3 | 49.5 |

Samples dried at lower air inlet temperatures typically had a higher total fragrance loading than those dried at higher air inlet temperatures.

When drying at the lower temperature (inlet around 69°C), isopropyl myristate resulted in higher total loading than Miglyol ^{®}812N for a fine emulsion. In addition, at these drying conditions the coarse emulsion resulted in higher loading.

When drying at a higher temperature (inlet at least 80°C) with the atomisation air flowrate reduced a higher total loading was achieved than with high air atomisation.

Fragrance 13B demonstrated that for fragrance materials of similar hydrophobicity it is possible to encapsulate materials with a range of volatilities. However as expected the least volatile fragrance materials are enriched in the encapsulate with benzyl salicylate accounting for between 48.4 and 56.3% of the fragrance whereas it was 20% of the fragrance before encapsulation. Drying at lower temperatures and with a coarse emulsion improved the encapsulation of the more volatile fragrance materials such as Limonene from 0.6% to 1.5% of the fragrance.

Fragrance Composition 13A was also successfully encapsulated as given in Table 15.

A first batch of fragrance composition was prepared as follows. A batch of protein hydrogel slurry of Example 8 of between 100g and 120g was diluted by 20 wt% with 3% acetic acid solution to give a slurry with a protein solids content of 8.0 wt%. The diluted slurry was then stirred with a Silverson lab mixer at 8000 rpm for 2 minutes in a 400ml Nalgene beaker. 28.3 wt% (based on the weight of the slurry) of Composition 13A/Miglyol^{®} 812N blend was then added to the slurry and emulsified using a Silverson mixer at 8000 rpm for a further 5 mins to form a fine emulsion. This fine emulsion was left to age at room temperature for approximately 2 days prior to spray drying. This mix was used in Run 1 TTA.

A second batch of fragrance composition was prepared as above. Immediately prior to use, 120g of the emulsion was diluted with 60g of 3% acetic acid solution with gentle agitation. This mix was used in Run 9 TTA.

A third batch of fragrance composition was prepared in the same manner as the first batch above except that the fragrance blend was sheared and emulsified just prior to spray-drying rather than being allowed to age. This mix was used in Run 3 TTA.

A fourth batch of fragrance composition was prepared as above except that the fragrance blend was emulsified just prior to spray-drying by gentle hand shaking rather than by use of the Silverson mixer. This mix was used in Run 2 TTA.

A fifth batch of fragrance composition was prepared in the same manner as the first batch but now with the Miglyol^{®}812N replaced by isopropyl myristate (IPM). This mix was used in Run 4TTA.

A sixth batch of fragrance composition was prepared in the same manner as the fifth batch and this mix was used in Run 5CPA.
The same equipment was used as in the earlier runs with drying air flowrates, emulsion flow rates and transfer air rates the same as for Fragrance Composition 13B. The dried microcapsule powder formed was again collected in the collection pot and also from the internal surfaces of the equipment where it had stuck, for example in the transfer pipe to the cyclone. The microcapsules particle size was measured by laser diffraction.

**Table 15**

| Fragrance composition 13A | Run1TT A | Run9TT A | Run3TT A | Run2TT A | Run4TT A | Run5CP A |
|---|---|---|---|---|---|---|
| Core | Miglyol^{®} 812N | Miglyol^{®} 812N | Miglyol^{®} 812N | Miglyol^{®} 812N | IPM | IPM |
| Emulsion type | Fine aged | Diluted Fine aged | Fine fresh | Coarse fresh | Fine aged | Fine aged |
| Atomisation air flow (Itr/min) | 15 | 10 | 15 | 15 | 15 | 10 |
| Air inlet temperature °C | 69.7-69.1 | 68.8-63.7 | 68.7-68.5 | 68.9-68.8 | 68.8-68.5 | 83.5-68.7 |
| Air outlet (transfer tube) temperature °C | 50.2-47.6 | 43.4-40.8 | 43.4-41.1 | 37.7-37.0 | 44.0-42.1 | 63.2-46.5 |
| Particle size, d50 | 12.9 | 15.3 | 29.9 | 53.8 | 16.4 | 22.3 |

The largest particle size was produced with the coarse fresh emulsion with Miglyol^{®}812N as the fragrance solvent prepared just before spray drying.
The fine aged emulsion, with Miglyol^{®}812N as the fragrance solvent, resulted in the smallest microencapsulate particle size. Dilution of the fine aged emulsion and running at lower drying temperatures did not affect the particle size. Changing the fragrance solvent from Miglyol^{®}812N to isopropyl myristate within the fine aged emulsion also had very little effect on the particle size.

A fine emulsion spray dried after ageing had a smaller particle size than the same freshly made emulsion.

The fragrance material composition of these microcapsules was analysed using the fragrance loading quantification method via GC described herein. Results are given in Table 16.

**Table 16**

| Fragrance composition 13A | Run1TTA | Run9TTA | Run3TTA | Run2TTA | Run4TTA | Run5CP A |
|---|---|---|---|---|---|---|
| Core | Miglyol81 2 | Miglyol81 2 | Miglyol81 2 | Miglyol81 2 | IPM | IPM |

| Emulsion type | Fine aged | Fine aged - diluted | Fine fresh | Coarse fresh | Fine aged | Fine aged |
|---|---|---|---|---|---|---|
| Total fragrance oil loading wt% | 29.1 | 24.6 | 27.8 | 26.7 | 26.3 | 30.4 |
| wt% Geraniol | 6.7 | 6.3 | 6.9 | 6.6 | 6.3 | 7.1 |
| Geraniol oil % | 21.3 | 25.6 | 25.1 | 24.7 | 24.0 | 23.3 |
| wt% Undecavert ol | 5.0 | 5.1 | 5.5 | 5.5 | 5.1 | 5.6 |
| Undecavert ol oil % | 17.2 | 20.7 | 19.8 | 20.6 | 19.4 | 18.4 |
| wt% Delta-damascone | 4.4 | 3.2 | 4.0 | 4.3 | 3.6 | 4.3 |
| Delta damascone oil % | 15.1 | 13.0 | 14.4 | 16.1 | 13.7 | 14.1 |
| wt% Dodecane nitrile | 13.1 | 10.0 | 11.5 | 10.4 | 11.3 | 13.4 |
| Dodecane nitrile oil % | 45.0 | 40.7 | 41.4 | 39.0 | 43.0 | 44.1 |

All samples had similar total fragrance loadings but the fine aged sample with IPM sprayed with the lower air atomisation rate had the highest loading at 30%. This is noticeably less than Fragrance Composition 13B which had a maximum total fragrance loading of 51%. Without wishing to be bound by theory this is likely due to the presence of very low logP fragrance materials in Fragrance 13A.

Fragrance Composition 13A contained 4 fragrance materials of similar moderate volatility and varying hydrophobicity and demonstrates that it is possible to encapsulate materials with a range of hydrophobicity. The composition of the encapsulated fragrance was richer in the most hydrophobic fragrance material, dodecane nitrile, for all the examples. This was least noticeable in the coarse emulsion sample.

Delta-damascone was the least enriched fragrance material despite having a higher logP than geraniol and undecavertol. These two alcohols were well encapsulated, particularly geraniol, demonstrating that it is still possible to encapsulate these relatively hydrophilic fragrance materials within a fragrance.

### Olfactive evaluation of spray dried microcapsules

A concentrated slurry of the microcapsule of Run 3TTA was prepared prior to making the diluted formulation for olfactive evaluation. Some single strength buffer solution with preservative was added drop by drop to the dry microcapsules in a 50 ml Falcon tube, vortexed at 2000 rpm in between drop additions, in order to obtain a slurry paste. When the paste appeared homogeneous, more buffer solution was added for further dilution, still using vortexing as a means of ensuring homogeneous dispersion of the microcapsules. The suspension was left to stand for 1h to allow for possible swelling of the microcapsules before being strained through a 40 µm cell strainer for 5 minutes. Any extra moisture was absorbed with paper tissue.

The total fragrance level was analysed by GC according to the method herein to be 12.6 wt%. This is lower than the total fragrance level of the spray dried microcapsules. Whilst not wishing to be bound by theory it is believed that as the hydrogel shell has been hydrated and swollen it has resulted in an increase in the mass of the shell. In addition a small amount of surface fragrance may have been lost into the aqueous phase.

A diluted slurry of the microcapsules containing gellan gum was prepared so that it contained 0.1 wt% total fragrance for olfactive evaluation. Gellan gum helps maintain the microcapsules well suspended. A solution of gellan gum was prepared by mixing 9.5 mg of gellan gum with 15 g of deionised water in a 50 ml Falcon tube. The mixture was heated to above 90°C for 20 minutes and stirred at 500 rpm using an Eppendorf Thermomixer C set at 100°C. After cooling to room temperature, 15 g of double strength buffer solution was added. The concentrated encapsulate slurry was added to the gellan gum solution using a micropipette with a low shear tip, and the sample tube was closed and inverted repeatedly to mix.

The pH of the diluted slurry was 3.05. The odour of the diluted slurry was evaluated according to the method herein described, and the results are shown in Table 17.

**Table 17**

| Substrate | | Glass | Card |
|---|---|---|---|
| Initial application | Strength | 3.75 | 4.25 |
| | Character | Fruity (orange), ester, white floral, green, slightly medicinal | Fruity (orange), ester, medicinal, aldehydic, green |
| 24 hours | Strength | 0.5 | 0.7 |
| | Character | slightly fruity, harsh | Slightly floral, powdery |
| Rubbing | Strength | 4.4 | 3.0 |
| | Character | Sweet, ester, fruity, tangerine peel, green, harsh/chemical, aldehydic, medicinal | Sharp fruity, aldehydic, fizzy sherbet |
| Pressure with a slide | Strength | 4.2 | Not applicable |
| | Character | Sweet, ester, fruity, tangerine peel, green, harsh/chemical, aldehydic, medicinal | Not applicable |

On initial application there was a strong characteristic odour of the fragrance indicating that some of the fragrance oil is associated with the surface of the hydrogel shell. After 24 hours of drying the odour was very weak indicating that most of the surface fragrance had evaporated. On rubbing or pressing on glass a strong characteristic odour was noticed as the force ruptures that microcapsule releasing the fragrance. On rubbing on card the odour was of moderate strength and the fragrance character was somewhat different to that on glass, indicating that there was only partial release of the encapsulated fragrance.

### Example 14 - Cross-linking of spray dried fragrance microcapsules

Spray dried microcapsules were prepared as in Example 13 Run 3TTA.

Hard water adjusted to pH3 was added drop by drop to 0.8 g of dry microcapsules in a 50 ml Falcon tube, vortexed at 2000 rpm in between drop additions, to obtain a slurry paste. When the paste appeared homogeneous, more hard water at pH3 was added for further dilution, still using vortexing as a means of ensuring homogeneous dispersion of the microcapsules. The suspended microcapsules were further diluted in a total of 390 ml of hard water and the pH was adjusted to 3.0 ± 0.5.

A 10 wt% tannic acid solution was prepared by hand shaking and vortexing for a few seconds using a Fisherbrand^{™} ZX4 IR Vortex Mixer, followed with immersion in an ultrasonic bath set at 50°C for 5-10 min, until complete dissolution.

10.6 g of the tannic acid solution was added to the microcapsules suspension under mechanical agitation at 170 rpm at 50°C, and left stirring for 5.5 hours. After this time, the suspension was stored in the fridge for around 36 hours. The crosslinked microcapsule suspension was poured over a 38 µm sieve, washed with hard water pH3 and resuspended in a clean separating funnel in hard water pH3. The decanted capsules were collected a second time on the 38 µm sieve, washed with single strength buffer solution with preservative and resuspended in this buffer solution in the separating funnel. The capsules were collected as concentrated a slurry after decantation and appeared as brown coloured microencapsulates, indicating that crosslinking had occurred.

The crosslinked capsule slurry was directly strained through a 40 µm cell strainer for 5 minutes and any extra moisture was absorbed with a paper tissue. The total fragrance level was analysed by Gas chromatography by the method herein to be 4.1 wt%. This is lower than the total fragrance level of the microcapsules prior to cross-linking. Without wishing to be bound by theory it is believed that this is due to surface and loosely associated fragrance being removed by the washing process during the cross-linking. The particle size was measured by laser diffraction to give a D₅₀ of 47.9 µm. This is larger than the particle size of the original spray dried encapsulate indicating that the hydrogel shell has been hydrated and the encapsulate has swollen.

### Olfactive evaluation of spray dried and cross-linked microcapsules

A diluted slurry of the microcapsules with gellan gum was prepared so that it contained 0.1 wt% total fragrance. The gellan gum helped maintain the microcapsules well suspended. This was done according to the method in Example 13. The pH of the diluted slurry was 3.04. The odour of the diluted slurry was evaluated according to the method herein described, and the results are shown in Table 18.

**Table 18**

| Substrate | | Glass | Card |
|---|---|---|---|
| Initial application | Strength | 2.8 | 3.1 |
| | Character | Fruity, lemon, sweet, floral | Fruity, lemon, sweet, medicinal, tangerine peel |
| 24 hours | Strength | 1.1 | 0.7 |
| | Character | Sweet, fruity, floral | Floral |
| Rubbing | Strength | 4.0 | 3.9 |
| | Character | Sweet, ester, artificial fruit, tangerine peel, floral | Sweet, fruity, ester, tangerine peel, medicinal |
| Pressure with a slide | Strength | 4.25 | Not applicable |
| | Character | Fresh, citrus, tangerine peel, fruity | Not applicable |

On initial application there was a moderate characteristic odour for the fragrance indicating that a small amount of the fragrance is associated with the surface of the hydrogel shell. After 24 hours of drying the odour was very weak indicating that most of the surface fragrance had evaporated. On rubbing or pressing on glass a strong characteristic odour was noticed as the force ruptures that microcapsule releasing the fragrance. On rubbing on card a moderate odour was noticed and the fragrance character was similar to that on glass.

### Example 15 - Preparation of spray-dried microcapsules containing Flaxseed oil

### Preparation and application of shear to the protein hydrogel

The protein hydrogel slurry was prepared as described in Example 8. The protein solid content was measured as 9.5 wt%.

### Preparation of spray-dried microcapsule

Flaxseed oil is a dietary supplement used for its high level of omega-3 fatty acids that is also a vegan alternative to fish oils. The slurry was homogenised with a Silverson L5M-A high-shear mixer for 2 minutes at 8000 rpm. Flaxseed oil was added to the slurry at a 1:1 oil: protein solids weight ratio and the mixture was homogenised further with the Silverson at 8000 RPM for 5 minutes to create a fine emulsion.

The slurry was spray-dried within a day of making, using a Buchi B290 Spray Dryer using a two-fluid nozzle with a tip size of 1.4mm. The aspirator flow rate was set at 100%, with a Q-flow setting of 40. The fluid was pumped into the spray dryer with a peristaltic pump at a speed setting of 13% (4-5 ml/min). The air inlet temperature varied between 183 and 180 °C and the air outlet temperature between 126 and 122°C.

Dry microcapsule powder formed indicating that the oil was efficiently encapsulated. The particle size was measured via laser diffraction according the method herein and found to have a d50 of 45.3 microns. The powder had no rancid off-odour indicating that the temperature in the drier had not degrade the flaxseed oil. It had a weak nutty and very slight vinegar odour indicating that there was only a low level of surface oil.

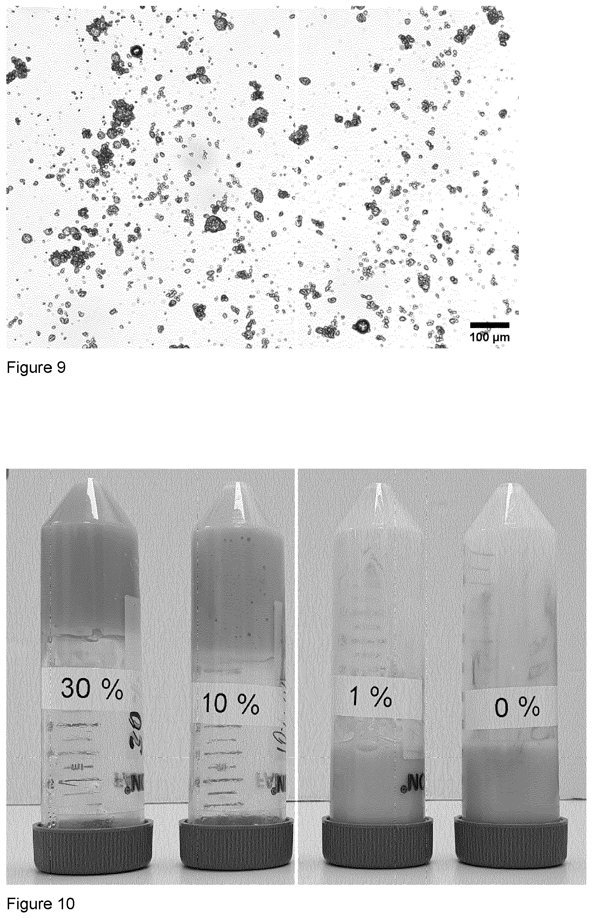

Figure 9 shows a 10X optical microscope image of Example 15 suspended in single strength citrate buffer where it can be seen that the encapsulates remain intact.

The microcapsule powder of Example 15 was also suspended in Miwadi Blackcurrant single concentrate by vortexing at 2000 rpm for 1 minute at a level of 12.5 g/L. The encapsulate did not impart any negative odour to the characteristic red fruit note of the original product demonstrating that the encapsulate was insoluble and the flaxseed oil remained encapsulated thereby having very little impact on product aroma. In comparison the same level of flaxseed oil could not be incorporated into the beverage without emulsifiers and would have provided a very noticeable nutty odour and characteristic effect on the beverage taste.

### Example 16 - Demonstration of formation of protein gel

Reverse osmosis (RO) water and pea protein isolate was added to a 1-litre stainless steel container and placed in a 92 °C water bath and mixed with an overhead stirrer at 1300 rpm. After stirring for 3 minutes, glacial acetic acid was added. The mixture was stirred for a total of 45 minutes at 1300 rpm, ensuring that the temperature was maintained above 80 °C. The mixture was then subjected to high shear with a Silverson L5M-A homogenizer, at 8000 rpm for 10 minutes. 20 ml aliquots of the liquid were poured into a 50 ml falcon tubes, sealed and refrigerated overnight in a normal upright position.

Different levels of organic acid were tested as given in Table 19.

**Table 19**

| Example | 16A | 16B | 16C | 16D |
|---|---|---|---|---|
| Mass glacial acetic acid (g) | 0 | 5 | 50 | 150 |
| Mass pea protein isolate (g) | 50 | 50 | 50 | 50 |
| Mass water (g) | 450 | 455 | 400 | 300 |
| wt% Acetic acid | 0 | 1 | 10 | 30 |
| Weight ratio acetic acid: water | 0 | 1:91 | 1:8 | 1:2 |
| Weight ratio acetic acid: Protein solids | 0 | 1:10 | 1:1 | 3:1 |

After a minimum of 12 hours of refrigeration the formation of a gel was evaluated by inverting the vial. As can be seen in Figure 10, use of 30% and 10% glacial acetic acid resulted in the formation of a solid gel that did not drop under gravity. Use of 1% glacial acetic acid and no acid did not form a gel and fell into the lid of the inverted Falcon tube.

## Claims

1. A method for the preparation of a biodegradable microcapsule, the method comprising:
(a) forming a solution comprising one or more plant-based protein(s) in a solvent system, wherein the solvent system comprises miscible co-solvents; wherein a first co-solvent increases solubility of the plant-based protein(s), and a second co-solvent decreases solubility of the plant-based protein(s); and wherein the co-solvents are added to the solution either in concentrated or in a diluted form; preferably wherein the pH of the plant-based protein solution is at least 0.5 pH units below the isoelectric point of the plant-based protein;
(b) inducing the plant-based protein in the solution to undergo a sol-gel transition to form a plant-based protein hydrogel;
(c) subjecting the plant-based protein hydrogel to a shear treatment to form a plant-based protein hydrogel slurry;
(d) dispersing an active ingredient(s) in said plant-based protein hydrogel slurry to form a composition; and
(e) drying said composition thereby evaporating at least part of both the first and second co-solvent to form a microcapsule.

2. A method according to claim 1, wherein the plant-based protein(s) is selected from pea protein, potato protein, rapeseed protein, lentil protein, chickpea protein, fava bean protein, mung bean protein, sunflower seed protein, pumpkin seed protein, flax protein, chia protein, canola protein, lupine protein, alfalfa protein, moringa protein and/or rice protein, preferably pea protein and/or potato protein.

3. The method according to claim 1 or claim 2, wherein the first co-solvent is an organic acid, preferably wherein the organic acid is acetic acid, lactic acid, formic acid, gluconic acid, propionic acid, an α-hydroxy acid, and/or a β-hydroxy acid, more preferably lactic acid or acetic acid, and wherein the first co-solvent is present in the solution of step (a) at a concentration based on weight% of equal or greater than the concentration of the protein solids.

4. The method according to any one of claims 1 to 3, wherein the second co-solvent is selected from water, ethanol, and/or ethyl acetate, more preferably water and/or ethanol, even more preferably water.

5. The method according to any one of claims 1 to 4, wherein in step (b) the protein solution is heated to a first temperature above the sol-gel transition temperature of the one or more plant-based protein(s) solution, then reduced to a second temperature below the sol-gel transition temperature of the one or more plant-based protein(s) solution to form a hydrogel.

6. A method according to any one of claims 1 to 5, further comprising a step of altering the pH of the plant-based protein hydrogel slurry such that it is different to the isoelectric point of the plant-based protein by more than 1 pH unit, preferably wherein said step of altering the pH of the plant-based protein hydrogel slurry is carried out after step (c) or sequentially with step (c).

7. A method according to any one of claims 1 to 6, wherein said active ingredient(s) is selected from a vitamin, a mineral, a flavour material, a fragrance material, a pro-flavour, a pro-fragrance, a flavour enhancer, a malodour counteractant, a nutraceutical, a live organism (e.g. a probiotic), a pharmaceutical, an anti-microbial agent, an anti-viral agent, an anti-inflammatory agent, a pesticide, a herbicide, a fertiliser, a fungicide, an insecticide, an animal repellent, an anti-acne agent, a skin lightening agent, an emollient, a skin moisturizing agent, a wrinkle control agent, a fabric softener active, a surface cleaning active, a skin conditioning agent, a hair conditioning agent, a dye, a pigment, and an adhesive, or combinations thereof, preferably wherein said active ingredient(s) is selected from Vitamin A, Vitamin B1, Vitamin B2, Vitamin B3, Vitamin B5, Vitamin B6, Vitamin B7, Vitamin B9, Vitamin B12, Vitamin C, Vitamin D, Vitamin E, Vitamin K, magnesium, sodium, potassium, zinc, iron, calcium, iodine, flaxseed oil, omeg-3 fatty acid, folic acid, thiamin, riboflavin, niacin and phosphorous, or mixtures thereof, more preferably Vitamin D.

8. A method for the preparation of a biodegradable microcapsule composition, the method comprising:
(a) preparing a biodegradable microcapsule according to the method as claimed in any one of claims 1 to 7; and
(b) suspending the biodegradable microcapsule in an external phase.

9. A biodegradable microcapsule comprising an active ingredient(s) and a plant-based protein carrier comprising a plant-based protein(s), wherein the plant-based protein carrier encapsulates the active ingredient(s), wherein the plant-based protein has a solubility of less than 50% when measured at a protein concentration of 5% w/w in an aqueous solution at pH 7 and 25°C, and wherein the plant-based protein(s) have a protein secondary structure with at least 40% intermolecular β-sheet, wherein the % intermolecular β-sheet content is measured by FTIR.

10. A biodegradable microcapsule according to claim 9, wherein the plant-based protein(s) is selected from pea protein, potato protein, rapeseed protein, lentil protein, chickpea protein, fava bean protein, mung bean protein, sunflower seed protein, pumpkin seed protein, flax protein, chia protein, canola protein, lupine protein, alfalfa protein, moringa protein and/or rice protein, preferably pea protein and/or potato protein, preferably wherein said plant-based protein(s) has been pre-treated with an organic acid, preferably wherein the organic acid is acetic acid, lactic acid, formic acid, propionic acid, an α-hydroxy acid and/or a β-hydroxy acid, more preferably lactic acid or acetic acid.

11. A biodegradable microcapsule according to any one of claims 9 to 10, wherein said active ingredient(s) is selected from a vitamin, a mineral, a flavour material, a fragrance material, a pro-favour, a pro-fragrance, a flavour enhancer, a malodour counteractant, a nutraceutical, a live organism (e.g. a probiotic), a pharmaceutical, an anti-microbial agent, an anti-viral agent, an anti-inflammatory agent, a pesticide, a herbicide, a fertiliser, a fungicide, an insecticide, an animal repellent, an anti-acne agent, a skin lightening agent, an emollient, a skin moisturizing agent, a wrinkle control agent, a fabric softener active, a surface cleaning active, a skin conditioning agent, a hair conditioning agent, a dye, a pigment, and an adhesive, or combinations thereof, preferably wherein said active ingredient(s) is a vitamin or a mineral, preferably selected from Vitamin A, Vitamin B1, Vitamin B2, Vitamin B3, Vitamin B5, Vitamin B6, Vitamin B7, Vitamin B9, Vitamin B12, Vitamin C, Vitamin D, Vitamin E, Vitamin K, magnesium, sodium, potassium, zinc, iron, calcium, iodine, flaxseed oil, omega-3 fatty acid, folic acid, thiamin, riboflavin, niacin and phosphorous, or mixtures thereof, more preferably Vitamin D.

12. A biodegradable microcapsule according to any one of claims 9 to 11 wherein the microcapsule has a d₅₀ as determined by laser diffraction of less than or equal to 250 µm, less than or equal to 200 µm, less than or equal to 150 µm, less than or equal to 100 µm, less than or equal to 50 µm, less than or equal to 30 µm, less than or equal to 10 µm.

13. A biodegradable microcapsule according to any one of claims 9 to 12, wherein the plant-based protein(s) have a protein secondary structure with, at least 50% intermolecular β-sheet, at least 60% intermolecular β-sheet, at least 70% intermolecular β-sheet, at least 80% intermolecular β-sheet, or at least 90% intermolecular β-sheet, wherein the % intermolecular β-sheet content is measured by FTIR.

14. The use of a biodegradable microcapsule as claimed in any one of claims 9 to 13 in a formulated product, preferably wherein the formulated product is a food, beverage, cosmetic, home care product, personal care product, pharmaceutical, industrial product (e.g. paint, adhesive, sandpaper, tape etc.), medical device, biomaterial, or agrochemical.

## Patentansprüche

1. Verfahren zur Herstellung einer biologisch abbaubaren Mikrokapsel, wobei das Verfahren Folgendes umfasst:
(a) Herstellen einer Lösung, die ein oder mehrere pflanzliche Proteine in einem Lösungsmittelsystem enthält, wobei das Lösungsmittelsystem mischbare Co-Lösungsmittel umfasst; wobei ein erstes Co-Lösungsmittel die Löslichkeit des/der pflanzlichen Proteine(s) erhöht und ein zweites Co-Lösungsmittel die Löslichkeit des/der pflanzlichen Proteine(s) verringert; und wobei die Co-Lösungsmittel der Lösung entweder in konzentrierter oder in verdünnter Form zugesetzt werden; vorzugsweise wobei der pH-Wert der pflanzlichen Proteinlösung mindestens 0,5 pH-Einheiten unter dem isoelektrischen Punkt des pflanzlichen Proteins liegt;
b) Induzieren eines Sol-Gel-Übergangs des pflanzlichen Proteins in der Lösung zur Bildung eines pflanzlichen Proteinhydrogels;
c) Unterziehen des pflanzlichen Proteinhydrogels einer Scherbehandlung zur Bildung einer pflanzlichen Proteinhydrogelsuspension;
d) Dispergieren eines oder mehrerer Wirkstoffe in der genannten pflanzlichen Proteinhydrogel-Suspension zur Bildung einer Zusammensetzung; und
(e) Trocknen der Zusammensetzung, wobei mindestens ein Teil sowohl des ersten als auch des zweiten Co-Lösungsmittels verdampft wird , um eine Mikrokapsel zu bilden.

2. Verfahren nach Anspruch 1 , wobei das/die pflanzliche(n) Protein(e) ausgewählt ist/sind aus Erbsenprotein, Kartoffelprotein, Rapsprotein, Linsenprotein, Kichererbsenprotein, Ackerbohnenprotein, Mungbohnenprotein, Sonnenblumenkernprotein, Kürbiskernprotein, Leinsamenprotein, Chiaprotein, Canolaprotein, Lupinenprotein, Alfalfaprotein, Moringaprotein und/oder Reisprotein, vorzugsweise Erbsenprotein und/oder Kartoffelprotein.

3. Verfahren nach Anspruch 1 oder 2, wobei das erste Co-Lösungsmittel eine organische Säure ist, vorzugsweise Essigsäure, Milchsäure, Ameisensäure, Gluconsäure, Propionsäure, eine α-Hydroxysäure und/oder eine β-Hydroxysäure, besonders bevorzugt Milchsäure oder Essigsäure, und wobei das erste Co-Lösungsmittel in der Lösung aus Schritt (a) in einer auf Gewichtsprozent basierenden Konzentration vorliegt, die gleich oder größer ist als die Konzentration der Proteinfeststoffe.

4. Verfahren nach einem der Ansprüche 1 bis 3 , wobei das zweite Co-Lösungsmittel ausgewählt ist aus Wasser, Ethanol und/oder Ethylacetat, besonders bevorzugt Wasser und/oder Ethanol, noch bevorzugter Wasser.

5. Verfahren nach einem der Ansprüche 1 bis 4 , wobei in Schritt (b) die Proteinlösung auf eine erste Temperatur oberhalb der Sol-Gel-Übergangstemperatur der einen oder mehreren pflanzlichen Proteinlösungen erhitzt und anschließend auf eine zweite Temperatur unterhalb der Sol-Gel-Übergangstemperatur der einen oder mehreren pflanzlichen Proteinlösungen abgekühlt wird, um ein Hydrogel zu bilden.

6. Verfahren nach einem der Ansprüche 1 bis 5, ferner umfassend einen Schritt der pH-Wert-Änderung der pflanzlichen Proteinhydrogelsuspension, sodass dieser sich um mehr als eine pH-Einheit vom isoelektrischen Punkt des pflanzlichen Proteins unterscheidet, wobei dieser Schritt der pH-Wert-Änderung der pflanzlichen Proteinhydrogelsuspension vorzugsweise nach Schritt (c) oder nacheinander mit Schritt (c) durchgeführt wird.

7. Verfahren nach einem der Ansprüche 1 bis 6, wobei der/die Wirkstoff(e) ausgewählt ist/sind aus einem Vitamin, einem Mineralstoff, einem Aromastoff, einem Duftstoff, einem Pro-Aromastoff, einem Pro-Duftstoff, einem Geschmacksverstärker, einem Geruchsneutralisator, einem Nutrazeutikum, einem lebenden Organismus (z. B. einem Probiotikum), einem Arzneimittel, einem antimikrobiellen Mittel, einem antiviralen Mittel, einem entzündungshemmenden Mittel, einem Pestizid, einem Herbizid, einem Düngemittel, einem Fungizid, einem Insektizid, einem Tierabwehrmittel, einem Mittel gegen Akne, einem Hautaufheller, einem Weichmacher, einem Feuchtigkeitsspender, einem Mittel gegen Falten, einem Weichspüler, einem Oberflächenreinigungsmittel, einem Hautpflegemittel, einem Haarpflegemittel, einem Farbstoff, einem Pigment und einem Klebstoff oder Kombinationen davon, vorzugsweise wobei der/die Wirkstoff(e) ausgewählt ist/sind aus Vitamin A, Vitamin B1, Vitamin B2, Vitamin B3, Vitamin B5, Vitamin B6, Vitamin B7, Vitamin B9 oder Vitamin B6 ausgewählt ist/sind . Vitamin B12, Vitamin C, Vitamin D, Vitamin E, Vitamin K, Magnesium, Natrium, Kalium, Zink, Eisen, Calcium, Jod, Leinöl, Omega-3-Fettsäure, Folsäure, Thiamin , Riboflavin, Niacin und Phosphor oder Mischungen davon, vorzugsweise Vitamin D.

8. Verfahren zur Herstellung einer biologisch abbaubaren Mikrokapselzusammensetzung, wobei das Verfahren Folgendes umfasst:
(a) Herstellung einer biologisch abbaubaren Mikrokapsel nach dem Verfahren eines der Ansprüche 1 bis 7; und
b) Suspension der biologisch abbaubaren Mikrokapsel in einer externen Phase.

9. Eine biologisch abbaubare Mikrokapsel, bestehend aus einem oder mehreren Wirkstoffen und einem pflanzlichen Proteinträger, der ein oder mehrere pflanzliche Proteine enthält, wobei der pflanzliche Proteinträger den/die Wirkstoff(e) einkapselt, wobei das pflanzliche Protein bei einer Proteinkonzentration von 5 Gew.-% in wässriger Lösung bei pH 7 und 25 ° C eine Löslichkeit von weniger als 50 % aufweist und wobei das/die pflanzliche(n) Protein(e) eine Proteinstruktur mit mindestens 40 % intermolekularem β-Faltblatt aufweist/aufweisen, wobei der Anteil an intermolekularem β-Faltblatt mittels FTIR gemessen wird.

10. Eine biologisch abbaubare Mikrokapsel nach Anspruch 8 oder 9 , wobei das/die pflanzliche(n) Protein(e) ausgewählt ist/sind aus Erbsenprotein, Kartoffelprotein, Rapsprotein, Linsenprotein , Kichererbsenprotein, Ackerbohnenprotein, Mungbohnenprotein, Sonnenblumenkernprotein, Kürbiskernprotein, Leinsamenprotein, Chiaprotein, Canolaprotein, Lupinenprotein, Luzerneprotein, Moringaprotein und/oder Reisprotein, vorzugsweise Erbsenprotein und/oder Kartoffelprotein, wobei das/die pflanzliche(n) Protein(e) vorzugsweise mit einer organischen Säure vorbehandelt wurde/wurden, wobei es sich bei der organischen Säure vorzugsweise um Essigsäure, Milchsäure, Ameisensäure, Propionsäure, eine α-Hydroxysäure und/oder eine β-Hydroxysäure handelt, besonders bevorzugt um Milchsäure oder Essigsäure.

11. Eine biologisch abbaubare Mikrokapsel nach einem der Ansprüche 8 bis 10, wobei der/die Wirkstoff(e) ausgewählt ist/sind aus einem Vitamin, einem Mineralstoff, einem Aromastoff, einem Duftstoff, einem Geschmacksverstärker, einem Geruchsneutralisator, einem Nutrazeutikum, einem lebenden Organismus (z. B. einem Probiotikum), einem Arzneimittel, einem antimikrobiellen Mittel, einem antiviralen Mittel, einem entzündungshemmenden Mittel, einem Pestizid, einem Herbizid, einem Düngemittel, einem Fungizid, einem Insektizid, einem Tierabwehrmittel, einem Mittel gegen Akne, einem Hautaufheller, einem Weichmacher, einem Feuchtigkeitsspender, einem Mittel gegen Falten, einem Weichspüler, einem Reinigungsmittel, einem Hautpflegemittel, einem Haarpflegemittel, einem Farbstoff, einem Pigment und einem Klebstoff oder Kombinationen davon, vorzugsweise wobei der/die Wirkstoff(e) ein Vitamin oder ein Mineralstoff ist/sind, vorzugsweise ausgewählt aus Vitamin A, Vitamin B1, Vitamin B2, Vitamin B3, Vitamin B4 Vitamin B5, Vitamin B6, Vitamin B7, Vitamin B9, Vitamin B12, Vitamin C, Vitamin D, Vitamin E, Vitamin K, Magnesium, Natrium, Kalium, Zink, Eisen, Calcium, Jod, Leinöl, Omega-3-Fettsäure, Folsäure , Thiamin , Riboflavin, Niacin und Phosphor oder Mischungen davon, vorzugsweise Vitamin D.

12. Eine biologisch abbaubare Mikrokapsel nach einem der Ansprüche 8 bis 11, wobei die Mikrokapsel einen Durchmesser d₅₀ aufweist, der durch Laserbeugung bestimmt wurde, von höchstens 250 µm , höchstens 200 µm, höchstens 150 µm , höchstens 100 µm , höchstens 50 µm , höchstens 30 µm oder höchstens 10 µm.

13. Eine biologisch abbaubare Mikrokapsel nach einem der Ansprüche 8 bis 12, wobei das/die pflanzliche(n) Protein(e) eine Proteinsekundärstruktur mit mindestens 50 %, mindestens 60 %, mindestens 70 %, mindestens 80 % oder mindestens 90 % intermolekularem β-Faltblatt aufweisen, wobei der Anteil an intermolekularem β-Faltblatt mittels FTIR gemessen wird.

14. Die Verwendung einer biologisch abbaubaren Mikrokapsel nach einem der Ansprüche 8 bis 13 in einem formulierten Produkt, vorzugsweise bei einem Lebensmittel, Getränk, Kosmetikum, Haushaltspflegeprodukt, Körperpflegeprodukt, Arzneimittel, Industrieprodukt (z. B. Farbe, Klebstoff, Schleifpapier, Klebeband usw.), Medizinprodukt, Biomaterial oder Agrochemikalie.

## Revendications

1. Procédé de préparation d'une microcapsule biodégradable, le procédé comprenant :
(a) la formation d'une solution comprenant une ou plusieurs protéines végétales dans un système solvant, dans lequel le système solvant comprend des cosolvants miscibles ; dans lequel un premier cosolvant augmente la solubilité de la ou des protéines végétales, et un second cosolvant diminue la solubilité de la ou des protéines végétales ; et dans lequel les cosolvants sont ajoutés à la solution sous forme concentrée ou diluée ; de préférence dans lequel le pH de la solution de protéines végétales est inférieur d'au moins 0,5 unité de pH au point isoélectrique de la protéine végétale ;
(b) induire la protéine végétale dans la solution à subir une transition sol-gel pour former un hydrogel de protéine végétale ;
(c) soumettre l'hydrogel de protéines végétales à un traitement de cisaillement pour former une suspension d'hydrogel de protéines végétales ;
(d) disperser un ou plusieurs ingrédients actifs dans ladite suspension d'hydrogel de protéines végétales pour former une composition ; et
(e) sécher ladite composition, évaporant ainsi au moins une partie des premier et deuxième co-solvants pour former une microcapsule.

2. Procédé selon la revendication 1, dans lequel la ou les protéines végétales sont choisies parmi les protéines de pois, de pomme de terre, de colza, de lentille, de pois chiche, de fève, de haricot mungo, de graine de tournesol, de graine de citrouille, de lin, de chia, de canola, de lupin, de luzerne, de moringa et/ou de riz, de préférence les protéines de pois et/ou de pomme de terre.

3. Procédé selon la revendication 1 ou la revendication 2, dans lequel le premier co-solvant est un acide organique, de préférence dans lequel l'acide organique est l'acide acétique, l'acide lactique, l'acide formique, l'acide gluconique, l'acide propionique, un acide α-hydroxy et/ou un acide β-hydroxy, de préférence l'acide lactique ou l'acide acétique, et dans lequel le premier co-solvant est présent dans la solution de l'étape (a) à une concentration en % en poids égale ou supérieure à la concentration des solides protéiques.

4. Procédé selon l'une quelconque des revendications 1 à 3, dans lequel le deuxième co-solvant est choisi parmi l'eau, l'éthanol et/ou l'acétate d'éthyle, de préférence l'eau et/ou l'éthanol, et plus préférablement encore l'eau.

5. Procédé selon l'une quelconque des revendications 1 à 4, dans lequel, à l'étape (b), la solution protéique est chauffée à une première température supérieure à la température de transition sol-gel de la ou des solutions protéiques d'origine végétale, puis refroidie à une deuxième température inférieure à la température de transition sol-gel de la ou des solutions protéiques d'origine végétale pour former un hydrogel.

6. Procédé selon l'une quelconque des revendications 1 à 5, comprenant en outre une étape consistant à modifier le pH de la suspension d'hydrogel de protéines végétales de telle sorte qu'il soit différent du point isoélectrique de la protéine végétale de plus d'une unité de pH, de préférence dans lequel ladite étape consistant à modifier le pH de la suspension d'hydrogel de protéines végétales est effectuée après l'étape (c) ou séquentiellement avec l'étape (c).

7. Procédé selon l'une quelconque des revendications 1 à 6, dans lequel ledit ou lesdits ingrédients actifs sont choisis parmi une vitamine, un minéral, un arôme, un parfum, un exhausteur d'arôme, un exhausteur de parfum, un exhausteur de goût, un neutralisant d'odeurs désagréables, un nutraceutique, un organisme vivant (par exemple un probiotique), un produit pharmaceutique, un agent antimicrobien, un agent antiviral, un agent anti-inflammatoire, un pesticide, un herbicide, un engrais, un fongicide, un insecticide, un répulsif pour animaux, un agent anti-acnéique, un agent éclaircissant pour la peau, un émollient, un agent hydratant pour la peau, un agent anti-rides, un agent assouplissant pour tissus, un agent nettoyant de surface, un agent revitalisant pour la peau, un agent revitalisant pour les cheveux, un colorant, un pigment et un adhésif, ou des combinaisons de ceux-ci, de préférence dans lesquels ledit ou lesdits ingrédients actifs sont choisis parmi la vitamine A, la vitamine B1, la vitamine B2, la vitamine B3, la vitamine B5, la vitamine B6, la vitamine B7, la vitamine B9, la vitamine B12, la vitamine C, la vitamine D, la vitamine E, vitamine K, le magnésium, le sodium, le potassium, le zinc, le fer, le calcium, l'iode, l'huile de lin, l'acide gras oméga-3, l'acide folique, la thiamine, la riboflavine, la niacine et le phosphore, ou des mélanges de ceux-ci, de préférence la vitamine D.

8. Procédé de préparation d'une composition de microcapsules biodégradables, le procédé comprenant :
(a) la préparation d'une microcapsule biodégradable selon le procédé revendiqué dans l'une quelconque des revendications 1 à 7 ; et
(b) la mise en suspension de la microcapsule biodégradable dans une phase externe.

9. Microcapsule biodégradable comprenant un ou plusieurs ingrédients actifs et un support protéique à base de plantes comprenant une ou plusieurs protéines à base de plantes, dans laquelle le support protéique à base de plantes encapsule le ou les ingrédients actifs, dans laquelle la protéine à base de plantes a une solubilité inférieure à 50 % lorsqu'elle est mesurée à une concentration en protéines de 5 % p/p dans une solution aqueuse à pH 7 et 25 °C, et dans lequel la ou les protéines végétales ont une structure secondaire protéique avec au moins 40 % de feuillets β intermoléculaires, le pourcentage de teneur en feuillets β intermoléculaires étant mesuré par FTIR.

10. Microcapsule biodégradable selon la revendication 8 ou la revendication 9, dans laquelle la ou les protéines végétales sont choisies parmi les protéines de pois, les protéines de pomme de terre, les protéines de colza, les protéines de lentilles, les protéines de pois chiches, les protéines de fèves, les protéines de haricots mungo, les protéines de graines de tournesol, protéine de graines de citrouille, protéine de lin, protéine de chia, protéine de canola, protéine de lupin, protéine de luzerne, protéine de moringa et/ou protéine de riz, de préférence la protéine de pois et/ou la protéine de pomme de terre, de préférence dans laquelle ladite ou lesdites protéines végétales ont été prétraitées avec un acide organique, de préférence dans laquelle l'acide organique est l'acide acétique, de l'acide lactique, de l'acide formique, de l'acide propionique, un acide α-hydroxy et/ou un acide β-hydroxy, de préférence l'acide lactique ou l'acide acétique.

11. Microcapsule biodégradable selon l'une quelconque des revendications 8 à 10, dans laquelle ledit ou lesdits ingrédients actifs sont choisis parmi une vitamine, un minéral, un arôme, un parfum, un exhausteur de goût, un exhausteur de parfum, un activateur d'arôme, un neutralisant de mauvaises odeurs, un nutraceutique, un organisme vivant (par exemple un probiotique), un produit pharmaceutique, un agent antimicrobien, un agent antiviral, un agent anti-inflammatoire, un pesticide, un herbicide, un engrais, un fongicide, un insecticide, un répulsif pour animaux, un agent anti-acnéique, un agent éclaircissant pour la peau, un émollient, un agent hydratant pour la peau, un agent anti-rides, un agent assouplissant pour tissus, un agent nettoyant de surface, un agent revitalisant pour la peau, un agent revitalisant pour les cheveux, un colorant, un pigment et un adhésif, ou des combinaisons de ceux-ci, de préférence dans lesquels ledit ou lesdits ingrédients actifs sont une vitamine ou un minéral, de préférence choisis parmi la vitamine A, la vitamine B1, la vitamine B2, la vitamine B3, la vitamine B5, la vitamine B6, la vitamine B7, la vitamine B9, la vitamine B12, la vitamine C, la vitamine D, la vitamine E, vitamine K, le magnésium, le sodium, le potassium, le zinc, le fer, le calcium, l'iode, l'huile de lin, l'acide gras oméga-3, l' e l'acide folique, la thiamine, la riboflavine, la niacine et le phosphore, ou des mélanges de ceux-ci, de préférence la vitamine D.

12. Microcapsule biodégradable selon l'une quelconque des revendications 8 à 11, dans laquelle la microcapsule a une d50 déterminée par diffraction laser inférieure ou égale à 250 µm, inférieure ou égale à 200 µm, inférieure ou égale à 150 µm, inférieure ou égale à 100 µm, inférieure ou égale à 50 µm, inférieure ou égale à 30 µm, inférieure ou égale à 10 µm.

13. Microcapsule biodégradable selon l'une quelconque des revendications 8 à 12, dans laquelle la ou les protéines d'origine végétal ont une structure secondaire protéique avec au moins 50 % de feuillets β intermoléculaires, au moins 60 % de feuillets β intermoléculaires, au moins 70 % de feuillets β intermoléculaires, au moins 80 % de feuillets β intermoléculaires ou au moins 90 % de feuillets β intermoléculaires, dans laquelle le pourcentage de contenu en feuillets β intermoléculaires est mesuré par FTIR.

14. L'utilisation d'une microcapsule biodégradable selon l'une quelconque des revendications 8 à 13 dans un produit formulé, de préférence dans lequel le produit formulé est un aliment, une boisson, un cosmétique, un produit d'entretien ménager, un produit de soins personnels, un produit pharmaceutique, un produit industriel (par exemple, une peinture, un adhésif, du papier de verre, du ruban adhésif, etc.), un dispositif médical, un biomatériau ou un produit agrochimique.
